# EUROPEAN PATENT APPLICATION

(11) **EP 4 353 236 A1**
(43) Date of publication of application: **17.04.2024**
(21) Application number: 22819598.8
(22) Date of filing: 09.06.2022
(51) Int. Cl.: A61K 31/52, A61K 31/522, A61K 31/282, A61P 35/00

(54) **COMPOUND AND METHOD FOR TREATING CHEMOTHERAPY-RELATED GASTROINTESTINAL SIDE EFFECTS**

(30) Priority: 10.06.2021 CN 202110650388
(71) Applicant: OnQuality Pharmaceuticals China Ltd., Baoshan District Shanghai 200431 (CN)
(72) Inventor: XIANG, Jinnan, Shanghai 201112 (CN); LI, Wenxi, Shanghai 201112 (CN); YANG, Linan, Shanghai 201112 (CN); ZHANG, Shiyi, Shanghai 201112 (CN)
(74) Representative: AWA Denmark A/S
(86) International application number: PCT/CN2022/097792
(87) International publication number: WO 2022/258001

(57) **Abstract**

A method for treating chemotherapy-related gastrointestinal side effects, in particular, relating to use of a CDK inhibitor in preparation of a drug. The drug is used for preventing, alleviating and/or treating the chemotherapy- related gastrointestinal side effects on a subject. The CDK inhibitor comprises a CDK1 inhibitor, a CDK2 inhibitor, a CDK3 inhibitor, a CDK4 inhibitor, a CDK5 inhibitor, a CDK6 inhibitor, a CDK7 inhibitor, a CDK 8 inhibitor, and/or CDK9 inhibitor. A chemotherapeutic agent may be selected from fluorouracil, oxaliplatin, topotecan, irinotecan, tetrahydrofolate, docetaxel, and the like. The gastrointestinal side effects comprise diarrhea, constipation, and the like.

## Description

### TECHNICAL FIELD

The present application relates to the field of biomedicine, and particularly to a method of treating chemotherapy-associated gastrointestinal side effects using the CDK inhibitor.

### BACKGROUND

Tumor chemotherapy is one of the most commonly used means for treating tumors clinically, which mainly utilizes a chemical medicament (a chemotherapeutic agent) to prevent the proliferation, infiltration, and metastasis of cancer cells, eventually killing cancer cells. However, the administration of a chemotherapeutic agent can not only kill rapidly growing and isolated tumor cells, it may also kill normal cells and immune cells at the same time, thus causing severe side effects, including nausea, vomiting, anemia, inflammation and infection, etc. Such side effects may result in withdrawal of the chemotherapeutic agent or dosage reduction, greatly affecting the therapeutic effect on the tumor of patients, and impairing the quality of life for patients, and endangering the patient's life in severe cases.

No successful therapeutic regimen has been available yet in the prior art for controlling the side effects associated with the administration of a chemotherapeutic agent. Therefore, there is an urgent need for therapeutic regimens that can successfully control these side effects.

### SUMMARY OF THE INVENTION

The present application provides a method of preventing, relieving and/or treating chemotherapy-associated gastrointestinal side effects (e.g., diarrhea or constipation) in a subject, comprising administering a cyclin-dependent kinase (CDK) inhibitor to the subject. The medicament of the present application is capable of effectively relieving chemotherapy-associated gastrointestinal side effects.

In one aspect, the present application provides a use of a cyclin-dependent kinase (CDK) inhibitor in preparing a medicament, which is used for preventing, relieving and/or treating chemotherapy-associated gastrointestinal side effects in a subject.

In some embodiments, the CDK inhibitor comprises a reagent that reduces the expression of CDK, and/or a reagent that decreases the activity of CDK.

In some embodiments, the CDK inhibitor acts directly on a CDK protein, a nucleic acid encoding a CDK protein, a cyclin and/or a nucleic acid encoding a cyclin.

In some embodiments, the CDK inhibitor comprises CDK1 inhibitor, CDK2 inhibitor, CDK3 inhibitor, CDK4 inhibitor, CDK5 inhibitor, CDK6 inhibitor, CDK7 inhibitor, CDK8 inhibitor and/or CDK9 inhibitor.

In some embodiments, the CDK inhibitor comprises a CDK2 inhibitor.

In some embodiments, the CDK inhibitor comprises a CDK4 inhibitor.

In some embodiments, the CDK inhibitor comprises a CDK6 inhibitor.

In some embodiments, the CDK inhibitor comprises a CDK9 inhibitor.

In some embodiments, the CDK inhibitor comprises a CDK4/6 inhibitor.

In some embodiments, the CDK inhibitor comprises a CDK2/4/6 inhibitor.

In some embodiments, the CDK inhibitor comprises a CDK4/6/9 inhibitor.

In some embodiments, the CDK inhibitor comprises a CDK inhibitor for local intestinal exposure.

In some embodiments, the CDK inhibitor comprises a small molecular compound, a protein and/or a nucleic acid molecule.

In some embodiments, the CDK inhibitor comprises a small molecular CDK inhibitor, a protein macromolecule that binds specifically to CDK, a RNAi that inhibits the expression of a CDK protein and/or an antisense oligonucleotide that inhibits the expression of a CDK protein.

In some embodiments, the small molecular CDK inhibitor comprises a small molecular CDK inhibitor that binds reversibly to CDK, a small molecular CDK inhibitor that binds irreversibly to CDK and/or a small molecular CDK inhibitor that binds specifically to mutant CDK.

In some embodiments, the small molecular CDK inhibitor has a molecular weight that is less than or equal to 2000 Daltons, less than or equal to 1500 Daltons, less than or equal to 1200 Daltons, less than or equal to 1000 Daltons, less than or equal to 900 Daltons, less than or equal to 800 Daltons, less than or equal to 700 Daltons, less than or equal to 600 Daltons, less than or equal to 500 Daltons, less than or equal to 400 Daltons, less than or equal to 300 Daltons, less than or equal to 200 Daltons and/or less than or equal to 100 Daltons.

In some embodiments, the CDK inhibitor comprises one or more compounds selected from the following group: Trilaciclib, Palbociclib, Ribociclib, Abemaciclib, FLX-925, SHR-6390, BPI-1178, BPI-16350, FCN 437, G2T28, XZP-3287, BEBT-209, TY-302, TQB-3616, HS-10342, PF-06842874, CS-3002, MM-D37K, zotiraciclib, XZP-3287, Rigosertib, KRX-0601, Riviciclib, roniciclib, Milciclib, Seliciclib, Roscovitine, Indisulam, Alvocidib, NUV-422, BEY-1107, GLR-2007, FN-1501, BCD-115, TP-1287, BAY-1251152, Atuveciclib, SEL-120, HX-301, Voruciclib, Fadraciclib, AGM-130, PHA-793887, PHA-690509, Dinaciclib, RO4584820, R547, AT-7519, RGB-286638, ZK-304709, IIIM-290, PF-07104091 and G1T38.

In some embodiments, the chemotherapy comprises administration of a chemotherapeutic agent. In some embodiments, the chemotherapeutic agent is a cytotoxic agent.

In some embodiments, the chemotherapeutic agent is one or more selected from the following group: a DNA synthesis inhibitor, an RNA synthesis inhibitor, a protein synthesis inhibitor, a cell division inhibitor, a DNA base analogue, a topoisomerase inhibitor and/or a telomerase synthesis inhibitor.

In some embodiments, the chemotherapeutic agent is selected from fluorouracil, oxaliplatin, tetrahydrofolic acid, irinotecan, topotecan, docetaxel, gemcitabine, etoposide, carboplatin, methotrexate, doxorubicin, cytarabine, vinorelbine and capecitabine, and a combination thereof.

In some embodiments, the chemotherapeutic agent does not include a tumor-targeted medicament. In some embodiments, the chemotherapeutic agent does not include a tyrosine kinase inhibitor. In some embodiments, the chemotherapeutic agent does not include an EGFR-targeted anti-tumor medicament. In some embodiments, the chemotherapeutic agent does not include a PI3K-targeted anti-tumor medicament. In some embodiments, the chemotherapeutic agent does not include a FGFR4-targeted anti-tumor medicament. In some embodiments, the chemotherapeutic agent does not include Afatinib, Idelalisib, Fisogatinib, Imatinib and Osimertinib.

In some embodiments, the chemotherapeutic agent is administered continuously and/or noncontinuously.

In some embodiments, the chemotherapeutic agent does not include chemotherapeutic agents of which the continuous administration time is 7 days or above.

In some embodiments, the chemotherapy is administered in combination with one or more other therapies.

In some embodiments, the chemotherapy-associated gastrointestinal side effects comprise gastrointestinal side effects caused by the chemotherapy.

In some embodiments, the chemotherapy-associated gastrointestinal side effects comprise gastrointestinal adverse events that occur or deteriorate after the administration of the chemotherapeutic agent.

In some embodiments, the gastrointestinal adverse events occur or deteriorate about 1 hr, about 2 hrs, about 3 hrs, about 4 hrs, about 5 hrs, about 6 hrs, about 7 hrs, about 8 hrs, about 9 hrs, about 10 hrs, about 11 hrs, about 12 hrs, about 1 day, about 2 days, about 4 days, about 7 days, about 2 weeks, about 3 weeks, about 1 month, about 2 months or longer after the administration of the chemotherapeutic agent, in the absence of prevention or treatment.

In some embodiments, the gastrointestinal side effects comprise gastric mucosal injury diseases and/or intestinal mucosal injury diseases. In some embodiments, the gastrointestinal side effects are not side effects caused by bone marrow suppression.

In some embodiments, the gastrointestinal side effects comprise diarrhea, abdominal pain, nausea, vomiting, mucositis, loss of appetite, gastric ulcer, gastritis, constipation, enteritis, intestinal perforation, intestinal bleeding, ulcer, intestinal necrosis.

In some embodiments, the gastrointestinal side effects comprise diarrhea, constipation.

In some embodiments, the severity of the gastrointestinal side effects is Grade 1 or above, Grade 2 or above, Grade 3 or above, Grade 4 or above, or Grade 5, as evaluated in accordance with NCI-CTCAE V5.0.

In some embodiments, the subject comprises a cancer patient.

In some embodiments, the medicament does not substantially affect the therapeutic effect of the chemotherapy.

In some embodiments, the medicament is administered about 0.5 hrs, about 1 hr, about 2 hrs, about 3 hrs, about 4 hrs, about 5 hrs, about 6 hrs, about 7 hrs, about 8 hrs, about 9 hrs, about 10 hrs, about 11 hrs, about 12 hrs, about 13 hrs, about 14 hrs, about 15 hrs, about 16 hrs, about 17 hrs, about 18 hrs, about 19 hrs, about 20 hrs or longer before the administration of the chemotherapy.

In some embodiments, the medicament is administered about 0.5-12 hrs before the administration of the chemotherapy.

In some embodiments, the medicament is prepared for oral administration, intravenous injection, subcutaneous injection, intraperitoneal injection and/or intramuscular injection.

In some embodiments, the medicament is prepared for injection administration. In some embodiments, the medicament is prepared as injection solution.

In some embodiments, the medicament is prepared for oral administration. In some embodiments, the medicament is prepared as tablets and/or capsules.

In some embodiments, the medicament further comprises one or more other active ingredients.

In some embodiments, the CDK inhibitor can improve or reduce the gastrointestinal side effects independently.

In another aspect, the present application provides a use of a cyclin-dependent kinase (CDK) inhibitor in preparing a medicament, which is used for preventing or treating chemotherapy-associated diarrhea in a subject.

In another aspect, the present application provides a use of a cyclin-dependent kinase (CDK) inhibitor in preparing a medicament, which is used for preventing or treating chemotherapy-associated constipation in a subject.

In another aspect, the present application provides a method of preventing, relieving and/or treating chemotherapy-associated gastrointestinal side effects, comprising administering to a subject in need thereof the CDK inhibitor of the present application.

In another aspect, the present application provides the CDK inhibitor, which is used for preventing, relieving and/or treating chemotherapy-associated gastrointestinal side effects.

In another aspect, the present application provides a method of preventing, relieving and/or treating chemotherapy-associated diarrhea, comprising administering to a subject in need thereof the CDK inhibitor of the present application.

In another aspect, the present application provides a method of preventing, relieving and/or treating chemotherapy-associated constipation, comprising administering to a subject in need thereof the CDK inhibitor of the present application.

In another aspect, the present application provides a pharmaceutical composition, comprising the CDK inhibitor and the medicament used for the chemotherapy.

In another aspect, the present application provides a kit, comprising the CDK inhibitor and the chemotherapeutic agent.

Other aspects and advantages of the present application will be readily apparent to those skilled in the art from the following detailed description. Only the exemplary embodiments of the present application are shown and described in the following detailed description. As will be appreciated by those skilled in the art, the disclosure of the present application allows persons skilled in the art to modify the disclosed embodiments without departing from the spirit and scope of the invention involved in the present application. Accordingly, the drawings and the description in the specification of the present application are merely exemplary, and not restrictive.

### BRIEF DESCRIPTION OF THE DRAWINGS

Specific features of the invention involved in the present application are set forth in the appended claims. The features and advantages of the invention involved in the present application can be better understood by referring to the exemplary embodiments detailed hereinafter and the accompanying drawings. A brief description of the drawings is as follows:
FIG. 1 shows the diarrhea photographs of typical mice in the chemotherapeutic agent groups of examples 1-229.
FIG. 2 shows partial results of diarrhea grading in the control group, the chemotherapeutic agent group, and the CDK inhibitor group of examples 1-229.
FIG. 3 shows partial results of diarrhea grading in the control group, the chemotherapeutic agent group, and the CDK inhibitor group of examples 230-271.
FIG. 4 shows the photographs showing the number and shape of feces in the control group, the chemotherapeutic agent group, and the CDK inhibitor group of examples 272-342.
FIG. 5 shows partial results of feces reduction rate in the control group, the chemotherapeutic agent group, and the CDK inhibitor group of examples 272-342.
FIG. 6 shows partial results of diarrhea grading in the control group, the chemotherapeutic agent group, the group of oral administration of CDK inhibitor and the group of intravenous injection of CDK inhibitor of examples 343-381.
FIG. 7 shows partial results of diarrhea grading in the control group, the chemotherapeutic agent group, the group of oral administration of CDK inhibitor and the group of other administration mode of CDK inhibitor of examples 382-405.
FIG. 8 shows partial results of feces reduction rate in the control group, the chemotherapeutic agent group, the group of oral administration of CDK inhibitor and the group of other administration mode of CDK inhibitor of examples 406-443.
FIG. 9 shows partial results of diarrhea grading in the control group, the chemotherapeutic agent group, and the CDK inhibitor group of examples 444-469.
FIG. 10 shows partial results of feces reduction rate in the control group, the chemotherapeutic agent group, and the CDK inhibitor group of examples 470-481.

### DETAILED DESCRIPTION

The embodiments of the present invention are described below by way of specific examples, and persons skilled in the art can readily appreciate other advantages and effects of the present invention from the disclosure of the present specification.

### Term Definition

In the present application, the term "CDK inhibitor" generally refers to any molecules that have been known in the art or will be found in the future and are capable of blocking, reducing or inhibiting the activity or function of CDK (cyclin-dependent kinase), including, but not limited to, a small molecular compound, a polynucleotide (e.g., DNA or RNA), and/or a polypeptide (e.g., an antibody or an antigen-binding portion thereof). The CDK inhibitor can act directly on CDK, e.g. through binding to CDK, or act indirectly, e.g. through interfering with the interaction between CDK and its ligand, inhibiting cyclin or inhibiting the activity of the substrate. CDK is a class of kinase in the protein kinase family, which can be used to regulate the cell cycle, and are also involved in the regulation of transcription, mRNA processing and the differentiation of nerve cells. The CDK in the present application may refer to an intact CDK or a kinase domain fragment, and also encompasses CDKs from various vertebrates (e.g., human, monkey, mouse, dog, rabbit, etc.). According to different bound cyclins, CDK can be divided into different types. In the present application, the CDK inhibitor can inhibit one or more of CDK1, CDK2, CDK3, CDK4, CDK5, CDK6, CDK7, CDK8, CDK9, CDK10, CLK, Cdc, CDK11, CDK12, CDK13 and CDK19. For example, there are four CDKs which are evidently involved in cell proliferation: CDK1, which mainly regulates the transition from phase G2 to phase M; CDK2, CDK4 and CDK6, which regulate the transition from phase G1 to phase S. The CDK inhibitor of the present application may include selective CDK inhibitors and broad-spectrum CDK inhibitors.

In the present application, the term "CDK2 inhibitor" generally refers to an inhibitor that acts on CDK2 in the CDK family. In all the members of CDK family, it mainly has an inhibition effect against CDK2, but not excluding the possibility of having inhibition effects on members of the CDK family other than CDK2, and also not excluding the possibility of having inhibition effects on other molecules besides the CDK family. Exemplary CDK2 inhibitors may include, but not limited to, A-674563, MK-8776 (SCH 900776), Dinaciclib (SCH727965), JNJ-7706621, R547, AZD5438, PHA-793887, BMS-265246, SU9516, SNS-032 (BMS-387032), Flavopiridol (Alvocidib), Flavopiridol (Alvocidib) HCl, Milciclib (PHA-848125), AT7519, P276-00, PHA-767491, Roscovitine (Seliciclib, CYC202), NU6027 and/or LDC000067.

In the present application, the term "CDK4 inhibitor" generally refers to an inhibitor that acts on CDK4 in the CDK family. In all the members of CDK family, it mainly has an inhibition effect against CDK4, but not excluding the possibility of having inhibition effects on members of the CDK family other than CDK4, and also not excluding the possibility of having inhibition effects on other molecules besides the CDK family. Exemplary CDK4 inhibitors may include, but not limited to, R547, LY2835219, Palbociclib (PD-0332991) HCl, Palbociclib (PD0332991) Isethionate, Flavopiridol (Alvocidib), Flavopiridol (Alvocidib) HCl, PHA-793887, P276-00, AT7519, Milciclib (PHA-848125), SU9516, BMS-265246, JNJ-7706621, SNS-032 (BMS-387032), LDC000067 and/or LEE011.

In the present application, the term "CDK6 inhibitor" generally refers to an inhibitor that acts on CDK6 in the CDK family. In all the members of CDK family, it mainly has an inhibition effect against CDK6, but not excluding the possibility of having inhibition effects on members of the CDK family other than CDK6, and also not excluding the possibility of having inhibition effects on other molecules besides the CDK family. Exemplary CDK6 inhibitors may include, but not limited to, LY2835219, Palbociclib (PD-0332991) HCl, Palbociclib (PD0332991) Isethionate, Flavopiridol (Alvocidib), Flavopiridol (Alvocidib) HCl, AT7519, JNJ-7706621, P276-00 and/or LEE011.

In the present application, the term "CDK9 inhibitor" generally refers to an inhibitor that acts on CDK9 in the CDK family. In all the members of CDK family, it mainly has an inhibition effect against CDK9, but not excluding the possibility of having inhibition effects on members of the CDK family other than CDK9, and also not excluding the possibility of having inhibition effects on other molecules besides the CDK family. Exemplary CDK9 inhibitors may include, but not limited to, LY2835219, Palbociclib (PD-0332991) HCl, Palbociclib (PD0332991) Isethionate, Flavopiridol (Alvocidib), Flavopiridol (Alvocidib) HCl, AT7519, JNJ-7706621, P276-00 and/or LEE011.

In the present application, the term "CDK2/4/6 inhibitor" generally refers to an inhibitor that acts on all of CDK2, CDK4 and CDK6 in the CDK family. In all the members of CDK family, it mainly has inhibition effects against CDK2, CDK4 and CDK6, but not excluding the possibility of having inhibition effects on members of the CDK family other than CDK2, CDK4 and CDK6, and also not excluding the possibility of having inhibition effects on other molecules besides the CDK family. It is not required that the CDK2/4/6 inhibitor has the same or similar effects against CDK2, CDK4 and CDK6. Exemplary CDK2/4/6 inhibitors may include, but not limited to, AT7519 HCl, Riviciclib hydrochloride (P276-00), Flavopiridol HCl, AT7519, JNJ-7706621 and/or Flavopiridol (L86-8275).

In the present application, the term "CDK4/6 inhibitor" generally refers to an inhibitor that acts on both CDK4 and CDK6 in the CDK family. In all the members of CDK family, it mainly has inhibition effects against CDK4 and CDK6, but not excluding the possibility of having inhibition effects on members of the CDK family other than CDK4 and CDK6, and also not excluding the possibility of having inhibition effects on other molecules besides the CDK family. It is not required that the CDK4/6 inhibitor has the same or similar effects against CDK4 and CDK6. Exemplary CDK4/6 inhibitors may include, but not limited to, Ribociclib succinate, Ribociclib hydrochloride, Palbociclib, Trilaciclib, G1T38, Abemaciclib, ON123300, AT7519 HCl, Ribociclib (LEE011), Abemaciclib mesylate (LY2835219), Riviciclib hydrochloride (P276-00), Palbociclib (PD0332991) Isethionate, Flavopiridol HCl, AT7519, JNJ-7706621, Flavopiridol (L86-8275) and/or Palbociclib (PD-0332991) HCl.

In the present application, the term "CDK4/6/9 inhibitor" generally refers to an inhibitor that acts on all of CDK4, CDK6 and CDK9 in the CDK family. In all the members of CDK family, it mainly has inhibition effects against CDK4, CDK6 and CDK9, but not excluding the possibility of having inhibition effects on members of the CDK family other than CDK4, CDK6 and CDK9, and also not excluding the possibility of having inhibition effects on other molecules besides the CDK family. It is not required that the CDK4/6/9 inhibitor has the same or similar effects against CDK4, CDK6 and CDK9. Exemplary CDK4/6/9 inhibitors may include, but not limited to, G1T38, AT7519 HCl, Riviciclib hydrochloride (P276-00), AT7519 and/or Flavopiridol (L86-8275).

In the present application, the term "chemotherapy" generally refers to a therapy that uses a chemotherapeutic agent to treat tumors, which can lead to the death of cancer cells, or interfere with the division, repair, growth and/or function of cancer cells. The medicament used in the chemotherapy is a chemotherapeutic agent. The chemotherapeutic agent comprises a chemical or biological substance which can cause the death of cancer cells or interfere with the growth, division, repair and/or function of cancer cells. For example, the chemotherapy may comprise cytotoxic agents, cell inhibitors and antitumor agents which can kill tumor cells, inhibit the growth or metastasis of tumor cells or disrupt the cell cycle of rapidly proliferating cells. The chemotherapeutic agent comprises natural compounds found in animals and plants, or artificial chemical substances. In the present application, the chemotherapy or chemotherapeutic agents have low selectivity on tumor cells and normal cells, so they may also interfere with the growth, division, repair and/or function of normal cells (e.g., bone marrow cells, hair follicle cells or digestive tract cells), finally resulting in the death of normal cells. The chemotherapeutic agents in the present application may not include tumor-targeted medicaments, i.e., the chemotherapeutic agents in the present application do not include substances which can specifically recognize tumor cells, tumor tissues, tumor organs or proteins specifically expressed by tumor cells (e.g., tumor-associated antigens or tumor specific antigens). Because of genetic mutation or other factors, some kinases or phosphoric acid kinases are activated, causing the reproduction of cancer cells. The chemotherapeutic agents of the present application do not include those kinase inhibitors which prevent the division of cancer cells by inhibiting abnormally activated kinases, either. The chemotherapeutic agents of the present application do not include antibodies and/or angiogenesis inhibitors, either.

In the present application, examples of the chemotherapeutic agents may include, but not limited to, alkylating agents, e.g., mechlorethamine, ethyleneimine compounds, alkyl sulfonates and other compounds having alkylating effects, e.g. nitrosourea, cisplatin and dacarbazine; antimetabolites, e.g., folic acid, purine or pyrimidine antagonists; mitotic inhibitors, e.g. vinca alkaloid and podophyllotoxin derivatives; cytotoxic antibiotics and camptothecin derivatives. The chemotherapeutic agents may also include amifostine cisplatin, dacarbazine (DTIC), dactinomycin, streptomycin, cyclophosphamide, carmustine (BCNU), lomustine (CCNU), doxorubicin, doxorubicin liposome, gemcitabine, erythromycin, daunorubicin liposome procarbazine, mitomycin, cytarabine, etoposide, methotrexate, 5-fluorouracil (5-FU), vinblastine, vincristine, bleomycin, paclitaxel, Docetaxel, Aldesleukin, Asparaginase, busulfan, carboplatin, cladribine, camptothecin, CPT-11, 10-hydroxyl-7-ethyl-camptothecin (SN38), floxuridine, fludarabine, hydroxyurea, ifosfamide, idarubicin, Mesna, interferon α, interferon β, irinotecan, mitoxantrone, topotecan, Lupron, megestrol, Melphalan, mercaptopurine, plicamycin, mitotane, Oncaspar, pegaspargase, pentostatin, pipobroman, plicamycin, streptozocin, tamoxifen, teniposide, testosterone, thioguanine, thiotepa, uramustine, vinorelbine, chlorambucil aromatase inhibitor and a combination thereof.

In the present application, the term "cytotoxic agent" generally refers to a reagent for inhibiting the biological process of cells or reducing the viability or proliferative potential of cells. The cytotoxic agent may act in various ways, for example, but not limited to, through inducing DNA damages, inducing cell cycle arrest, inhibiting DNA synthesis, inhibiting transcription, inhibiting translation or protein synthesis, inhibiting cell division or inducing apoptosis.

In the present application, the term "does not substantially affect" generally refers to that as compared to the therapeutic effect of using the chemotherapy alone, the use of the combination of the medicament and the chemotherapy of the present application has a comparable therapeutic effect, or does not produce a significant disadvantage. For example, for any subject, as compared to the therapeutic effect of using the chemotherapy alone, the use of the combination of the medicament and the chemotherapy causes the same degree of tumor size reduction, or the reduction degree is not less than about 5%, not less than about 4%, not less than about 3%, not less than about 2%, not less than about 1%, not less than about 0.5%, not less than about 0.1%, not less than about 0.01%, not less than about 0.001% or smaller.

In the present application, the term "continuous administration" generally refers to repeated administration of the same medicament every day. Repeated administration every day may be once a day, twice a day, 3 or more times a day. For example, continuous administration may be administration once a day for at least more than 2 days. For example, a continuous administration time of 2 days may mean administration once a day (twice, 3 or more times) continuously for 2 days. For example, a continuous administration time of 3 days may mean administration once a day (twice, 3 or more times) continuously for 3 days. For example, a continuous administration time of 5 days may mean administration once a day (twice, 3 or more times) continuously for 5 days. For example, a continuous administration time of 7 days may mean administration once a day (twice, 3 or more times) continuously for 7 days. For example, a continuous administration time of 10 days may mean administration once a day (twice, 3 or more times) continuously for 10 days. For example, a continuous administration time of 14 days may mean administration once a day (twice, 3 or more times) continuously for 14 days. For example, a continuous administration time of more than 7 days may mean administration once a day (twice, 3 or more times) continuously for more than 7 days.

In the present application, the term "noncontinuous administration" generally refers to an administration mode in which the same medicament is not administered every day in an administration cycle, also known as intermittent administration and/or pulsed administration. Noncontinuous administration may be regular or irregular. In noncontinuous administration, in the case that the administration cycle is longer than 7 days, for any continuous period of time, if the administration is performed every day and continued for 7 days or above, it also belongs to the case of "a continuous administration time of 7 days or above".

In the present application, the term "gastrointestinal adverse events" generally refers to harmful and undesirable reactions, effects, actions, consequences, results or influences associated with the gastrointestinal tract or displayed in the gastrointestinal tract area, which are caused by a certain medicament or other medical treatments such as chemotherapy or surgery. They are also known as gastrointestinal adverse effects, gastrointestinal adverse influences or gastrointestinal adverse consequences. The gastrointestinal adverse events comprise adverse events in various parts (e.g., digestive tracts and glands) of the digestive system. For example, gastrointestinal adverse events may include, but not limited to, abdominal distension, abdominal pain, anal fissure, anal fistula, anal bleeding, anal mucositis, anal necrosis, anal pain, anal stenosis, anal ulcer, ascites, hiccup, cecal bleeding, cheilitis, chylous ascites, colitis, colonic fistula, colonorrhagia, colonic obstruction, colonic perforation, colonic stricture, colonic ulcer, constipation, dental caries, diarrhea, xerostomia, duodenal fistula, duodenal bleeding, duodenal obstruction, duodenal perforation, duodenal stenosis, duodenal ulcer, dyspepsia, dysphagia, enterocolitis, intestinal fistula, esophageal fistula, esophageal bleeding, esophageal necrosis, esophageal obstruction, esophageal pain, esophageal perforation, esophageal stenosis, esophageal ulcer, esophageal variceal bleeding, oesophagitis, fecal incontinence, flatus, gastric fistula, gastrorrhagia, gastric necrosis, gastric perforation, gastric stenosis, gastric ulcer, gastritis, gastroesophageal reflux disease, gastrointestinal diseases (others, please noted), gastrointestinal fistula, gastrointestinal pain, gastroparesis, gum pain, hemorrhoidal bleeding, haemorrhoids, ileal fistula, ileal bleeding, ileocleisis, ileal perforation, ileal stenosis, ileal ulcer, ileus, intraperitoneal hemorrhage, jejunal fistula, jejunal bleeding, jejunal obstruction, jejunal perforation, jejunal stenosis, jejunal ulcer, chilalgia, lower gastrointestinal hemorrhage, malabsorption, oral mucositis, nausea, gastric obstruction, oral fistula, oral dysaesthesia, oral hemorrhage, oral pain, pancreatic-duct stenosis, pancreatic fistula, pancreatic bleeding, pancreatic necrosis, pancreatitis, periodontal diseases, peritoneal necrosis, rectitis, rectal rupture, rectofistula, rectal bleeding, rectal mucositis, rectal necrosis, rectal obstruction, rectal pain, rectal perforation, rectostenosis, rectal ulcer, retroperitoneal hemorrhage, salivary duct inflammation, salivary gland fistula, small bowel mucositis, small bowel obstruction, small bowel perforation, small bowel stenosis, small bowel ulcer, abdominal pain, dental developmental disturbance, dental stain, toothache, appendicitis, upper gastrointestinal hemorrhage, visceral artery ischemia, vomiting.

In the present application, the term "gastrointestinal side effects" generally refers to harmful and adverse effects associated with the gastrointestinal tract or displayed in the gastrointestinal tract area, which are caused by a preventive medicine or a therapeutic medicine. Adverse effects are always undesirable, but undesirable effects are not necessarily adverse. The adverse effects of the preventive medicine or the therapeutic medicine may be harmful, unsuitable or dangerous. Gastrointestinal side effects include side effects in various parts (e.g., digestive tract and gland) of the digestive system. Chemotherapy-associated gastrointestinal side effects may refer to any abnormal clinical manifestations of gastrointestinal tract associated with the time of using the chemotherapeutic agent, but there may be no causal relationship between these abnormal manifestations and the administration of the chemotherapeutic agent.

In the present application, the term "gastric mucosal injury diseases" generally refers to the diseases or disorders with symptoms of gastric mucosal injury, which can include abnormal color, bleeding spots, congestion and erosion of the gastric mucosa.

In the present application, the term "intestinal mucosal injury diseases" generally refers to the diseases or disorders with symptoms of intestinal mucosal injury, which can include abnormal color, bleeding spots, congestion and erosion of the intestinal mucosa.

In the present application, the term "diarrhea" generally refers to a disease or disorder with the features of increased peristalsis frequency and/or relaxation or watery peristalsis. In the present application, the diarrhea may occur or deteriorate after administration of the chemotherapeutic agent. In some cases, diarrhea can also be evaluated referring to animal experiments: for example, the severity of diarrhea is scored in accordance with the method of Akinobu Kurita (Cancer Chemother Pharmacol 2000; 46:211-20.), wherein, Grade 0: normal feces; Grade 1: mild diarrhea, the stools are slightly wet and soft; Grade 2: moderate diarrhea, loose stools and mild perianal contamination; Grade 3: severe diarrhea, watery stools and accompanied by severe perianal staining.

In the present application, the term "constipation" generally refers to a disease or disorder with the features of irregular defecation, infrequent defecation or difficult defecation of intestines. In the present application, the constipation may occur or deteriorate after administration of the chemotherapy.

In the present application, the term "in the absence of prevention or treatment" generally refers to a condition without performing prevention or treatment on gastrointestinal adverse events, and without taking measures to make the gastrointestinal adverse events to be weakened or disappear. The measures to make the gastrointestinal adverse events to be weakened or disappear may be, for example, administration of a medicament or other means to prevent or treat gastrointestinal adverse events, stopping the administration of medicaments or other means that cause gastrointestinal adverse events, including administration of the CDK inhibitor or the medicament of the present application. In the present application, after administering the chemotherapy of the present application, if no measures are taken to make the gastrointestinal adverse events to be weakened or disappear, the gastrointestinal adverse events will occur or deteriorate 1 hr, 2 hrs, 3 hrs, 4 hrs, 5 hrs, 6 hrs, 7 hrs, 8 hrs, 9 hrs, 10 hrs, 11 hrs, 12 hrs, 1 day, 2 days, 4 days, 7 days, 2 weeks, 3 weeks, 1 month, 2 months or longer after the administration of the chemotherapy. If the CDK inhibitor or the medicament of the present application is administered, the gastrointestinal adverse events may be weakened or disappear.

In the present application, the term "NCI-CTCAE" generally refers to a standardized definition of adverse events issued by National Cancer Institute (NCI)-Common Terminology Criteria for Adverse Events (CTCAE), which is used to describe the severity of organ toxicity in patients treated for cancer. With the development of scientific evidence, the criteria can be updated continuously. In the present application, diarrhea can be evaluated with reference to the evaluation criteria of "NCI-CTCAE" in some cases. NCI-CTCAE may comprise any version of "NCI-CTCAE". In CTCAE V5.0, constipation and diarrhea are defined into 5 grades respectively, as shown in Table 1 and Table 2.

**Table 1 Standardized definition on the grading of constipation in CTCAE V5.0**

| CTCAE Grading | Criteria |
|---|---|
| Grade 1 | Incidental or intermittent symptoms; occasionally employing stool softener, laxatives, antisecosis or enteroclysis |
| Grade 2 | Persistent symptoms in need of long-term use of laxatives or enteroclysis; limited instrumental activities of daily living (ADL) |
| Grade 3 | Constipation with manual evacuation indications; limited self-care |
| Grade 4 | Life-threatening consequences; indicating emergency interventions |
| Grade 5 | Death |

**Table 2 Standardized definition on the grading of diarrhea in CTCAE V5.0**

| CTCAE Grading | Criteria |
|---|---|
| Grade 1 | An increasement of < 4 times of defecation compared with the basic standard every day; compared with the baseline, the output of neostomy increases slightly |
| Grade 2 | An increasement of 4-6 times of defecation compared with the basic standard every day; compared with the baseline, the output of neostomy increases |
| | moderately; limited instrumental ADL |
| Grade 3 | An increasement of >= 7 times of defecation compared with the basic standard every day; indicating hospitalization; compared with the baseline, the output of neostomy increases significantly; limited self-care |
| Grade 4 | Life-threatening consequences; indicating emergency interventions |
| Grade 5 | Death |

In the present application, the term "local intestinal exposure", when used to describe a compound, generally means that the compound preferentially acts in the enteric cavity through a certain administration or delivery method, rather than being exposed fully to systemic circulation, also known as "intestinal limitation" or "intestinal limiting compound". Local intestinal exposure can reduce the systemic exposure of a compound or derivatives thereof, reduce the effects on cells, tissues and organs/organ systems which are not related with the desired disease treatment, thus improving the safety of molecules. The compound can be delivered locally or targeted to the intestinal tract by means of adjusting the type of the compound, the administration route, the dosage form, the administration mode, the administration dosage, and using auxiliary means such as devices, thereby achieving local intestinal exposure. The compound for local intestinal exposure has one or more of the following properties: (1) gut targeted exposure (due to the administration mode, etc.), and (2) the systemic medicament concentration is lower than the minimum inhibitory concentration (IC50). For example, administration may be through oral administration or an artificial route, e.g. intubation.

In the present application, the term "independently improving or reducing" generally means that without the aid of other medicaments or therapies, the gastrointestinal side effects can be improved or reduced by only using the medicament or pharmaceutical composition of the present application.

In the present application, the term "cancer" generally refers to any medical conditions mediated by the growth, proliferation or metastasis of tumors or malignant cells and causing solid tumors and nonsolid tumors (e.g., leukemia). The cancer in the present application may include, but not limited to, malignant epithelial tumors (epithelium-derived cancers), lung cancer (e.g., non-small cell lung cancer), breast cancer, skin cancer, bladder cancer, colon cancer, gastrointestinal (GI) cancer, prostatic cancer, pancreatic cancer, uterine cancer, cervical cancer, ovarian cancer, esophagus cancer, head and neck cancer, gastric cancer and throat cancer.

### Detailed description of the invention

In one aspect, the present application provides a use of a CDK inhibitor in preparing a medicament, which is used for preventing or treating chemotherapy-associated gastrointestinal side effects in a subject. In another aspect, the present application provides a method of preventing, relieving and/or treating chemotherapy-associated gastrointestinal side effects in a subject, comprising administering the CDK inhibitor to a subject in need thereof.

### CDK inhibitors

In the present application, the CDK inhibitor can directly bind to the domain of a CDK kinase to block the activity of the kinase; or occupies the ligand-binding site of a CDK ligand or a part thereof, or occupies the CDK binding site of cyclin or a part thereof, so as to reduce or block the biological activity of CDK.

In the present application, the CDK inhibitors may be nonspecific inhibitors of CDK, that is, these inhibitors also inhibit other targets except CDK.

In the present application, the CDK inhibitor acts directly on a CDK protein or a nucleic acid encoding a CDK protein. In some embodiments, the CDK inhibitor acts directly on a CDK protein. In the present application, when describing an inhibitor and a target protein, the term "act directly on" generally means that the inhibitor may directly bind to the target protein without the aid of any other molecules (including covalently binding and non-covalently binding). In some embodiments, the CDK inhibitor may be a small molecular EGFR inhibitor, a protein macromolecule that binds specifically to EGFR, an RNAi or an antisense oligonucleotide that inhibits expression of EGFR proteins. In some embodiments, the CDK inhibitor may be a small molecular CDK inhibitor.

In the present application, the CDK inhibitor may be an ATP competitive inhibitor, an allosteric site inhibitor, a covalent inhibitor and a non-ATP competitive polypeptide mimic.

For example, the CDK inhibitor may be a CDK inhibitor that inhibits one or more selected from CDK1, CDK2, CDK3, CDK4, CDK5, CDK6, CDK7, CDK9, CLK, Cdc, CDK8, CDK10, CDK12, CDK13, CDK19 and CDK11. For example, the CDK inhibitor may be a CDK4/6 inhibitor that is capable of inhibiting CDK6 and CDK4 at the same time. For example, the CDK inhibitor may be a CDK2/4/6 inhibitor that is capable of inhibiting CDK2, CDK4 and CDK6 at the same time. For example, the CDK inhibitor may be a CDK4/6/9 inhibitor that is capable of inhibiting CDK9, CDK4 and CDK6 at the same time.

For example, exemplary CDK inhibitors may include but not limited to, Trilaciclib, Palbociclib, Ribociclib, Abemaciclib, FLX-925, SHR-6390, BPI-1178, BPI-16350, FCN 437, G2T28, XZP-3287, BEBT-209, TY-302, TQB-3616, HS-10342, PF-06842874, CS-3002, MM-D37K, zotiraciclib, XZP-3287, Rigosertib, KRX-0601, Riviciclib, roniciclib, Milciclib, Seliciclib, Roscovitine, Indisulam, Alvocidib, NUV-422, BEY-1107, GLR-2007, FN-1501, BCD-115, TP-1287, BAY-1251152, Atuveciclib, SEL-120, HX-301, Voruciclib, Fadraciclib, AGM-130, PHA-793887, PHA-690509, Dinaciclib, RO4584820, R547, AT-7519, RGB-286638, ZK-304709, IIIM-290, PF-07104091 and G1T38, and other salts, salt forms, crystal forms, solvates and prodrugs thereof.

The CDK inhibitor may be identified or screened by well-known methods in the art, e.g., by detecting the changes in the expression level of CDK or the activity of kinase after administering the compound to be tested. The expression level of CDK or the activity of kinase may be detected by well-known methods in the art, e.g., immunohistochemistry, PCR, RT-PCR, in situ hybridization, Southern blot, Western blot, Northern blot, spectrophotometry and ELISA, etc.

### Chemotherapy

In the present application, the chemotherapeutic agent used in the chemotherapy may be any one kind of compound or medicament used for treating cancers selected from those known to persons skilled in the art. In terms of the mechanism of action, the chemotherapeutic agent may comprise a DNA synthesis inhibitor, an RNA synthesis inhibitor, a protein synthesis inhibitor, a cell division inhibitor, a DNA base analogue, a topoisomerase inhibitor and/or a telomerase synthesis inhibitor.

In the present application, the chemotherapeutic agent may be toxic to cells. In the present application, the chemotherapeutic agent may inhibit cell growth. In the present application, the administered chemotherapeutic agent may be a chemotherapeutic agent for DNA damages. In the present application, the chemotherapeutic agent is a protein synthesis inhibitor, a DNA-damage chemotherapeutic agent, an alkylating agent, a topoisomerase inhibitor, a RNA synthesis inhibitor, a DNA composite binding agent, a thiolate alkylating agent, a guanine alkylating agent, a tubulin binding agent, a DNA polymerase inhibitor, an anti-cancer enzyme, a RAC1 inhibitor, a thymidylic acid synthetase inhibitor, an oxazophosphorine compound, an integrin inhibitor, such as cilengitide, camptothecin or homocamptothecin, an antifolate, a folic acid antimetabolite, a telomerase inhibitor and/or a telomere DNA-binding compound.

For example, the alkylating agent may comprise alkyl sulfonates, such as busulfan, improsulfan and piposulfan; aziridines, such as benzodizepa, carboquone, meturedepa and uredepa; ethylene imines and methyl melamines, such as altretamine, triethylene melamine, triethylene phosphamide, triethylene thiophosphamide and trimethylol melamine; mechlorethamines, such as chlorambucil, chlomaphazine, cyclophosphamide, estramustine, dichloromethyl diethylamine, mechlorethamine oxide hydrochloride, Melphalan, novembichine, phenesterine, prednimustine, trofosfamide and uracil mustard; and nitrosoureas, such as carmustine, chlorozotocin, fotemustine, lomustine, nimustine and ranimustine. Other chemotherapeutic agents may comprise daunorubicin, doxorubicin, idarubicin, epirubicin, mitomycin and streptozotocin. Antimetabolites for the chemotherapy may comprise gemcitabine, mercaptopurine, thioguanine, cladribine, fludarabine phosphate, fluorouracil (5-FU), floxuridine, cytarabine, pentostatin, methotrexate, azathioprine, acyclovir, adenine β-1-D-arabinoside, amethopterin, aminopterin, 2-aminopurine, aphidicolin, 8-azaguanine, azaserine, 6-azauracil, 2'-azido-2'-deoxynucleoside, 5-bromodeoxycytidine, cytosine β-1-D-arabinoside, diazoxonorleucine, dideoxynucleoside, 5-fluorodeoxycytidine, 5-fluorodeoxyuridine and hydroxyurea.

For example, the protein synthesis inhibitor may comprise abrin, aurin tricarboxylic acid, chloramphenicol, colicin E3, cycloheximide, diphtheria toxin, edeine A, emetine, erythromycin, ethionine, fluoride, 5-fluorotryptophan, fusidic acid, guanylyl methylene bisphosphonate and guanylyl imino diphosphate, kanamycin, kasugamycin, kirromycin and O-methylthreonine. Other protein synthesis inhibitors comprise modeccin, neomycin, norvaline, pactamycin, paromomycine, puromycin, ricin, Shiga toxin, showdomycin, sparsomycin, spectinomycin, streptomycin, tetracycline, thiostreptone and trimethoprim.

For example, the DNA synthesis inhibitor may comprise an alkylating agent, such as dimethyl sulfate, mechlorethamine and sulfur mustard; an intercalating agent, such as acridine dyes, actinomycin, anthracenes, benzopyrene, ethidium bromide, propidium diiodide-intertwining; a topoisomerase inhibitor, such as irinotecan, teniposide, coumermycin, nalidixic acid, novobiocin and oxolinic acid; a cell division inhibitor, including demecolcine, mitoxantrone, colchicine, vinblastine and vincristine; and other medicaments, such as distamycin and netropsin.

In the present application, the chemotherapeutic agent may be a DNA composite binding agent, e.g., camptothecin or etoposide; a thiolate alkylating agent, e.g., nitrosourea, BCNU, CCNU, ACNU or fotesmustine; a guanine alkylating agent, e.g., temozolomide; a tubulin binding agent, such as vinblastine, vincristine, vinorelbine, vinflunine, cryptophycin 52, halichondrin such as halichondrin B, aplysiatoxin such as aplysiatoxin 10 and aplysiatoxin 15, hemiasterlins (such as hemiasterlin A and hemiasterlin B), colchicine, combrestatins, 2-methoxy estradiol, E7010, paclitaxel, Docetaxel, epothilone, discodermolide; a DNA polymerase inhibitor, e.g., cytarabine; an anti-cancer enzyme, e.g., asparaginase; a RAC1 inhibitor, e.g., 6-thioguanine; a thymidylic acid synthetase inhibitor, e.g., capecitabine or 5-FU; an oxazophosphorine compound, e.g., Endoxan; an integrin inhibitor, e.g., cilengitide; an antifolate, e.g., pralatrexate; a folic acid antimetabolite, e.g., pemetrexed; or camptothecin or homocamptothecin, e.g., diflomotecan.

In the present application, the CDK inhibitor can be used for treating the above one or more chemotherapy-associated gastrointestinal side effects. In the present application, the CDK inhibitor can be used for treating gastrointestinal side effects associated with the administration of the following chemotherapeutic agents: fluorouracil, oxaliplatin, irinotecan (CPT-11), docetaxel (DTX), gemcitabine (GEM), paclitaxel, carboplatin, doxorubicin (Dox), methotrexate (MTX), cytarabine (Ara-C), vinorelbine (NVB), topotecan (TP), etoposide and cisplatin, and any combination of the above.

In the present application, the CDK inhibitor can be used for treating gastrointestinal side effects associated with the administration of the following chemotherapeutic agents: fluorouracil, oxaliplatin, topotecan, irinotecan, tetrahydrofolic acid, docetaxel, gemcitabine, carboplatin, cisplatin, etoposide, methotrexate, doxorubicin, cytarabine, vinorelbine and capecitabine, and any combination of the above.

In the present application, the CDK inhibitor can be used for treating gastrointestinal side effects associated with the administration of the following chemotherapeutic agents: fluorouracil, oxaliplatin, irinotecan, docetaxel, gemcitabine, carboplatin, methotrexate, doxorubicin, cytarabine, vinorelbine and capecitabine, and any combination of the above.

In the present application, the chemotherapeutic agent may not include a tumor-targeted medicament. In the present application, the chemotherapeutic agent may not include those kinase inhibitors which prevent the division of cancer cells by inhibiting abnormally activated kinases. In the present application, the chemotherapeutic agent may not include an antibody and/or an angiogenesis inhibitor.

In the present application, the chemotherapy may be administered continuously. For example, the chemotherapy may be administered continuously for 2 days, 3 days, 4 days, 5 days, 6 days, 7 days, 8 days, 9 days or more days. In some embodiments, the days for the continuous administration of the chemotherapy are no more than 7 days. For example, the chemotherapy may be administered continuously for 2 days, 3 days, 4 days, 5 days or 6 days. In the present application, the administration frequency of the chemotherapy may be once a day, twice a day, 3 times a day or others.

In the present application, the chemotherapy may be administered noncontinuously. In the present application, the administration frequency of the chemotherapy may be once every two days, once every three days, twice every three days or others.

In the present application, the administration frequency of the chemotherapeutic agent may be once a day, and the administration may be continued for less than 7 days and/or no more than 7 days.

In the present application, the administration cycle of the chemotherapeutic agent may be 3 days, 5 days, 7 days, 2 weeks, 20 days, 1 month, 2 months or longer.

In the present application, one or more different chemotherapeutic agents may be used. In the present application, two or more different chemotherapeutic agents may be used in combination. In some embodiments, the CDK inhibitor may be used in combination with one or more other cancer therapies. The other cancer therapies may be common methods used for treating cancers in the art, e.g., cytotoxic anticancer agent, immunotherapeutic anticancer agent or hormone therapy anticancer agent. In accordance with the present application, the medicament used for treating a cancer may also be used in combination with radiotherapy or surgery. In some embodiments, in the case of the combination use of a CDK inhibitor and other anticancer agents, they can be administered to the subject at the same time, or separately administered at a certain interval.

### Gastrointestinal side effects

In the present application, the CDK inhibitor can prevent, relieve and/or treat gastrointestinal side effects associated with the administration of chemotherapy in a subject. In the present application, chemotherapy-associated gastrointestinal side effects may mean that the gastrointestinal side effects are caused by the administration of the chemotherapy and will occur or deteriorate after the administration of the chemotherapeutic agent. In the absence of prevention or treatment, the gastrointestinal side effects will occur or deteriorate 1 hr, 2 hrs, 3 hrs, 4 hrs, 5 hrs, 6 hrs, 7 hrs, 8 hrs, 9 hrs, 10 hrs, 11 hrs, 12 hrs, 1 day, 2 days, 4 days, 7 days, 2 weeks, 3 weeks, 1 month, 2 months or longer after the administration of the chemotherapeutic agent.

In some embodiments, before administering the chemotherapeutic agent to the subject, the subject does not develop the gastrointestinal side effects; after administering the chemotherapeutic agent to the subject, the subject develops the gastrointestinal side effects.

In some embodiments, before administering the chemotherapeutic agent to the subject, the subject has developed the gastrointestinal side effects; after administering the chemotherapeutic agent to the subject, the severity of the gastrointestinal side effects in the subject increases.

In the present application, after the administration of the chemotherapeutic agent, the symptoms of gastrointestinal side effects in the subject may deteriorate by at least about 10%, e.g., by about 15%, about 20%, about 25%, about 30%, about 35%, about 40%, about 45%, about 50%, about 55%, about 60%, about 65%, about 70%, about 75%, about 80%, about 85%, about 90%, about 95%, about 99% or higher.

For example, in accordance with the criteria of NCI-CTCAE, after administering the chemotherapeutic agent to the subject, the severity of the gastrointestinal side effects (e.g., diarrhea or constipation) in the subject may increase from Grade 1 to Grade 2, from Grade 1 to Grade 3, from Grade 1 to Grade 4, from Grade 1 to Grade 5, from Grade 2 to Grade 3, from Grade 2 to Grade 4, from Grade 2 to Grade 5, from Grade 3 to Grade 4, from Grade 3 to Grade 5 or from Grade 4 to Grade 5. For example, in accordance with the method of Akinobu Kurita, after administering the chemotherapeutic agent to the subject, the scoring of constipation in the subject increases from Grade 0 to Grade 1, from Grade 0 to Grade 2, from Grade 0 to Grade 3, from Grade 1 to Grade 2, from Grade 1 to Grade 3 or from Grade 2 to Grade 3.

In some embodiments, the gastrointestinal side effects comprise gastric mucosal injury diseases and/or intestinal mucosal injury diseases.

In some embodiments, the gastrointestinal side effects comprise diarrhea, abdominal pain, nausea, vomiting, mucositis, loss of appetite, gastric ulcer, gastritis, constipation, enteritis, intestinal perforation, intestinal bleeding, ulcer and/or intestinal necrosis. In some embodiments, the gastrointestinal side effects comprise abnormal defecation. In some embodiments, the gastrointestinal side effects comprise diarrhea and/or constipation.

In some embodiments, the chemotherapy-associated gastrointestinal side effects comprise chemotherapy-associated gastric mucosal injury diseases and/or chemotherapy-associated intestinal mucosal injury diseases.

In some embodiments, the chemotherapy-associated gastrointestinal side effects comprise chemotherapy-associated diarrhea, chemotherapy-associated abdominal pain, chemotherapy-associated nausea, chemotherapy-associated vomiting, chemotherapy-associated mucositis, chemotherapy-associated loss of appetite, chemotherapy-associated gastric ulcer, chemotherapy-associated gastritis, chemotherapy-associated constipation, chemotherapy-associated enteritis, chemotherapy-associated intestinal perforation, chemotherapy-associated intestinal bleeding, chemotherapy-associated ulcer and/or chemotherapy-associated intestinal necrosis. In some embodiments, the chemotherapy-associated gastrointestinal side effects comprise chemotherapy-associated abnormal defecation. In some embodiments, the chemotherapy-associated gastrointestinal side effects comprise chemotherapy-associated diarrhea and/or chemotherapy-associated constipation.

In the present application, after administration of the CDK inhibitor of the present application, the severity of chemotherapy-associated gastrointestinal side effects in the subject is relieved. In the present application, the relief may generally mean that the occurrence or development of the gastrointestinal side effects in the subject is delayed. In the present application, after administration of the CDK inhibitor, the symptoms of gastrointestinal side effects in the subject may be ameliorated. In the present application, after administration of the CDK inhibitor, the symptoms of gastrointestinal side effects in the subject may be ameliorated by at least about 10%, e.g., by about 15%, about 20%, about 25%, about 30%, about 35%, about 40%, about 45%, about 50%, about 55%, about 60%, about 65%, about 70%, about 75%, about 80%, about 85%, about 90%, about 95%, about 99% or more.

For example, in accordance with the criteria of NCI-CTCAE, after administration of the CDK inhibitor, the symptoms of gastrointestinal side effects (e.g., diarrhea and/or constipation) in the subject may decrease from Grade 5 to Grade 4, from Grade 5 to Grade 3, from Grade 5 to Grade 2, from Grade 5 to Grade 1, from Grade 4 to Grade 3, from Grade 4 to Grade 2, from Grade 4 to Grade 1, from Grade 3 to Grade 2, from Grade 3 to Grade 1 or from Grade 2 to Grade 1.

Alternatively, in accordance with the method of Akinobu Kurita, after administration of the CDK inhibitor, the scoring of diarrhea in the subject may decrease from Grade 3 to Grade 2, from Grade 3 to Grade 1, from Grade 3 to Grade 0, from Grade 2 to Grade 1, from Grade 2 to Grade 0 or from Grade 1 to Grade 0.

In the present application, after administration of the CDK inhibitor, the symptoms of gastrointestinal side effects in the subject may be eliminated. However, the case that the gastrointestinal side effects recur or deteriorate again after stopping the administration of the CDK inhibitor may not be excluded.

### Therapeutic method

The term "prevention" as used herein generally refers to the prevention of occurrence, recurrence, or spread of diseases or one or more symptoms thereof. In the context of the present application, the "prevention" may be interchangeably used with the "preventive treatment". In some embodiments, the "prevention" generally refers to the treatment of providing a patient suffering from the diseases or disorders as described in the present application with the medicament in accordance with the present application with or without administration of other medicaments as described in the present application prior to the occurrence of any symptoms. In some embodiments, patients with a family history of a particular disease can be the candidates of a prevention program. In some embodiments, patients with a history of recurring symptoms are also potential objects of prevention.

The term "treatment" as used herein generally refers to eliminating or improving diseases or one or more symptoms associated with the diseases. In some embodiments, the treatment generally refers to eliminating or ameliorating a disease by administering one or more therapeutic agents to the patients suffering from the disease. In some embodiments, the "treatment" may be administering a medicament in the presence or absence of other therapeutic agent(s) after the occurrence of symptoms of a particular disease.

The term "subject" as used herein generally refers to a human or non-human animal (including mammals) in need of diagnosing, prognosing, improving, preventing, relieving and/or treating diseases, especially those in need of treatment or prevention by using the CDK inhibitor. In some embodiments, the subject may comprise a cancer patient. For example, the cancer patient may have been, is being, and/or will be administered with the chemotherapy.

In some embodiments, the subject may be a human or a non-human mammal. The non-human mammals may include any mammalian species other than human, e.g., livestocks (e.g., cow, pig, sheep, chick, rabbit or horse), or rodents (e.g., rat and mouse), or primates (e.g., gorilla and monkey), or domestic animals (e.g., dog and cat). The "subject" may be male or female, and also may be at different ages.

The term "effective amount" as used herein generally refers to an amount of medicament capable of ameliorating or eliminating diseases or symptoms of the subject, or preventively inhibiting or prohibiting the occurrence of diseases or symptoms. The effective amount may be an amount of medicament capable of ameliorating one or more diseases or symptoms to some extent in the subject; an amount of medicament capable of partially or completely restoring one or more physiological or biochemical parameters associated with the causes of diseases or symptoms to normal; and/or an amount of medicament capable of reducing the possibility of the occurrence of diseases or symptoms.

In the present application, before the administration of the chemotherapy, for example, 0.5 hrs, 1 hr, 2 hrs, 3 hrs, 4 hrs, 5 hrs, 6 hrs, 7 hrs, 8 hrs, 9 hrs, 10 hrs, 11 hrs, 12 hrs, 13 hrs, 14 hrs, 15 hrs, 16 hrs, 17 hrs, 18 hrs, 19 hrs, 20 hrs or longer before the administration of the chemotherapy, the medicament or the CDK inhibitor can be administered to prevent, relieve and/or treat the occurrence or deterioration of the gastrointestinal side effects. For example, the medicament or the CDK inhibitor is administered 0.5-12 hrs before the administration of the chemotherapy to prevent, relieve and/or treat the occurrence or deterioration of the gastrointestinal side effects.

In the present application, the administration site of the CDK inhibitor may be or not be the occurrence site of cancer or the potential metastatic site of cancer.

The CDK inhibitor of the present application may be administered in a manner known in the art, e.g., by injection (e.g., subcutaneous, intraperitoneal, intraarticular, intraarterial, intrathecal, intrasternal, intrathecal, intralesional, intracranial, intramuscular, intracutaneous and intravenous injection or infusion) or non-injection (e.g., oral, nasal, sublingual, vaginal, rectal, or topical administration). The CDK inhibitor of the present application may be administered in a form of a pharmaceutical combination or a kit. In some embodiments, the CDK inhibitor of the present application may be administered in the same administration route as that of the chemotherapy or in a different route.

In the present application, the medicament and/or the CDK inhibitor may be prepared for oral administration. In the present application, the CDK inhibitor may preferentially act in the enteric cavity, or may preferentially get into the enteric cavity, rather than being exposed to the systemic circulation. The CDK inhibitor may be prepared as a dosage form suitable for being delivered into the enteric cavity or taking effects in the enteric cavity (e.g., the effects of preventing, relieving and/or treating gastrointestinal side effects). The CDK inhibitor can be administered in a route or manner that is suitable for delivering it into the enteric cavity or facilitating it to take effects in the enteric cavity, or auxiliary means such as devices can also be used to deliver the CDK inhibitor into the enteric cavity or make it to take effects in the enteric cavity. In the present application, the systemic medicament concentration of the CDK inhibitor is lower than the minimum inhibitory concentration (IC50).

In the present application, the medicament and/or the CDK inhibitor may be prepared for gastrointestinal administration, e.g., powders, tablets, granules, capsules, solution, emulsion and/or suspension. In the present application, the medicament and/or the CDK inhibitor may be prepared as a dosage form suitable for translumenal administration, e.g., suppositories and/or drop pills. In the present application, the medicament and/or the CDK inhibitor may be prepared a dosage form suitable for being delivered to the gastrointestinal tract by an artificial auxiliary means, e.g., by intubation.

In the present application, the medicament and/or the CDK inhibitor is prepared as a dosage form suitable for gastrointestinal exposure. The dosage form suitable for gastrointestinal exposure may be a dosage form suitable for being delivered to the gastrointestinal tract, a dosage form suitable for gastrointestinal administration, a dosage form suitable for translumenal administration, a dosage form suitable for oral administration and/or a dosage form suitable for being delivered to the gastrointestinal tract by an artificial auxiliary means.

In the present application, the administration dosage of the CDK inhibitor may be about 0.01-1000 mg/kg, for example, may be about 0.01-800 mg/kg, about 0.01-900 mg/kg, about 0.01-800 mg/kg, about 0.01-700 mg/kg, about 0.01-600 mg/kg, about 0.01-500 mg/kg, about 0.01-400 mg/kg, about 0.01-300 mg/kg, about 0.01-200 mg/kg, about 0.01-100 mg/kg, about 0.1-1000 mg/kg, about 1-1000 mg/kg, about 10-1000 mg/kg, about 50-1000 mg/kg, about 100-1000 mg/kg, about 0.1-800 mg/kg, about 1-600 mg/kg, about 10-500 mg/kg, about 10-400 mg/kg, about 15-300 mg/kg, about 50-250 mg/kg or about 50-200 mg/kg.

For example, the administration dosage of the CDK inhibitor in oral administration may be about 0.01-1000 mg/kg, for example, may be about 0.01-800 mg/kg, about 0.01-900 mg/kg, about 0.01-800 mg/kg, about 0.01-700 mg/kg, about 0.01-600 mg/kg, about 0.01-500 mg/kg, about 0.01-400 mg/kg, about 0.01-300 mg/kg, about 0.01-200 mg/kg, about 0.01-100 mg/kg, about 0.1-1000 mg/kg, about 1-1000 mg/kg, about 10-1000 mg/kg, about 50-1000 mg/kg, about 100-1000 mg/kg, about 0.1-800 mg/kg, about 1-600 mg/kg, about 10-500 mg/kg, about 10-400 mg/kg, about 15-300 mg/kg, about 50-250 mg/kg or about 50-200 mg/kg.

A certain dosage may be administered intervals in many times, e.g. once a day, twice a day or more times a day, once a week, once every two weeks, once every three weeks, once a month or once every two or more months. In some embodiments, the administration dosage may vary with the course of treatment. For example, in some embodiments, the initial administration dosage may be higher than the subsequent administration dosage. In some embodiments, the administration dosage is adjusted in the course of treatment in accordance with the response of the administration object. When being used to improve the conditions of the subject, the CDK inhibitor of the present application may be administered at a maintenance dose as needed. Then, the dosage or frequency of administration or both can be reduced to a level for maintaining the improved conditions when the symptoms are ameliorated to the desired level. In some embodiments, the medicament may be administered at intervals depending on the conditions of disease in the subject.

For example, the CDK inhibitor may be administered about 0.5 h-about 24 h before the administration of the chemotherapeutic agent, may be administered orally, and the administration dosage may be about 30-200 mg/kg, the administration cycle may be once a week to once every two weeks.

For example, the CDK inhibitor may be administered about 0.5 h-about 24 h before the administration of the chemotherapeutic agent, may be administered orally, and the administration dosage may be about 50-200 mg/kg, the administration cycle may be once every 1-2 weeks.

For example, the CDK inhibitor may be administered about 0.5 h-about 24 h before the administration of the chemotherapeutic agent, may be administered orally, and the administration dosage may be about 30-200 mg/kg, the administration cycle may be once every 7 days, once every 8 days, once every 9 days or once every 10 days.

For example, the CDK inhibitor may be administered about 0.5 h-about 12 h before the administration of the chemotherapeutic agent, may be administered orally, and the administration dosage may be about 50-200 mg/kg, the administration cycle may be once every 7 days, once every 8 days, once every 9 days or once every 10 days.

For example, the CDK inhibitor may be administered about 0.5 h-about 12 h before the administration of the chemotherapeutic agent, may be administered orally, and the administration dosage may be about 50-100 mg/kg, the administration cycle may be once every 7 days, once every 8 days, once every 9 days or once every 10 days.

For example, the CDK inhibitor may be administered about 0.5 h-about 12 h before the administration of the chemotherapeutic agent, may be administered orally, and the administration dosage may be about 100-200 mg/kg, the administration cycle may be once every 7 days, once every 8 days, once every 9 days or once every 10 days.

For example, the CDK inhibitor may be administered about 0.5 h-about 24 h before the administration of the chemotherapeutic agent, may be administered by injection, and the administration dosage may be about 30-200 mg/kg, the administration cycle may be once a week to once every two weeks.

For example, the CDK inhibitor may be administered about 0.5 h-about 12 h before the administration of the chemotherapeutic agent, may be administered by injection, and the administration dosage may be about 50-200 mg/kg, the administration cycle may be once every 7 days, once every 8 days, once every 9 days or once every 10 days.

In the present application, palbociclib can be used for treating gastrointestinal side effects (e.g., diarrhea or constipation) associated with the administration of a chemotherapeutic agent. For example, the palbociclib may be administered about 0.5 h-about 12 h before the administration of the chemotherapeutic agent, the palbociclib may be administered orally, and the administration dosage may be about 10-200 mg/kg. The chemotherapeutic agent may be administered orally or by injection. In the present application, palbociclib can be used for treating gastrointestinal side effects (e.g., diarrhea or constipation) associated with the administration of fluorouracil (5-Fu). For example, the palbociclib may be administered about 0.5 h-about 12 h before the administration of fluorouracil (5-Fu), the palbociclib may be administered orally, and the administration dosage may be about 125 mg/kg-about 150 mg/kg. For example, fluorouracil (5-Fu) may be administered orally or by injection.

In the present application, palbociclib can be used for treating gastrointestinal side effects (e.g., diarrhea or constipation) associated with the administration of oxaliplatin. For example, the palbociclib may be administered about 0.5 h-about 12 h (e.g., about 6 h) before the administration of oxaliplatin, the palbociclib may be administered orally, and the administration dosage may be about 125 mg/kg-about 150 mg/kg. For example, oxaliplatin may be administered by injection.

In the present application, palbociclib can be used for treating gastrointestinal side effects (e.g., diarrhea or constipation) associated with the administration of irinotecan. For example, the palbociclib may be administered about 0.5 h-about 24 h before the administration of irinotecan, the palbociclib may be administered orally, and the administration dosage may be about 50 mg/kg-about 200 mg/kg. For example, irinotecan may be administered by injection.

In the present application, palbociclib can be used for treating gastrointestinal side effects (e.g., diarrhea or constipation) associated with the administration of gemcitabine. For example, the palbociclib may be administered about 0.5 h-about 12 h before the administration of gemcitabine, the palbociclib may be administered orally, and the administration dosage may be about 50 mg/kg-about 200 mg/kg. For example, gemcitabine may be administered by injection.

In the present application, palbociclib can be used for treating gastrointestinal side effects (e.g., diarrhea or constipation) associated with the administration of docetaxel. For example, the palbociclib may be administered about 0.5 h-about 12 h before the administration of docetaxel, the palbociclib may be administered orally, and the administration dosage may be about 150 mg/kg-about 200 mg/kg. For example, docetaxel may be administered by injection.

In the present application, palbociclib can be used for treating gastrointestinal side effects (e.g., diarrhea or constipation) associated with the combined administration of gemcitabine and paclitaxel. For example, the palbociclib may be administered about 0.5 h-about 12 h before the combined administration of gemcitabine and paclitaxel, the palbociclib may be administered orally, and the administration dosage may be about 150 mg/kg-about 200 mg/kg. For example, gemcitabine and paclitaxel may be administered by injection.

In the present application, palbociclib can be used for treating gastrointestinal side effects (e.g., diarrhea or constipation) associated with capecitabine. For example, the palbociclib may be administered about 0.5 h-about 12 h before the administration of capecitabine, the palbociclib may be administered orally, and the administration dosage may be about 150 mg/kg-about 200 mg/kg. For example, capecitabine may be administered by injection.

In the present application, palbociclib can be used for treating gastrointestinal side effects (e.g., diarrhea or constipation) associated with the combined administration of etoposide and carboplatin. For example, the palbociclib may be administered about 0.5 h-about 12 h before the combined administration of etoposide and carboplatin, the palbociclib may be administered orally, and the administration dosage may be about 150 mg/kg-about 200 mg/kg. For example, etoposide and carboplatin may be administered by injection.

In the present application, ribociclib can be used for treating chemotherapy-associated gastrointestinal side effects (e.g., diarrhea or constipation). For example, the ribociclib may be administered about 0.5 h-about 24 h before the administration of the chemotherapeutic agent, the ribociclib may be administered orally, and the administration dosage may be about 20 mg/kg-about 200 mg/kg. For example, the chemotherapeutic agent may be administered by injection or orally.

In the present application, ribociclib can be used for treating gastrointestinal side effects (e.g., diarrhea or constipation) associated with fluorouracil (5-FU). For example, the ribociclib may be administered about 0.5 h-about 12 h before the administration of fluorouracil (5-FU), the ribociclib may be administered orally, and the administration dosage may be about 150 mg/kg-about 200 mg/kg. For example, fluorouracil (5-FU) may be administered by injection.

In the present application, ribociclib can be used for treating gastrointestinal side effects (e.g., diarrhea or constipation) associated with irinotecan. For example, the ribociclib may be administered about 0.5 h-about 24 h before the administration of irinotecan, the ribociclib may be administered orally, and the administration dosage may be about 100 mg/kg-about 200 mg/kg. For example, irinotecan may be administered orally.

In the present application, ribociclib can be used for treating gastrointestinal side effects (e.g., diarrhea or constipation) associated with the combined administration of etoposide and carboplatin. For example, the ribociclib may be administered about 0.5 h-about 12 h before the administration of etoposide and carboplatin, the ribociclib may be administered orally, and the administration dosage may be about 50 mg/kg-about 200 mg/kg. For example, etoposide and carboplatin may be administered by injection.

In the present application, ribociclib can be used for treating gastrointestinal side effects (e.g., diarrhea or constipation) associated with the administration of docetaxel (DTX). For example, the ribociclib may be administered about 0.5 h-about 24 h before the administration of docetaxel (DTX), the ribociclib may be administered orally, and the administration dosage may be about 100 mg/kg-about 200 mg/kg. For example, docetaxel (DTX) may be administered by injection.

In the present application, abemaciclib can be used for treating gastrointestinal side effects (e.g., diarrhea or constipation) associated with the administration of the chemotherapeutic agent. For example, the abemaciclib may be administered about 0.5 h-about 12 h before the administration of the chemotherapeutic agent, the abemaciclib may be administered orally, and the administration dosage may be about 150 mg/kg-about 200 mg/kg. For example, the chemotherapeutic agent may be administered by injection or orally.

In the present application, abemaciclib can be used for treating gastrointestinal side effects (e.g., diarrhea or constipation) associated with fluorouracil (5-Fu). For example, the abemaciclib may be administered about 0.5 h-about 12 h before the administration of fluorouracil (5-Fu), the abemaciclib may be administered orally, and the administration dosage may be about 150 mg/kg-about 200 mg/kg. For example, capecitabine may be administered by injection.

In the present application, abemaciclib can be used for treating gastrointestinal side effects (e.g., diarrhea or constipation) associated with irinotecan. For example, the abemaciclib may be administered about 0.5 h-about 24 h before the administration of irinotecan, the abemaciclib may be administered orally, and the administration dosage may be about 10 mg/kg-about 200 mg/kg. For example, irinotecan may be administered by injection.

In the present application, abemaciclib can be used for treating gastrointestinal side effects (e.g., diarrhea or constipation) associated with gemcitabine (GEM). For example, the abemaciclib may be administered about 0.5 h-about 24 h before the administration of gemcitabine (GEM), the abemaciclib may be administered orally, and the administration dosage may be about 150 mg/kg-about 200 mg/kg. For example, gemcitabine (GEM) may be administered by injection.

In the present application, G1T28 can be used for treating gastrointestinal side effects (e.g., diarrhea or constipation) associated with the administration of the chemotherapeutic agent. For example, the G1T28 may be administered about 0.5 h-about 24 h before the administration of the chemotherapeutic agent, the G1T28 may be administered orally, and the administration dosage may be about 150 mg/kg-about 200 mg/kg. For example, the chemotherapeutic agent may be administered by injection or orally.

In the present application, G1T28 can be used for treating gastrointestinal side effects (e.g., diarrhea or constipation) associated with fluorouracil (5-Fu). For example, the G1T28 may be administered about 0.5 h-about 24 h before the administration of fluorouracil (5-Fu), the G1T28 may be administered orally, and the administration dosage may be about 100 mg/kg-about 200 mg/kg. For example, fluorouracil (5-Fu) may be administered by injection.

In the present application, G1T28 can be used for treating gastrointestinal side effects (e.g., diarrhea or constipation) associated with topotecan. For example, the G1T28 may be administered about 0.5 h-about 12 h before the administration of topotecan, the G1T28 may be administered orally, and the administration dosage may be about 100 mg/kg-about 200 mg/kg. For example, topotecan may be administered by injection. In the present application, G1T28 can be used for treating gastrointestinal side effects (e.g., diarrhea or constipation) associated with oxaliplatin. For example, the G1T28 may be administered about 0.5 h-about 24 h before the administration of oxaliplatin, the G1T28 may be administered orally, and the administration dosage may be about 100 mg/kg-about 200 mg/kg. For example, oxaliplatin may be administered by injection.

In the present application, G1T28 can be used for treating gastrointestinal side effects (e.g., diarrhea or constipation) associated with gemcitabine (GEM). For example, the G1T28 may be administered about 0.5 h-about 24 h before the administration of gemcitabine (GEM), the G1T28 may be administered orally, and the administration dosage may be about 100 mg/kg-about 200 mg/kg. For example, gemcitabine (GEM) may be administered by injection.

In the present application, the medicament may further include one or more pharmaceutically acceptable carriers. The pharmaceutically acceptable carriers may include, but not limited to, e.g., pharmaceutically acceptable liquid, gel or solid carriers, aqueous medium, non-aqueous medium, antimicrobial substances, isotonic substances, buffer solution, antioxidants, anaesthetics, suspending agents/dispersing agents, chelating agents, emulsifiers, diluents, adjuvants, excipients, non-toxic auxiliary substances, fillers, binders, disintegrants, buffer solution, preservatives, lubricants, flavoring agents, thickening agents, colorants, emulsifiers, other components well known in the art or various combinations of the above.

In another aspect, the present application provides a method of preventing, relieving and/or treating chemotherapy-associated gastrointestinal side effects with the CDK inhibitor. In another aspect, the present application provides a use of the CDK inhibitor in preparing a medicament for preventing, relieving and/or treating chemotherapy-associated gastrointestinal side effects. In another aspect, the present application provides the CDK inhibitor, which is used for preventing or treating chemotherapy-associated gastrointestinal side effects. In some embodiments, the chemotherapy does not include chemotherapy with a continuous administration time of 7 days or above. In some embodiments, the chemotherapy does not include a tumor-targeted therapy. In some embodiments, the chemotherapy is administered orally.

In another aspect, the present application provides a method of preventing, relieving and/or treating chemotherapy-associated diarrhea with the CDK inhibitor. In another aspect, the present application provides a use of the CDK inhibitor in preparing a medicament for preventing, relieving and/or treating chemotherapy-associated diarrhea. In another aspect, the present application provides the CDK inhibitor, which is used for preventing or treating chemotherapy-associated diarrhea. In some embodiments, the chemotherapy does not include chemotherapy with a continuous administration time of 7 days or above. In some embodiments, the chemotherapy does not include a tumor-targeted therapy. In some embodiments, the chemotherapy is administered orally.

In another aspect, the present application provides a method of preventing, relieving and/or treating chemotherapy-associated constipation with the CDK inhibitor. In another aspect, the present application provides a use of the CDK inhibitor in preparing a medicament for preventing, relieving and/or treating chemotherapy-associated constipation. In another aspect, the present application provides the CDK inhibitor, which is used for preventing or treating chemotherapy-associated constipation. In some embodiments, the chemotherapy does not include chemotherapy with a continuous administration time of 7 days or above. In some embodiments, the chemotherapy does not include a tumor-targeted therapy. In some embodiments, the chemotherapy is administered orally.

Without being limited by any theory, the following examples are only for the purpose of illustrating various technical schemes of the present application, and not intended to limit the invention scope of the present application.

### Examples

### Examples 1-229 Relieving effect of oral administration of the CDK inhibitor on diarrhea caused by a chemotherapeutic agent

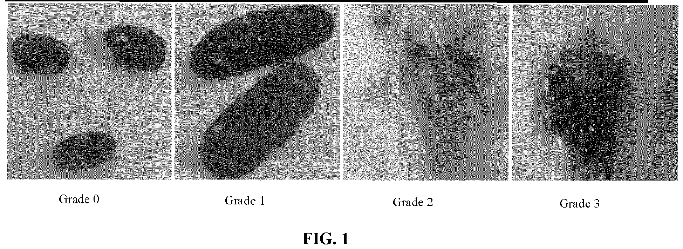

Construction of mouse animal models: Chemotherapeutic agent-caused diarrhea models of Balb/c mice were constructed according to the administration mode and frequency of the chemotherapeutic agent as shown in Table 3. After administration for several days, mice developed different extents of diarrhea symptoms (as shown in FIG. 1), which were similar to the conditions in human. Thus, chemotherapeutic agent-caused diarrhea models of mice are very good models to mimic diarrhea in human caused by the chemotherapeutic agent.

Balb/c mice were fed for 1 week, and then divided into groups. The experiment was divided into a control group, a chemotherapeutic agent group, and a CDK inhibitor group, each of which comprised 10 mice, to perform the administration experiment, for which the administration dosage, mode, time and frequency were shown in Table 3. The control group: being intragastrically administered with the same solvent as that used in the CDK inhibitor group (the time was shown in Table 3), and then being injected/intragastrically administered with the same solvent as that used in the chemotherapeutic agent group (the administration mode was shown in Table 3); the chemotherapeutic agent group: being intragastrically administered with the same solvent as that used in the CDK inhibitor group (the time was shown in Table 3), and then being injected/intragastrically administered with the chemotherapeutic agent (the type, administration mode and dosage were shown in Table 3); the CDK inhibitor group: being intragastrically administered with the CDK inhibitor (the time was shown in Table 3), and then being administered with the chemotherapeutic agent (the mode and dosage were shown in Table 3).

### Diarrhea observation

The scoring of diarrhea can refer to the method of Akinobu Kurita (Cancer Chemother Pharmacol 2000; 46:211-20.), Grade 0: normal feces; Grade 1: mild diarrhea, the stools is slightly wet and soft; Grade 2: moderate diarrhea, loose stools and mild perianal contamination; Grade 3: severe diarrhea, watery stools and accompanied by severe perianal staining (see FIG. 1). It is regarded as effective remission when the diarrhea grading of mice in the CDK inhibitor group is reduced compared with the average diarrhea grading of mice in the chemotherapeutic agent group.

The diarrhea grading of mice was observed and recorded every day. At the end of the experiment, the number of mice in the CDK inhibitor group whose diarrhea grading was lower than that of mice in the chemotherapeutic agent group was recorded. Table 3 lists various animal experiment combinations of chemotherapeutic agents and CDK inhibitors, as well as the corresponding experimental results. (Relative remission rate%= the number of mice in the CDK inhibitor group whose diarrhea has been effectively relieved/the total number of mice in the CDK inhibitor group * 100%. The establishment rate of diarrhea model in the chemotherapeutic agent group was 50%-70%, that is, about 5-7 of 10 mice developed diarrhea during the experiment, and there were cases of individual mice death or not developing diarrhea.)

**Table 3: Experimental Conditions and Results of Examples 1-229**

| Ex. * | Chemo. * | Ad min .m.* | Dos. * | F r e q * | CDK i* | Adm in. t.* | Admin. m.* | Dos * . | Freq * . | Ex. cycle (d) | Rel. rem. (%)* |
|---|---|---|---|---|---|---|---|---|---|---|---|
| 1 | 5-FU | I.P. | 125 | S | Palbo | 0.5 h | Oral | 150 | S | 10 | 20 |
| 2 | 5-FU | I.P. | 125 | S | Palbo | 1 h | Oral | 150 | S | 10 | 20 |
| 3 | 5-FU | I.P. | 125 | S | Palbo | 2 h | Oral | 150 | S | 10 | 30 |
| 4 | 5-FU | I.P. | 125 | S | Palbo | 3 h | Oral | 150 | S | 10 | 40 |
| 5 | 5-FU | I.P. | 125 | S | Palbo | 4 h | Oral | 150 | S | 10 | 40 |
| 6 | 5-FU | I.P. | 125 | S | Palbo | 5 h | Oral | 150 | S | 10 | 50 |
| 7 | 5-FU | I.P. | 125 | S | Palbo | 6 h | Oral | 150 | S | 10 | 50 |
| 8 | 5-FU | I.P. | 125 | S | Palbo | 7 h | Oral | 150 | S | 10 | 60 |
| 9 | 5-FU | I.P. | 125 | S | Palbo | 8 h | Oral | 150 | S | 10 | 60 |
| 10 | 5-FU | I.P. | 125 | S | Palbo | 9 h | Oral | 150 | S | 10 | 50 |
| 11 | 5-FU | I.P. | 125 | S | Palbo | 10 h | Oral | 150 | S | 10 | 60 |
| 12 | 5-FU | I.P. | 125 | S | Palbo | 11 h | Oral | 150 | S | 10 | 70 |
| 13 | 5-FU | I.P. | 125 | S | Palbo | 12 h | Oral | 150 | S | 10 | 80 |
| 14 | 5-FU | I.P. | 125 | S | Palbo | 13 h | Oral | 12.5 | S | 10 | 40 |
| 15 | 5-FU | I.P. | 125 | S | Palbo | 14 h | Oral | 25 | S | 10 | 80 |
| 16 | 5-FU | I.P. | 125 | S | Palbo | 15 h | Oral | 50 | S | 10 | 60 |
| 17 | 5-FU | I.P. | 125 | S | Palbo | 16 h | Oral | 100 | S | 10 | 80 |
| 18 | 5-FU | I.P. | 125 | S | Palbo | 17 h | Oral | 150 | S | 10 | 80 |
| 19 | 5-FU | I.P. | 125 | S | Palbo | 18 h | Oral | 250 | S | 10 | 60 |
| 20 | 5-FU | I.P. | 125 | S | Palbo | 14 h | Oral | 150 | S | 10 | 70 |
| 21 | 5-FU | I.P. | 125 | S | Palbo | 16 h | Oral | 150 | S | 10 | 60 |
| 22 | 5-FU | I.P. | 125 | S | Palbo | 18 h | Oral | 150 | S | 10 | 60 |
| 23 | 5-FU | I.P. | 125 | S | Palbo | 20 h | Oral | 150 | S | 10 | 50 |
| 24 | 5-FU | I.P. | 125 | S | Palbo | 22 h | Oral | 150 | S | 10 | 40 |
| 25 | 5-FU | I.P. | 125 | S | Palbo | 24 h | Oral | 150 | S | 10 | 40 |
| 26 | 5-FU | I.P. | 125 | S | Palbo | 48 h | Oral | 150 | S | 10 | 0 |
| 27 | 5-FU | I.P. | 125 | S | FLX-925 | 4 h | Oral | 75 | S | 10 | 50 |
| 28 | 5-FU | I.P. | 125 | S | SHR-6390 | 8 h | Oral | 150 | S | 10 | 70 |
| | 5-FU | I.P. | 125 | S | SHR-6390 | 8 h | Oral | 150 | S | 10 | 70 |
| 29 | 5-FU | I.P. | 125 | S | BPI-1178 | 6 h | Oral | 80 | S | 10 | 40 |
| | 5-FU | I.P. | 125 | S | BPI-1178 | 6 h | Oral | 80 | S | 10 | 40 |
| 30 | 5-FU | I.P. | 125 | S | BPI-16350 | 6 h | Oral | 150 | S | 10 | 80 |
| | 5-FU | I.P. | 125 | S | BPI-16350 | 6 h | Oral | 150 | S | 10 | 80 |
| 31 | 5-FU | I.P. | 125 | S | TY-302 | 6 h | Oral | 30 | S | 10 | 30 |
| | 5-FU | I.P. | 125 | S | TY-303 | 6 h | Oral | 30 | S | 10 | 30 |
| 32 | 5-FU | I.P. | 125 | S | TQB-3616 | 4 h | Oral | 70 | S | 10 | 30 |
| | 5-FU | I.P. | 125 | S | TQB-3616 | 4 h | Oral | 70 | S | 10 | 30 |
| 33 | 5-FU | I.P. | 125 | S | Zotira | 4 h | Oral | 100 | S | 10 | 20 |
| | 5-FU | I.P. | 125 | S | Zotira | 4 h | Oral | 100 | S | 10 | 20 |
| 34 | 5-FU | I.P. | 125 | S | XZP-3287 | 4 h | Oral | 60 | S | 10 | 60 |
| | 5-FU | I.P. | 125 | S | XZP-3287 | 4 h | Oral | 60 | S | 10 | 60 |
| 35 | 5-FU | I.P. | 125 | S | Rivi | 8 h | Oral | 65 | S | 10 | 40 |
| | 5-FU | I.P. | 125 | S | Rivi | 8 h | Oral | 65 | S | 10 | 40 |
| 36 | 5-FU | I.P. | 125 | S | FN-1501 | 6 h | Oral | 200 | S | 10 | 60 |
| | 5-FU | I.P. | 125 | S | FN-1501 | 6 h | Oral | 200 | S | 10 | 60 |
| 37 | 5-FU | I.P. | 125 | S | TP-1287 | 10 h | Oral | 10 | S | 10 | 40 |
| | 5-FU | I.P. | 125 | S | TP-1287 | 10 h | Oral | 10 | S | 10 | 40 |
| 38 | 5-FU | I.P. | 125 | S | BAY | 8 h | Oral | 60 | S | 10 | 20 |
| | 5-FU | I.P. | 125 | S | BAY | 8 h | Oral | 60 | S | 10 | 20 |
| 39 | 5-FU | I.P. | 125 | S | AGM-130 | 12 h | Oral | 80 | S | 10 | 30 |
| | 5-FU | I.P. | 125 | S | AGM-130 | 12 h | Oral | 80 | S | 10 | 30 |
| 40 | 5-FU | I.P. | 125 | S | G1T38 | 1 h | Oral | 150 | S | 10 | 20 |
| | 5-FU | I.P. | 125 | S | G1T38 | 1 h | Oral | 150 | S | 10 | 20 |
| 41 | 5-FU | I.P. | 125 | S | G1T38 | 4 h | Oral | 150 | S | 10 | 40 |
| | 5-FU | I.P. | 125 | S | G1T38 | 4 h | Oral | 150 | S | 10 | 40 |
| 42 | 5-FU | I.P. | 125 | S | G1T38 | 8 h | Oral | 150 | S | 10 | 70 |
| | 5-FU | I.P. | 125 | S | G1T38 | 8 h | Oral | 150 | S | 10 | 70 |
| 43 | 5-FU | I.P. | 125 | S | G1T38 | 16 h | Oral | 150 | S | 10 | 50 |
| | 5-FU | I.P. | 125 | S | G1T38 | 16 h | Oral | 150 | S | 10 | 50 |
| 44 | 5-FU | Oral | 250 | S | Palbo | 4 h | Oral | 125 | S | 10 | 60 |
| | 5-FU | Oral | 250 | S | Palbo | 4 h | Oral | 125 | S | 10 | 60 |
| 45 | 5-FU | I.V. | 175 | S | Ribo | 0.5 h | Oral | 200 | S | 8 | 40 |
| | 5-FU | I.V. | 175 | S | Ribo | 0.5 h | Oral | 200 | S | 8 | 40 |
| 46 | 5-FU | I.V. | 175 | S | Rosco | 2 h | Oral | 250 | S | 10 | 30 |
| | 5-FU | I.V. | 175 | S | Rosco | 2 h | Oral | 250 | S | 10 | 30 |
| 47 | 5-FU | I.V. | 175 | S | NUV-422 | 4 h | Oral | 80 | S | 10 | 30 |
| | 5-FU | I.V. | 175 | S | NUV-423 | 4 h | Oral | 80 | S | 10 | 30 |
| 48 | 5-FU | I.M. | 125 | S | Abema. | 8 h | Oral | 150 | S | 10 | 40 |
| | 5-FU | I.M. | 125 | S | Abema. | 8 h | Oral | 150 | S | 10 | 40 |
| 49 | Oxa | I.P. | 20 | S | G1T28 | 0.5 h | Oral | 100 | S | 8 | 60 |
| 50 | Oxa | I.P. | 20 | S | G1T28 | 1 h | Oral | 100 | S | 8 | 50 |
| 51 | Oxa | I.P. | 20 | S | G1T28 | 2 h | Oral | 100 | S | 8 | 40 |
| 52 | Oxa | I.P. | 20 | S | G1T28 | 3 h | Oral | 100 | S | 8 | 60 |
| 53 | Oxa | I.P. | 20 | S | G1T28 | 4 h | Oral | 100 | S | 8 | 50 |
| 54 | Oxa | I.P. | 20 | S | G1T28 | 5 h | Oral | 100 | S | 8 | 30 |
| 55 | Oxa | I.P. | 20 | S | G1T28 | 6 h | Oral | 100 | S | 8 | 80 |
| 56 | Oxa | I.P. | 20 | S | G1T28 | 7 h | Oral | 100 | S | 8 | 60 |
| 57 | Oxa | I.P. | 20 | S | G1T28 | 8 h | Oral | 100 | S | 8 | 60 |
| 58 | Oxa | I.P. | 20 | S | G1T28 | 9 h | Oral | 100 | S | 8 | 80 |
| 59 | Oxa | I.P. | 20 | S | G1T28 | 10 h | Oral | 100 | S | 8 | 70 |
| 60 | Oxa | I.P. | 20 | S | G1T28 | 11 h | Oral | 100 | S | 8 | 70 |
| 61 | Oxa | I.P. | 20 | S | G1T28 | 12 h | Oral | 100 | S | 8 | 50 |
| 62 | Oxa | I.P. | 20 | S | G1T28 | 14 h | Oral | 100 | S | 8 | 60 |
| 63 | Oxa | I.P. | 20 | S | G1T28 | 16 h | Oral | 100 | S | 8 | 80 |
| 64 | Oxa | I.P. | 20 | S | G1T28 | 18 h | Oral | 100 | S | 8 | 80 |
| 65 | Oxa | I.P. | 20 | S | G1T28 | 20 h | Oral | 100 | S | 8 | 70 |
| 66 | Oxa | I.P. | 20 | S | G1T28 | 22 h | Oral | 100 | S | 8 | 50 |
| 67 | Oxa | I.P. | 20 | S | G1T28 | 24 h | Oral | 100 | S | 8 | 50 |
| 68 | Oxa | I.P. | 20 | S | G1T28 | 48 h | Oral | 100 | S | 8 | 0 |
| 69 | Oxa | I.P. | 20 | S | FLX-925 | 4 h | Oral | 75 | S | 8 | 60 |
| 70 | Oxa | I.P. | 20 | S | SHR-6390 | 4 h | Oral | 150 | S | 8 | 60 |
| 71 | Oxa | I.P. | 20 | S | BPI-16350 | 4 h | Oral | 150 | S | 8 | 40 |
| 72 | Oxa | I.P. | 20 | S | HS-10342 | 6 h | Oral | 95 | S | 8 | 70 |
| 73 | Oxa | I.P. | 20 | S | Roni | 6 h | Oral | 10 | S | 8 | 30 |
| 74 | Oxa | I.P. | 20 | S | Indi | 4 h | Oral | 175 | S | 8 | 20 |
| 75 | Oxa | I.P. | 20 | S | SEL 120 | 8 h | Oral | 80 | S | 8 | 40 |
| 76 | Oxa | I.P. | 20 | S | Voru | 4 h | Oral | 250 | S | 8 | 70 |
| 77 | Oxa | I.P. | 20 | S | R547 | 10 h | Oral | 80 | S | 8 | 60 |
| 78 | Oxa | I.P. | 20 | S | ZK-30470 9 | 4 h | Oral | 35 | S | 8 | 30 |
| 79 | Oxa | I.P. | 20 | S | G1T38 | 1 h | Oral | 150 | S | 8 | 20 |
| 80 | Oxa | I.P. | 20 | S | G1T38 | 2 h | Oral | 150 | S | 8 | 40 |
| 81 | Oxa | I.P. | 20 | S | G1T38 | 4 h | Oral | 150 | S | 8 | 40 |
| 82 | Oxa | I.P. | 20 | S | G1T38 | 8 h | Oral | 150 | S | 8 | 70 |
| 83 | Oxa | I.P. | 20 | S | G1T38 | 12 h | Oral | 150 | S | 8 | 70 |
| 84 | Oxa | I.P. | 20 | S | G1T38 | 16 h | Oral | 150 | S | 8 | 50 |
| 85 | Oxa | I.V. | 17.5 | S | FLX-925 | 6 h | Oral | 75 | S | 8 | 60 |
| 86 | Oxa | I.V. | 17.5 | S | SHR-6390 | 6 h | Oral | 150 | S | 8 | 70 |
| 87 | Oxa | I.V. | 17.5 | S | HS-10342 | 4 h | Oral | 95 | S | 8 | 60 |
| 88 | Oxa | I.V. | 17.5 | S | Palbo | 6 h | Oral | 150 | S | 8 | 50 |
| 89 | CPT-11 | I.P. | 240 | S | Abema. | 0.5 h | Oral | 150 | S | 7 | 30 |
| 90 | CPT-11 | I.P. | 240 | S | Abema. | 1 h | Oral | 150 | S | 7 | 30 |
| 91 | CPT-11 | I.P. | 240 | S | Abema. | 2 h | Oral | 150 | S | 7 | 40 |
| 92 | CPT-11 | I.P. | 240 | S | Abema. | 3 h | Oral | 150 | S | 7 | 30 |
| 93 | CPT-11 | I.P. | 240 | S | Abema. | 4 h | Oral | 150 | S | 7 | 50 |
| 94 | CPT-11 | I.P. | 240 | S | Abema. | 5 h | Oral | 150 | S | 7 | 60 |
| 95 | CPT-11 | I.P. | 240 | S | Abema. | 6 h | Oral | 150 | S | 7 | 60 |
| 96 | CPT-11 | I.P. | 240 | S | Abema. | 7 h | Oral | 150 | S | 7 | 60 |
| 97 | CPT-11 | I.P. | 240 | S | Abema. | 8 h | Oral | 150 | S | 7 | 70 |
| 98 | CPT-11 | I.P. | 240 | S | Abema. | 9 h | Oral | 150 | S | 7 | 50 |
| 99 | CPT-11 | I.P. | 240 | S | Abema. | 10 h | Oral | 12.5 | S | 7 | 20 |
| 100 | CPT-11 | I.P. | 240 | S | Abema. | 10 h | Oral | 25 | S | 7 | 40 |
| 101 | CPT-11 | I.P. | 240 | S | Abema. | 10 h | Oral | 50 | S | 7 | 80 |
| 102 | CPT-11 | I.P. | 240 | S | Abema. | 10 h | Oral | 100 | S | 7 | 50 |
| 103 | CPT-11 | I.P. | 240 | S | Abema. | 10 h | Oral | 150 | S | 7 | 70 |
| 104 | CPT-11 | I.P. | 240 | S | Abema. | 11 h | Oral | 100 | S | 7 | 80 |
| 105 | CPT-11 | I.P. | 240 | S | Abema. | 12 h | Oral | 100 | S | 7 | 80 |
| 106 | CPT-11 | I.P. | 240 | S | Abema. | 14 h | Oral | 100 | S | 7 | 90 |
| 107 | CPT-11 | I.P. | 240 | S | Abema. | 16 h | Oral | 100 | S | 7 | 70 |
| 108 | CPT-11 | I.P. | 240 | S | Abema. | 18 h | Oral | 100 | S | 7 | 70 |
| 109 | CPT-11 | I.P. | 240 | S | Abema. | 20 h | Oral | 100 | S | 7 | 40 |
| 110 | CPT-11 | I.P. | 240 | S | Abema. | 22 h | Oral | 100 | S | 7 | 40 |
| 111 | CPT-11 | I.P. | 240 | S | Abema. | 24 h | Oral | 100 | S | 7 | 40 |
| 112 | CPT-11 | I.P. | 240 | S | Abema. | 48 h | Oral | 100 | S | 7 | 0 |
| 113 | CPT-11 | Oral | 300 | S | Ribo | 24 h | Oral | 100 | S | 11 | 60 |
| 114 | CPT-11 | I.M. | 250 | S | FLX-925 | 4 h | Oral | 75 | S | 7 | 50 |
| 115 | CPT-11 | I.P. | 240 | S | XZP-3287 | 4 h | Oral | 60 | S | 7 | 40 |
| 116 | CPT-11 | I.P. | 240 | S | Rigo | 4 h | Oral | 250 | S | 7 | 20 |
| 117 | CPT-11 | I.P. | 240 | S | Roni | 6 h | Oral | 10 | S | 7 | 20 |
| 118 | CPT-11 | I.P. | 240 | S | Seli | 6 h | Oral | 250 | S | 7 | 30 |
| 119 | CPT-11 | I.P. | 240 | S | Alvo | 6 h | Oral | 70 | S | 7 | 40 |
| 120 | CPT-11 | I.P. | 240 | S | SEL 120 | 8 h | Oral | 80 | S | 7 | 40 |
| 121 | CPT-11 | I.P. | 240 | S | ON123300 | 8 h | Oral | 250 | S | 7 | 60 |
| 122 | CPT-11 | I.P. | 240 | S | Fadra | 8 h | Oral | 80 | S | 7 | 30 |
| 123 | CPT-11 | I.P. | 240 | S | PHA-125 | 12 h | Oral | 60 | S | 7 | 40 |
| 124 | CPT-11 | I.P. | 240 | S | RGB | 12 h | Oral | 100 | S | 7 | 60 |
| 125 | CPT-11 | I.P. | 240 | S | G1T38 | 4 h | Oral | 150 | S | 7 | 50 |
| 126 | CPT-11 | I.P. | 240 | S | G1T38 | 6 h | Oral | 150 | S | 7 | 60 |
| 127 | CPT-11 | I.M. | 240 | S | G1T28 | 1 h | Oral | 50 | S | 7 | 70 |
| 128 | CPT-11 | I.M. | 240 | S | G1T38 | 2 h | Oral | 150 | S | 7 | 50 |
| 129 | CPT-11 | I.V. | 200 | S | Palbo | 14 h | Oral | 200 | S | 7 | 60 |
| 130 | CPT-11 | I.V. | 200 | S | RGB | 8 h | Oral | 100 | S | 7 | 50 |
| 131 | DTX | I.P. | 10 | S | Dina | 0.5 h | Oral | 40 | S | 8 | 10 |
| 132 | DTX | I.P. | 10 | S | Dina | 1 h | Oral | 40 | S | 8 | 10 |
| 133 | DTX | I.P. | 10 | S | Dina | 2 h | Oral | 40 | S | 8 | 20 |
| 134 | DTX | I.P. | 10 | S | Dina | 4 h | Oral | 40 | S | 8 | 20 |
| 135 | DTX | I.P. | 10 | S | Dina | 6 h | Oral | 40 | S | 8 | 30 |
| 136 | DTX | I.P. | 10 | S | Dina | 8 h | Oral | 40 | S | 8 | 30 |
| 137 | DTX | I.P. | 10 | S | Dina | 10 h | Oral | 40 | S | 8 | 30 |
| 138 | DTX | I.P. | 10 | S | Dina | 12 h | Oral | 40 | S | 8 | 20 |
| 139 | DTX | I.P. | 10 | S | Dina | 14 h | Oral | 40 | S | 8 | 20 |
| 140 | DTX | I.P. | 10 | S | Dina | 16 h | Oral | 40 | S | 8 | 40 |
| 141 | DTX | I.P. | 10 | S | Dina | 18 h | Oral | 40 | S | 8 | 30 |
| 142 | DTX | I.P. | 10 | S | Dina | 20 h | Oral | 40 | S | 8 | 30 |
| 143 | DTX | I.P. | 10 | S | Dina | 22 h | Oral | 40 | S | 8 | 20 |
| 144 | DTX | I.P. | 10 | S | Dina | 24 h | Oral | 40 | S | 8 | 10 |
| 145 | DTX | I.P. | 10 | S | Dina | 48 h | Oral | 40 | S | 8 | 0 |
| 146 | DTX | I.P. | 10 | S | SHR-6390 | 4 h | Oral | 150 | S | 8 | 80 |
| 147 | DTX | I.P. | 10 | S | FCN 437 | 8 h | Oral | 90 | S | 8 | 60 |
| 148 | DTX | I.P. | 10 | S | TQB-3616 | 4 h | Oral | 70 | S | 8 | 50 |
| 149 | DTX | I.P. | 10 | S | CS-3002 | 4 h | Oral | 200 | S | 8 | 70 |
| 150 | DTX | I.P. | 10 | S | MM-D37K | 6 h | Oral | 60 | S | 8 | 70 |
| 151 | DTX | I.P. | 10 | S | IIIM-290 | 6 h | Oral | 80 | S | 8 | 50 |
| 152 | DTX | I.P. | 10 | S | Zotira | 8 h | Oral | 100 | S | 8 | 30 |
| 153 | DTX | I.P. | 10 | S | NUV-422 | 8 h | Oral | 80 | S | 8 | 60 |
| 154 | DTX | I.P. | 10 | S | BEY-1107 | 8 h | Oral | 150 | S | 8 | 40 |
| 155 | DTX | I.P. | 10 | S | GLR2007 | 8 h | Oral | 60 | S | 8 | 50 |
| 156 | DTX | I.P. | 10 | S | FN-1501 | 10 h | Oral | 200 | S | 8 | 60 |
| 157 | DTX | I.P. | 10 | S | BCD-115 | 10 h | Oral | 80 | S | 8 | 40 |
| 158 | DTX | I.P. | 10 | S | Atuve | 10 h | Oral | 50 | S | 8 | 30 |
| 159 | DTX | I.P. | 10 | S | Fadra | 12 h | Oral | 80 | S | 8 | 20 |
| 160 | DTX | I.P. | 10 | S | PHA-793 | 4 h | Oral | 70 | S | 8 | 30 |
| 161 | DTX | I.P. | 10 | S | PF-071 | 8 h | Oral | 50 | S | 8 | 40 |
| 162 | DTX | I.P. | 10 | S | G1T38 | 4 h | Oral | 150 | S | 8 | 40 |
| 163 | DTX | I.P. | 10 | S | G1T38 | 8 h | Oral | 150 | S | 8 | 70 |
| 164 | DTX | I.P. | 10 | S | G1T38 | 12 h | Oral | 150 | S | 8 | 60 |
| 165 | DTX | Oral | 30 | S | KRX-0601 | 3 h | Oral | 250 | S | 8 | 10 |
| 166 | DTX | I.M. | 20 | S | Palbo | 5 h | Oral | 150 | S | 8 | 80 |
| 167 | DTX | I.V. | 10 | S | Ribo | 9 h | Oral | 200 | S | 8 | 80 |
| 168 | DTX | I.V. | 10 | S | GLR2007 | 4 h | Oral | 60 | S | 8 | 30 |
| 169 | DTX | I.V. | 10 | S | FN-1501 | 8 h | Oral | 200 | S | 8 | 50 |
| 170 | GEM | I.P. | 120 | S | G1T28 | 0.5 h | Oral | 60 | S | 8 | 50 |
| 171 | GEM | I.P. | 120 | S | G1T28 | 1 h | Oral | 60 | S | 8 | 50 |
| 172 | GEM | I.P. | 120 | S | G1T28 | 2 h | Oral | 60 | S | 8 | 40 |
| 173 | GEM | I.P. | 120 | S | G1T28 | 4 h | Oral | 60 | S | 8 | 60 |
| 174 | GEM | I.P. | 120 | S | G1T28 | 6 h | Oral | 60 | S | 8 | 50 |
| 175 | GEM | I.P. | 120 | S | G1T28 | 8 h | Oral | 60 | S | 8 | 50 |
| 176 | GEM | I.P. | 120 | S | G1T28 | 10 h | Oral | 60 | S | 8 | 50 |
| 177 | GEM | I.P. | 120 | S | G1T28 | 12 h | Oral | 60 | S | 8 | 40 |
| 178 | GEM | I.P. | 120 | S | G1T28 | 14 h | Oral | 60 | S | 8 | 60 |
| 179 | GEM | I.P. | 120 | S | G1T28 | 16 h | Oral | 60 | S | 8 | 30 |
| 180 | GEM | I.P. | 120 | S | G1T28 | 18 h | Oral | 60 | S | 8 | 30 |
| 181 | GEM | I.P. | 120 | S | G1T28 | 20 h | Oral | 60 | S | 8 | 40 |
| 182 | GEM | I.P. | 120 | S | G1T28 | 22 h | Oral | 60 | S | 8 | 30 |
| 183 | GEM | I.P. | 120 | S | G1T28 | 24 h | Oral | 60 | S | 8 | 20 |
| 184 | GEM | I.P. | 120 | S | G1T28 | 48 h | Oral | 60 | S | 8 | 0 |
| 185 | GEM | I.P. | 120 | S | BPI-1178 | 2 h | Oral | 80 | S | 8 | 60 |
| 186 | GEM | I.P. | 120 | S | XZP-3287 | 4 h | Oral | 60 | S | 8 | 60 |
| 187 | GEM | I.P. | 120 | S | BEBT-209 | 4 h | Oral | 80 | S | 8 | 40 |
| 188 | GEM | I.P. | 120 | S | XZP-3287 | 8 h | Oral | 60 | S | 8 | 50 |
| 189 | GEM | I.P. | 120 | S | Rigo | 6 h | Oral | 250 | S | 8 | 30 |
| 190 | GEM | I.P. | 120 | S | Indi | 4 h | Oral | 175 | S | 8 | 40 |
| 191 | GEM | I.P. | 120 | S | GLR2007 | 10 h | Oral | 60 | S | 8 | 50 |
| 192 | GEM | I.V. | 120 | S | Rivi | 6 h | Oral | 180 | S | 8 | 70 |
| 193 | GEM | Oral | 300 | S | G1T28 | 2 h | Oral | 200 | S | 8 | 60 |
| 194 | GEM/Pac. | I.P. | 60 (G) | S | Mil | 2 h | Oral | 60 | S | 8 | 40 |
| | GEM/Pac. | I.P. | 10 (P) | S | Mil | 2 h | Oral | 60 | S | 8 | 40 |
| 195 | GEM/Pac. | I.V. | 60 (G) | S | Seli | 0.5 h | Oral | 400 | S | 8 | 30 |
| | GEM/Pac. | I.V. | 10 (P) | S | Seli | 0.5 h | Oral | 400 | S | 8 | 30 |
| 196 | GEM/Pac. | I.P. | 60 (G) | S | BPI-16350 | 2 h | Oral | 150 | S | 8 | 50 |
| | GEM/Pac. | I.P. | 10 (P) | S | BPI-16350 | 2 h | Oral | 150 | S | 8 | 50 |
| 197 | GEM/Pac. | I.V. | 60 (G) | S | TQB-3616 | 4 h | Oral | 70 | S | 8 | 60 |
| | GEM/Pac. | I.V. | 10 (P) | S | TQB-3616 | 4 h | Oral | 70 | S | 8 | 60 |
| 198 | GEM/Pac. | I.P. | 60 (G) | S | MM-D37K | 2 h | Oral | 60 | S | 8 | 50 |
| | GEM/Pac. | I.P. | 10 (P) | S | MM-D37K | 2 h | Oral | 60 | S | 8 | 50 |
| 199 | GEM/Pac. | I.V. | 60 (G) | S | Seli | 2 h | Oral | 250 | S | 8 | 20 |
| | GEM/Pac. | I.V. | 10 (P) | S | Seli | 2 h | Oral | 250 | S | 8 | 20 |
| 200 | GEM/Pac. | I.P. | 60 (G) | S | Rosco | 6 h | Oral | 250 | S | 8 | 40 |
| | GEM/Pac. | I.P. | 10 (P) | S | Rosco | 6 h | Oral | 250 | S | 8 | 40 |
| 201 | GEM/Pac. | I.V. | 60 (G) | S | Alvo | 6 h | Oral | 70 | S | 8 | 50 |
| | GEM/Pac. | I.V. | 10 (P) | S | Alvo | 6 h | Oral | 70 | S | 8 | 50 |
| 202 | GEM/Pac. | I.P. | 60 (G) | S | ON123300 | 2 h | Oral | 250 | S | 8 | 50 |
| | GEM/Pac. | I.P. | 10 (P) | S | ON123300 | 2 h | Oral | 250 | S | 8 | 50 |
| 203 | GEM/Pac. | I.V. | 60 (G) | S | Voru | 8 h | Oral | 250 | S | 8 | 80 |
| | GEM/Pac. | I.V. | 10 (P) | S | Voru | 8 h | Oral | 250 | S | 8 | 80 |
| 204 | GEM/Pac. | I.P. | 60 (G) | S | Dina | 4 h | Oral | 70 | S | 8 | 30 |
| | GEM/Pac. | I.P. | 10 (P) | S | Dina | 4 h | Oral | 70 | S | 8 | 30 |
| 205 | GEM/Pac. | I.V. | 60(G)1 0 (P) | S | AT-7519 | 4 h | Oral | 50 | S | 8 | 20 |
| 206 | CBP/Pac | I.P. | 5 (C) | S | FCN 437 | 4 h | Oral | 90 | S | 10 | 40 |
| | CBP/Pac | I.P. | 10 (P) | S | FCN 437 | 4 h | Oral | 90 | S | 10 | 40 |
| 207 | CBP/Pac | I.P. | 5 (C) | S | BEBT-209 | 4 h | Oral | 80 | S | 10 | 50 |
| | CBP/Pac | I.P. | 10 (P) | S | BEBT-209 | 4 h | Oral | 80 | S | 10 | 50 |
| 208 | CBP/Pac | I.P. | 5 (C) | S | TY-302 | 6 h | Oral | 30 | S | 10 | 30 |
| | CBP/Pac | I.P. | 10 (P) | S | TY-302 | 6 h | Oral | 30 | S | 10 | 30 |
| 209 | CBP/Pac | I.P. | 5 (C) | S | HS-10342 | 6 h | Oral | 95 | S | 10 | 70 |
| | CBP/Pac | I.P. | 10 (P) | S | HS-10342 | 6 h | Oral | 95 | S | 10 | 70 |
| 210 | CBP/Pac | I.P. | 5 (C) | S | PF-068 | 6 h | Oral | 80 | S | 10 | 40 |
| | CBP/Pac | I.P. | 10 (P) | S | PF-068 | | Oral | 80 | S | 10 | 40 |
| 211 | CBP/Pac | I.P. | 5 (C) | S | G1T38 | 8 h | Oral | 150 | S | 10 | 40 |
| | CBP/Pac | I.P. | 10 (P) | S | G1T38 | | Oral | 150 | S | 10 | 40 |
| 212 | CBP/Pac | I.P. | 5 (C) | S | G1T38 | 12 h | Oral | 150 | S | 10 | 70 |
| | CBP/Pac | I.P. | 10 (P) | S | G1T38 | 12 h | Oral | 150 | S | 10 | 70 |
| 213 | 5-FU/CTP-11 | I.V. | 100(F) ,200 (I) | S | SHR-6390 | 0.5 h | Oral | 75 | S | 10 | 60 |
| 214 | 5-FU/CTP-11 | I.V. | 100(F) ,200 (I) | S | Rigo | 2 h | Oral | 250 | S | 10 | 20 |
| 215 | 5-FU/CTP-11 | I.V. | 100 (F) | S | Rivi | 2 h | Oral | 65 | S | 10 | 50 |
| | 5-FU/CTP-11 | I.V. | 200 (I) | S | Rivi | 2 h | Oral | 65 | S | 10 | 50 |
| 216 | 5-FU/CTP-11 | I.V. | 100 (F) | S | BEY-1107 | 2 h | Oral | 150 | S | 10 | 30 |
| | 5-FU/CTP-11 | I.V. | 200 (I) | S | BEY-1107 | 2 h | Oral | 150 | S | 10 | 30 |
| 217 | 5-FU/CTP-11 | I.V. | 100 (F) | S | BCD-115 | 4 h | Oral | 80 | S | 10 | 20 |
| | 5-FU/CTP-11 | I.V. | 200 (I) | S | BCD-115 | 4 h | Oral | 80 | S | 10 | 20 |
| 218 | 5-FU/CTP-11 | I.V. | 100 (F) | S | RO45 | 4 h | Oral | 70 | S | 10 | 50 |
| | 5-FU/CTP-11 | I.V. | 200 (I) | S | RO45 | 4 h | Oral | 70 | S | 10 | 50 |
| 219 | 5-FU/ Oxa | I.P. | 100 (F) | S | AMG925 | 2 h | Oral | 150 | S | 10 | 60 |
| | 5-FU/ Oxa | I.P. | 10 (O) | S | AMG925 | 2 h | Oral | 150 | S | 10 | 60 |
| 220 | 5-FU/ Oxa | I.P. | 100 (F) | S | TQB-3616 | 6 h | Oral | 70 | S | 10 | 60 |
| | 5-FU/ Oxa | I.P. | 10 (O) | S | TQB-3616 | 6 h | Oral | 70 | S | 10 | 60 |
| 221 | 5-FU/ Oxa | I.P. | 100 (F) | S | HS-10342 | 1 h | Oral | 95 | S | 10 | 50 |
| | 5-FU/ Oxa | I.P. | 10 (O) | S | HS-10342 | 1 h | Oral | 95 | S | 10 | 50 |
| 222 | 5-FU/ Oxa | I.P. | 100 (F) | S | PF-068 | 4 h | Oral | 80 | S | 10 | 30 |
| | 5-FU/ Oxa | I.P. | 10 (O) | S | PF-068 | 4 h | Oral | 80 | S | 10 | 30 |
| 223 | 5-FU/ Oxa | I.P. | 100 (F) | S | CS-3002 | 2 h | Oral | 200 | S | 10 | 50 |
| | 5-FU/ Oxa | I.P. | 10 (O) | S | CS-3002 | 2 h | Oral | 200 | S | 10 | 50 |
| 224 | 5-FU/ Oxa | I.P. | 100 (F) | S | MM-D37K | 10 h | Oral | 60 | S | 10 | 30 |
| | 5-FU/ Oxa | I.P. | 10 (O) | S | MM-D37K | 10 h | Oral | 60 | S | 10 | 30 |
| 225 | 5-FU/Oxa/ CTP-11 | I.P. | 50 (F) | S | BPI-1178 | 12 h | Oral | 100 | S | 10 | 50 |
| | 5-FU/Oxa/ CTP-11 | I.P. | 5 (O) | S | BPI-1178 | 12 h | Oral | 100 | S | 10 | 50 |
| | 5-FU/Oxa/ CTP-11 | I.P. | 50 (I) | S | BPI-1178 | 12 h | Oral | 100 | S | 10 | 50 |
| 226 | TP | I.P. | 10 | S | Abema. | 6 h | Oral | 150 | S | 14 | 40 |
| 227 | TP | I.V. | 20 | S | G1T28 | 0.5 h | Oral | 100 | S | 14 | 70 |
| 228 | Etopl/CBP | I.P. | 60 (E) | S | Palbo | 12 h | Oral | 150 | S | 10 | 60 |
| | Etopl/CBP | I.P. | 5 (C) | S | Palbo | 12 h | Oral | 150 | S | 10 | 60 |
| 229 | Etopl/CBP | I.P. | 60 (E) | S | Ribo | 12 h | Oral | 200 | S | 10 | 50 |
| | Etopl/CBP | I.P. | 5 (C) | S | Ribo | 12 h | Oral | 200 | S | 10 | 50 |

| | | | | | | | | | | | |
|---|---|---|---|---|---|---|---|---|---|---|---|
| Ex.*: Example No.; Chemo*: Chemotherapeutic agent; Admin.m.*: Administration mode; Dos*: Dosage (mg/kg); Freq*: Frequency; CDK. i*; CDK inhibitor; Admin.t.*: Administration time (before the chemotherapeutic agent); Ex. Cycle (d):Experimental cycle (days); Rel.rem.(%)*: Relative remission rate (%); S: Single; 5-FU: Fluorouracil; Oxa: Oxaliplatin; CTP-11: Irinotecan; DTX: Doceataxel; GEM: Gemcitabine; Pac: Paclitaxe; TP: topotecan; Etopl: Etoposide; Car: Carboplatin; Palbo: Palbociclib; Zotira: Zotiraciclib; Rivi: Riviciclib; BAY: BAY-1251152; Ribo: Ribociclib; Rosco: Roscovitine; Abema: Abemaciclib; Roni: Roniciclib; Indi: Indisulam; Voru: Voruciclib; Rigo: Rigosertib; Seli: Seliciclib; Alvo: Alvociclib; Fadra: Fadraciclib; RGB: RGB-286638; Dina: Dinaciclib; Atuve: Atuveciclib; Mil: Milciclib; PF-071: PF-07104091; PF-06842874; PHA-793: PHA-793887; PHA-690509; RO45: RO4584820; I.P.: Intraperitoneal injection; I.V.: Intravenous injection; I.M.: Intramuscular injection; Oral: Oral administration. | | | | | | | | | | | |

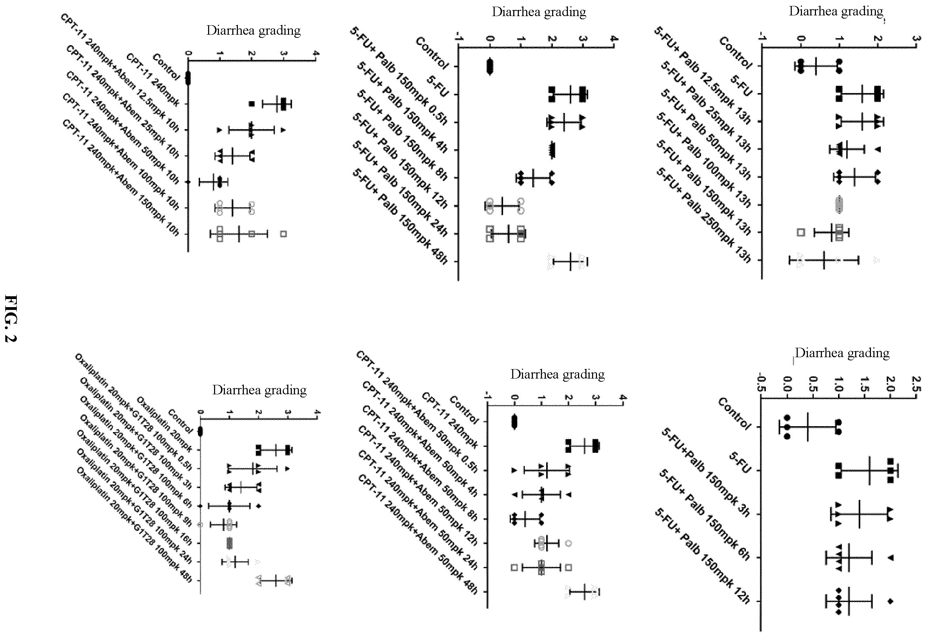

FIG. 1 shows typical photographs showing different diarrhea gradings of mice in the chemotherapeutic agent group in Table 3. FIG. 2 shows partial diarrhea grading results of the control group, the chemotherapeutic agent group, and the CDK inhibitor group in Table 3.

It can be seen from the results in Table 3 and FIG. 2 that, the diarrhea grading in the CDK inhibitor group was relieved to different degrees compared with that in the chemotherapeutic agent group. Therefore, oral administration of the CDK inhibitor in advance can effectively relieve diarrhea caused by the chemotherapeutic agent.

### Examples 230-271 Relieving effect of oral administration of the CDK inhibitor on diarrhea caused by a chemotherapeutic agent

Balb/c mice were fed for 1 week, and then divided into groups. The experiment was divided into a control group, a chemotherapeutic agent group, and a CDK inhibitor group, each of which comprised 10 mice, to perform the administration experiment, for which the administration dosage, mode, time and frequency were shown in Table 4. The control group: being intragastrically administered with the same solvent as that used in the CDK inhibitor group (the time was shown in Table 4), and then being injected/intragastrically administered with the same solvent as that used in the chemotherapeutic agent group (the administration mode was shown in Table 4); the chemotherapeutic agent group: being intragastrically administered with the same solvent as that used in the CDK inhibitor group (the time was shown in Table 4), and then being injected/intragastrically administered with the chemotherapeutic agent (the type, administration mode and dosage were shown in Table 4); the CDK inhibitor group: being intragastrically administered with the CDK inhibitor (the time was shown in Table 4), and then being administered with the chemotherapeutic agent (the mode and dosage were shown in Table 4).

### Diarrhea observation

The scoring of diarrhea can refer to the method of Akinobu Kurita (Cancer Chemother Pharmacol 2000; 46:211-20.), Grade 0: normal feces; Grade 1: mild diarrhea, the stools is slightly wet and soft; Grade 2: moderate diarrhea, loose stools and mild perianal contamination; Grade 3: severe diarrhea, watery stools and accompanied by severe perianal staining (see FIG. 1). It is regarded as effective remission when the diarrhea grading of mice in the CDK inhibitor group is reduced compared with the average diarrhea grading of mice in the chemotherapeutic agent group.

The diarrhea grading of mice was observed and recorded every day. At the end of the experiment, the number of mice in the CDK inhibitor group whose diarrhea grading was lower than that of mice in the chemotherapeutic agent group was recorded. Table 4 lists various animal experiment combinations of chemotherapeutic agents and CDK inhibitors, as well as the corresponding experimental results. (Relative remission rate%= the number of mice in the CDK inhibitor group whose diarrhea has been effectively relieved/the total number of mice in the CDK inhibitor group * 100%. The establishment rate of diarrhea model in the chemotherapeutic agent group was 50%-70%, that is, about 5-7 of 10 mice developed diarrhea during the experiment, and there were cases of individual mice death or not developing diarrhea.)

**Table 4: Experimental Conditions and Results of Examples 230-271**

| Ex. * | Chemo. * | Admi n.m.* | Dos.* | F r e q * | CDK i* | Admin. t.* | Admin.m * | Dos.* | Freq. * | Ex. Cycle (d) | Rel. rem. (%)* |
|---|---|---|---|---|---|---|---|---|---|---|---|
| 230 | Dox | I.P. | 10 | S | Palbo | 0.5 h | Oral | 150 | S | 12 | 50 |
| 231 | Dox | I.P. | 10 | S | Palbo | 4 h | Oral | 150 | S | 12 | 70 |
| 232 | Dox | I.P. | 10 | S | Palbo | 8 h | Oral | 150 | S | 12 | 70 |
| 233 | Dox | I.P. | 10 | S | Palbo | 12 h | Oral | 150 | S | 12 | 80 |
| 234 | Dox | I.P. | 10 | S | Palbo | 24 h | Oral | 150 | S | 12 | 50 |
| 235 | Dox | I.P. | 10 | S | Palbo | 48 h | Oral | 150 | S | 12 | 0 |
| 236 | Dox | I.V. | 12 | S | G1T28 | 1 h | Oral | 180 | S | 10 | 40 |
| 237 | Dox | I.V. | 12 | S | G1T29 | 12 h | Oral | 180 | S | 10 | 60 |
| 238 | Dox | I.V. | 12 | S | G1T30 | 24 h | Oral | 180 | S | 10 | 60 |
| 239 | Dox | I.V. | 12 | S | G1T31 | 48 h | Oral | 180 | S | 10 | 0 |
| 240 | Dox | I.M. | 10 | S | Dina | 0.5 h | Oral | 40 | S | 10 | 30 |
| 241 | Dox | I.M. | 10 | S | Dina | 12 h | Oral | 40 | S | 10 | 20 |
| 242 | Dox | I.M. | 10 | S | Dina | 24 h | Oral | 40 | S | 10 | 0 |
| 243 | MTX | I.V. | 300 | S | Ribo | 0.5 h | Oral | 125 | S | 7 | 80 |
| 244 | MTX | I.V. | 300 | S | Ribo | 4 h | Oral | 125 | S | 7 | 60 |
| 245 | MTX | I.V. | 300 | S | Ribo | 8 h | Oral | 125 | S | 7 | 80 |
| 246 | MTX | I.V. | 300 | S | Ribo | 12 h | Oral | 125 | S | 7 | 40 |
| 247 | MTX | I.V. | 300 | S | Ribo | 24 h | Oral | 125 | S | 7 | 60 |
| 248 | MTX | I.V. | 300 | S | Ribo | 48 h | Oral | 125 | S | 7 | 0 |
| 249 | MTX | Oral | 400 | S | Palbo | 4 h | Oral | 125 | S | 7 | 60 |
| 250 | MTX | Oral | 400 | S | Palbo | 24 h | Oral | 125 | S | 7 | 60 |
| 251 | MTX | I.P. | 260 | S | Ribo | 12 h | Oral | 200 | S | 7 | 50 |
| 252 | MTX | I.P. | 260 | S | Ribo | 48 h | Oral | 200 | S | 7 | 0 |
| 253 | MTX | I.M. | 300 | S | Abema | 8 h | Oral | 150 | S | 7 | 70 |
| 254 | Ara-C | I.P. | 600 | S | G1T28 | 0.5 h | Oral | 100 | S | 8 | 60 |
| 255 | Ara-C | I.P. | 600 | S | G1T29 | 4 h | Oral | 100 | S | 8 | 80 |
| 256 | Ara-C | I.P. | 600 | S | G1T30 | 8 h | Oral | 100 | S | 8 | 80 |
| 257 | Ara-C | I.P. | 600 | S | G1T31 | 12 h | Oral | 100 | S | 8 | 60 |
| 258 | Ara-C | I.P. | 600 | S | G1T32 | 24 h | Oral | 100 | S | 8 | 40 |
| 259 | Ara-C | I.P. | 600 | S | G1T33 | 48 h | Oral | 100 | S | 8 | 0 |
| 260 | Ara-C | I.V. | 600 | S | Palbo | 6 h | Oral | 150 | S | 8 | 60 |
| 261 | Ara-C | I.M. | 600 | S | SHR-6390 | 12 h | Oral | 150 | S | 8 | 60 |
| 262 | Ara-C | Oral | 1000 | S | Rivi | 24 h | Oral | 150 | S | 8 | 20 |
| 263 | NVB | I.V. | 10 | S | Abema | 0.5 h | Oral | 150 | S | 7 | 60 |
| 264 | NVB | I.V. | 10 | S | Abema | 4 h | Oral | 150 | S | 7 | 80 |
| 265 | NVB | I.V. | 10 | S | Abema | 8 h | Oral | 150 | S | 7 | 80 |
| 266 | NVB | I.V. | 10 | S | Abema | 12 h | Oral | 150 | S | 7 | 60 |
| 267 | NVB | I.V. | 10 | S | Abema | 24 h | Oral | 150 | S | 7 | 50 |
| 268 | NVB | I.V. | 10 | S | Abema | 48 h | Oral | 150 | S | 7 | 0 |
| 269 | NVB | Oral | 15 | S | Ribo | 24 h | Oral | 100 | S | 11 | 50 |
| 270 | NVB | I.M. | 10 | S | G1T28 | 1 h | Oral | 50 | S | 7 | 50 |
| 271 | NVB | I.M. | 10 | S | Palbo | 12 h | Oral | 200 | S | 7 | 70 |

| | | | | | | | | | | | |
|---|---|---|---|---|---|---|---|---|---|---|---|
| Ex.*: Example No.; Chemo*: Chemotherapeutic agent; Admin.m.*: Administration mode; Dos*: Dosage (mg/kg); Freq*: Frequency; CDK. i*; CDK inhibitor; Admin.t.*: Administration time (before the chemotherapeutic agent); Ex. Cycle (d):Experimental cycle (days); Rel.rem.(%)*: Relative remission rate (%); S: Single; Dox: Doxorubicin; MTX: Methrotrexate; Ara-C: Cytarabine; NVB: Vinorelbine; Palbo: Palbociclib; Dina: Dinaciclib; Ribo: Ribociclib; Abema: Abemaciclib; Rivi: Riviciclib; I.P.: Intraperitoneal injection; I.V.: Intravenous injection; I.M.: Intramuscular injection; Oral: Oral adiminstration. | | | | | | | | | | | |

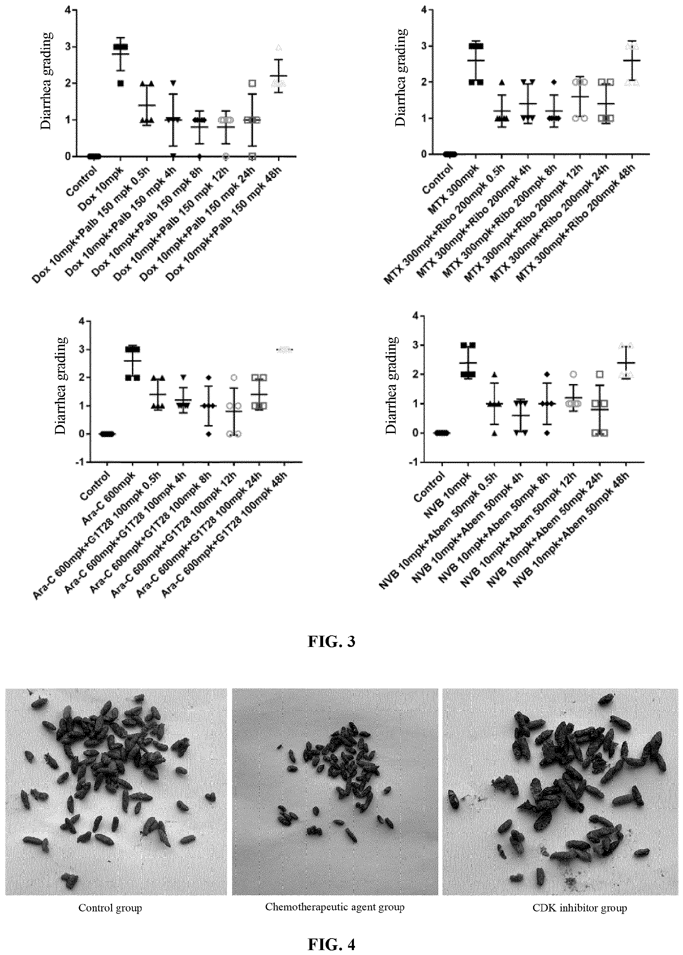

FIG. 3 shows partial diarrhea grading results of the control group, the chemotherapeutic agent group, and the CDK inhibitor group in Table 4.

It can be seen from the results in Table 4 and FIG. 3 that, the diarrhea grading in the CDK inhibitor group was relieved to different degrees compared with that in the chemotherapeutic agent group. Therefore, oral administration of the CDK inhibitor in advance can effectively relieve diarrhea caused by the chemotherapeutic agent.

### Examples 272-342 Relieving effect of oral administration of the CDK inhibitor on constipation caused by a chemotherapeutic agent

Construction of mouse animal models: Chemotherapeutic agent-caused constipation models of Balb/c mice were constructed according to the administration mode and frequency of the chemotherapeutic agent as shown in Table 5. After administration for several days, mice showed decreased stool volume (as shown in FIG. 4), which was consistent with the symptoms of constipation caused by a chemotherapeutic agent in human. Thus, chemotherapeutic agent-caused constipation models of mice are very good models to mimic constipation caused by the chemotherapeutic agent.

Balb/c mice were fed for 1 week, and then divided into groups. The experiment was divided into a control group, a chemotherapeutic agent group, and a CDK inhibitor group, each of which comprised 10 mice, to perform the administration experiment, for which the administration dosage, mode, time and frequency were shown in Table 5. The control group: being intragastrically administered with the same solvent as that used in the CDK inhibitor group (the time was shown in Table 5), and then being injected/intragastrically administered with the same solvent as that used in the chemotherapeutic agent group (the administration mode was shown in Table 5); the chemotherapeutic agent group: being intragastrically administered with the same solvent as that used in the CDK inhibitor group (the time was shown in Table 3), and then being injected/intragastrically administered with the chemotherapeutic agent (the type, administration mode and dosage were shown in Table 5); the CDK inhibitor group: being intragastrically administered with the CDK inhibitor (the time was shown in Table 5), and then being administered with the chemotherapeutic agent (the mode and dosage were shown in Table 5).

### Constipation observation

The feces of mice within 3 hours were collected, which were observed for shape and weighed with an electronic balance. The feces reduction rate was calculated with reference to the method of Ji Eun Kim (Lab Anim Res. 2016 Dec; 32(4): 231-240.). Feces reduction rate (%) = (the control group - the chemotherapeutic agent group or the CDK inhibitor group)/the control group*100%

It is regarded as effective remission when the feces reduction rate of mice in the CDK inhibitor group is reduced compared with that in the chemotherapeutic agent group.

The feces amount of mice within 3 hours was observed and recorded every day. At the end of the experiment, the number of mice in the CDK inhibitor group whose feces reduction rate was lower than that of mice in the chemotherapeutic agent group was recorded. Table 5 lists various animal experiment combinations of chemotherapeutic agents and CDK inhibitors, as well as the corresponding experimental results. (Relative remission rate%= the number of mice in the CDK inhibitor group which have been effectively relieved/the total number of mice in the CDK inhibitor group * 100%. The establishment rate of constipation model in the chemotherapeutic agent group was 30%-60%, that is, about 3-6 of 10 mice showed reduced feces amount during the experiment, and there were cases of individual mice death or with no changes or an increasement in the feces amount.)

**Table 5: Experimental Conditions and Results of Examples 272-342**

| Ex. * | Chemo. * | Admin .m.* | Dos. * | Fr e q. * | CDK i* | Admi n. t. * | Admi n.m.* | Dos. * | Freq. * | Ex. cycle (d) | Rel. rem. (%)* |
|---|---|---|---|---|---|---|---|---|---|---|---|
| 272 | 5-FU | I.P. | 175 | S | G1T28 | 0.5 h | Oral | 100 | S | 7 | 50 |
| 273 | 5-FU | I.P. | 175 | S | G1T29 | 1 h | Oral | 100 | S | 7 | 50 |
| 274 | 5-FU | I.P. | 175 | S | G1T30 | 2 h | Oral | 100 | S | 7 | 50 |
| 275 | 5-FU | I.P. | 175 | S | G1T31 | 3 h | Oral | 100 | S | 7 | 40 |
| 276 | 5-FU | I.P. | 175 | S | G1T32 | 4 h | Oral | 100 | S | 7 | 60 |
| 277 | 5-FU | I.P. | 175 | S | G1T33 | 5 h | Oral | 100 | S | 7 | 40 |
| 278 | 5-FU | I.P. | 175 | S | G1T34 | 6 h | Oral | 100 | S | 7 | 60 |
| 279 | 5-FU | I.P. | 175 | S | G1T35 | 7 h | Oral | 100 | S | 7 | 70 |
| 280 | 5-FU | I.P. | 175 | S | G1T36 | 8 h | Oral | 100 | S | 7 | 70 |
| 281 | 5-FU | I.P. | 175 | S | G1T37 | 9 h | Oral | 100 | S | 7 | 60 |
| 282 | 5-FU | I.P. | 175 | S | G1T38 | 10 h | Oral | 100 | S | 7 | 60 |
| 283 | 5-FU | I.P. | 175 | S | G1T39 | 11 h | Oral | 100 | S | 7 | 50 |
| 284 | 5-FU | I.P. | 175 | S | G1T40 | 12 h | Oral | 100 | S | 7 | 70 |
| 285 | 5-FU | I.P. | 175 | S | G1T41 | 13 h | Oral | 100 | S | 7 | 40 |
| 286 | 5-FU | I.P. | 175 | S | G1T42 | 14 h | Oral | 100 | S | 7 | 60 |
| 287 | 5-FU | I.P. | 175 | S | G1T43 | 16 h | Oral | 100 | S | 7 | 50 |
| 288 | 5-FU | I.P. | 175 | S | G1T44 | 18 h | Oral | 100 | S | 7 | 60 |
| 289 | 5-FU | I.P. | 175 | S | G1T45 | 20 h | Oral | 100 | S | 7 | 50 |
| 290 | 5-FU | I.P. | 175 | S | G1T46 | 22 h | Oral | 100 | S | 7 | 30 |
| 291 | 5-FU | I.P. | 175 | S | G1T47 | 24 h | Oral | 100 | S | 7 | 30 |
| 292 | 5-FU | I.P. | 175 | S | G1T48 | 48 h | Oral | 100 | S | 7 | 0 |
| 293 | 5-FU | Oral | 300 | S | Palbo | 8 h | Oral | 150 | S | 7 | 50 |
| 294 | 5-FU | I.V. | 225 | S | Ribo | 0.5 h | Oral | 150 | S | 7 | 50 |
| | 5-FU | I.V. | 225 | S | Ribo | 0.5 h | Oral | 150 | S | 7 | 50 |
| 295 | 5-FU | I.M. | 200 | S | Abema | 22 h | Oral | 100 | S | 7 | 60 |
| | 5-FU | I.M. | 200 | S | Abema | 22 h | Oral | 100 | S | 7 | 60 |
| 296 | Oxa | I.P. | 20 | S | Abema | 0.5 h | Oral | 100 | S | 8 | 50 |
| 297 | Oxa | I.P. | 20 | S | Abema | 4 h | Oral | 100 | S | 8 | 40 |
| 298 | Oxa | I.P. | 20 | S | Abema | 8 h | Oral | 100 | S | 8 | 60 |
| 299 | Oxa | I.P. | 20 | S | Abema | 12 h | Oral | 100 | S | 8 | 60 |
| 300 | Oxa | I.P. | 20 | S | Abema | 24 h | Oral | 100 | S | 8 | 40 |
| 301 | Oxa | I.P. | 20 | S | Abema | 48 h | Oral | 100 | S | 8 | 0 |
| 302 | Oxa | I.V. | 20 | S | Ribo | 1 h | Oral | 150 | S | 8 | 50 |
| 303 | 5-FU/Oxa | I.P. | 100 (F) | S | G1T28 | 24 h | Oral | 100 | S | 10 | 40 |
| | 5-FU/Oxa | I.P. | 10 (O) | S | G1T28 | 25 h | Oral | 100 | S | 10 | 40 |
| 304 | CPT-11 | I.P. | 260 | S | Palbo | 0.5 h | Oral | 150 | S | 7 | 40 |
| 305 | CPT-11 | I.P. | 260 | S | Palbo | 4 h | Oral | 150 | S | 7 | 60 |
| 306 | CPT-11 | I.P. | 260 | S | Palbo | 8 h | Oral | 150 | S | 7 | 80 |
| 307 | CPT-11 | I.P. | 260 | S | Palbo | 12 h | Oral | 150 | S | 7 | 70 |
| 308 | CPT-11 | I.P. | 260 | S | Palbo | 24 h | Oral | 150 | S | 7 | 30 |
| 309 | CPT-11 | I.P. | 260 | S | Palbo | 48 h | Oral | 150 | S | 7 | 0 |
| 310 | CPT-11 | Oral | 300 | S | Ribo | 20 h | Oral | 100 | S | 11 | 50 |
| 311 | CPT-11 | I.M. | 260 | S | G1T28 | 1 h | Oral | 50 | S | 7 | 60 |
| 312 | CPT-11 | I.V. | 260 | S | Abema | 14 h | Oral | 150 | S | 7 | 50 |
| 313 | 5-FU/ CPT-11 | I.V. | 150 (F) | S | SHR-6390 | 0.5 h | Oral | 150 | S | 10 | 70 |
| | 5-FU/CTP -11 | I.V. | 200 (I) | S | SHR-6391 | 0.5 h | Oral | 150 | S | 10 | 70 |
| 314 | 5-FU/Oxa /CPT-11 | I.P. | 70 (F) | S | G1T28 | 12 h | Oral | 180 | S | 10 | 60 |
| | 5-FU/Oxa /CPT-12 | I.P. | 5 (O) | S | G1T28 | 13 h | Oral | 180 | S | 10 | 60 |
| | 5-FU/Oxa /CPT-13 | I.P. | 100 (I) | S | G1T28 | 14 h | Oral | 180 | S | 10 | 60 |
| 315 | DTX | I.P. | 15 | S | Mil | 0.5 h | Oral | 60 | S | 8 | 30 |
| 316 | DTX | I.P. | 15 | S | Mil | 4 h | Oral | 60 | S | 8 | 20 |
| 317 | DTX | I.P. | 15 | S | Mil | 8 h | Oral | 60 | S | 8 | 20 |
| 318 | DTX | I.P. | 15 | S | Mil | 12 h | Oral | 60 | S | 8 | 30 |
| 319 | DTX | I.P. | 15 | S | Mil | 24 h | Oral | 60 | S | 8 | 10 |
| 320 | DTX | I.P. | 15 | S | Mil | 48 h | Oral | 60 | S | 8 | 0 |
| 321 | DTX | Oral | 30 | S | Seli | 3 h | Oral | 400 | S | 8 | 20 |
| 322 | DTX | I.M. | 20 | S | Roni | 5 h | Oral | 1 | S | 8 | 10 |
| 323 | DTX | I.V. | 10 | S | G1T28 | 9 h | Oral | 100 | S | 8 | 60 |
| 324 | GEM/Pac | I.P. | 60 (G) | S | Abema | 24 h | Oral | 150 | S | 8 | 60 |
| | GEM/Pac | | 10 (P) | S | Abema | 25 h | Oral | 150 | S | 8 | 60 |
| 325 | GEM/Pac | I.V. | 60 (G) | S | Ribo | 0.5 h | Oral | 125 | S | 8 | 50 |
| | GEM/Pac | I.V. | 10 (P) | S | Ribo | 0.5 h | Oral | 125 | S | 8 | 50 |
| 326 | GEM/Pac | I.P. | 5 (C) | S | SHR-6390 | 24 h | Oral | 100 | S | 10 | 50 |
| | GEM/Pac | I.P. | 10 (P) | S | SHR-6390 | 24 h | Oral | 100 | S | 10 | 50 |
| 327 | Ara-C | I.P. | 600 | S | G1T28 | 0.5 h | Oral | 100 | S | 8 | 80 |
| 328 | Ara-C | I.P. | 600 | S | G1T28 | 4 h | Oral | 100 | S | 8 | 60 |
| 329 | Ara-C | I.P. | 600 | S | G1T28 | 8 h | Oral | 100 | S | 8 | 40 |
| 330 | Ara-C | I.P. | 600 | S | G1T28 | 12 h | Oral | 100 | S | 8 | 40 |
| 331 | Ara-C | I.P. | 600 | S | G1T28 | 24 h | Oral | 100 | S | 8 | 40 |
| 332 | Ara-C | I.P. | 600 | S | G1T28 | 48 h | Oral | 100 | S | 8 | 0 |
| 333 | Ara-C | I.V. | 600 | S | Palbo | 6 h | Oral | 150 | S | 8 | 50 |
| 334 | Ara-C | I.M. | 600 | S | KRX-0601 | 12 h | Oral | 40 | S | 8 | 10 |
| 335 | Ara-C | Oral | 1000 | S | Rivi | 24 h | Oral | 60 | S | 8 | 20 |
| 336 | MTX | I.V. | 300 | S | Ribo | 0.5 h | Oral | 125 | S | 7 | 40 |
| 337 | MTX | I.V. | 300 | S | Ribo | 4 h | Oral | 125 | S | 7 | 40 |
| 338 | MTX | I.V. | 300 | S | Ribo | 8 h | Oral | 125 | S | 7 | 50 |
| 339 | MTX | I.V. | 300 | S | Ribo | 12 h | Oral | 125 | S | 7 | 30 |
| 340 | MTX | I.V. | 300 | S | Ribo | 24 h | Oral | 125 | S | 7 | 60 |
| 341 | MTX | I.V. | 300 | S | Ribo | 48 h | Oral | 125 | S | 7 | 0 |
| 342 | MTX | I.P. | 300 | S | Abema | 8 h | Oral | 150 | S | 7 | 30 |

| | | | | | | | | | | | |
|---|---|---|---|---|---|---|---|---|---|---|---|
| Ex.*: Example No.; Chemo*: Chemotherapeutic agent; Admin.m.*: Administration mode; Dos*: Dosage (mg/kg); Freq*: Frequency; CDK. i*; CDK inhibitor; Admin.t.*: Administration time (before the chemotherapeutic agent); Ex. Cycle (d):Experimental cycle (days); Rel.rem.(%)*: Relative remission rate (%); S: Single; 5-FU: Fluorouracil; Oxa: Oxaliplatin; CTP-11: Irinotecan; DTX: Doceataxel; GEM: Gemcitabine; Pac: Paclitaxe; Palbo: Palbociclib; Ribo: Ribociclib: Abema: Abemaciclib; Mil: Milciclib; Seli: Seliciclib; Roni: Roniciclib; Rivi: Riviciclib; MTX: Methrotrexate; Ara-C: Cytarabine; DTX: Docetaxel; I.P.: Intraperitoneal injection; I.V.: Intravenous injection; I.M.: Intramuscular injection; Oral: Oral adiminstration. | | | | | | | | | | | |

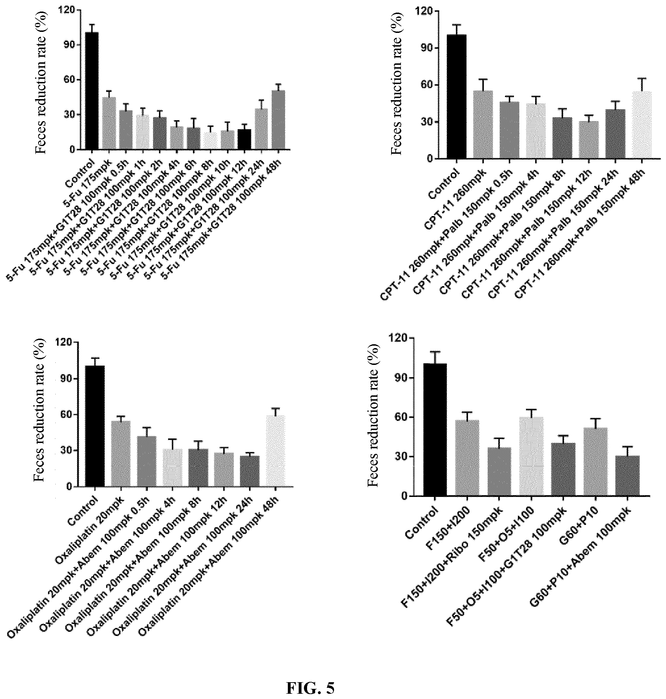

FIG. 4 shows typical photographs showing the feces amount within 3 hours in the control group, the chemotherapeutic agent group, and the CDK inhibitor group in Table 5. FIG. 5 shows partial results of feces reduction rate in the control group, the chemotherapeutic agent group, and the CDK inhibitor group in Table 5.

It can be seen from the results in Table 5 and FIGs. 4-5 that, the feces reduction rate in the CDK inhibitor group was reduced to different degrees compared with that in the chemotherapeutic agent group. Therefore, oral administration of the CDK inhibitor in advance can effectively relieve constipation caused by the chemotherapeutic agent.

### Examples 343-381 Both oral administration of a CDK inhibitor and intravenous injection of a CDK inhibitor can relieve diarrhea caused by a chemotherapeutic agent

Balb/c mice were fed for 1 week, and then divided into groups. The experiment was divided into a control group, a chemotherapeutic agent group, and an oral administration group of a CDK inhibitor, and an intravenous injection group of a CDK inhibitor, each of which comprised 10 mice, to perform the administration experiment, for which the administration dosage, mode, time and frequency were shown in Table 6. The control group: being injected/intragastrically administered with the same solvent as that used in the CDK inhibitor group (the time was shown in Table 6), and then being injected/intragastrically administered with the same solvent as that used in the chemotherapeutic agent group (the administration mode was shown in Table 6); the chemotherapeutic agent group: being injected/intragastrically administered with the same solvent as that used in the CDK inhibitor group (the time was shown in Table 6), and then being injected/intragastrically administered with the chemotherapeutic agent (the type, administration mode and dosage were shown in Table 6); the oral administration group of the CDK inhibitor: being intragastrically administered with the CDK inhibitor (the time was shown in Table 6), and then being administered with the chemotherapeutic agent (the mode and dosage were shown in Table 6); the intravenous injection group of the CDK inhibitor: being injected with the CDK inhibitor through tail vein (the time was shown in Table 6), and then being administered with the chemotherapeutic agent (the mode and dosage were shown in Table 6).

### Diarrhea observation

The scoring of diarrhea can refer to the method of Akinobu Kurita (Cancer Chemother Pharmacol 2000; 46:211-20.), Grade 0: normal feces; Grade 1: mild diarrhea, the stools is slightly wet and soft; Grade 2: moderate diarrhea, loose stools and mild perianal contamination; Grade 3: severe diarrhea, watery stools and accompanied by severe perianal staining (see FIG. 1). It is regarded as effective remission when the diarrhea grading of mice in the oral administration group of the CDK inhibitor/the intravenous injection group of the CDK inhibitor is reduced compared with the average diarrhea grading of mice in the chemotherapeutic agent group.

The diarrhea grading of mice was observed and recorded every day. At the end of the experiment, the number of mice in the oral administration group of the CDK inhibitor/the intravenous injection group of the CDK inhibitor whose diarrhea grading was lower than that of mice in the chemotherapeutic agent group was recorded. Table 6 lists various animal experiment combinations of chemotherapeutic agents and CDK inhibitors, as well as the corresponding experimental results. (Relative remission rate%= the number of mice in the oral administration or intravenous injection group of the CDK inhibitor whose diarrhea has been effectively relieved/the total number of mice in the oral administration or intravenous injection group of the CDK inhibitor * 100%. The establishment rate of diarrhea model in the chemotherapeutic agent group was 50%-70%, that is, about 5-7 of 10 mice developed diarrhea during the experiment, and there were cases of individual mice death or not developing diarrhea.)

**Table 6: Experimental Conditions and Results of Examples 343-381**

| Ex. * | Chemo.* | Ad min. m.* | Dos.* | Ex .cy cle (d) | CDK i* | Ad min. t.* | Ad min. m.* | Dos. and Freq* | Rel. rem. oral (%) * | Ad min. m.* | Dos. And Freq* | Rel .re m. I.V (%) * |
|---|---|---|---|---|---|---|---|---|---|---|---|---|
| 343 | Etopl | I.V. | 75 S | 8 | Palbo | 0.5 h | Oral | 150, S | 40 | I.V. | 150, S | 10 |
| 344 | Etopl | I.V. | 75 S | 8 | Palbo | 4 h | Oral | 150, S | 80 | I.V. | 150, S | 10 |
| 345 | Etopl | I.V. | 75 S | 8 | Palbo | 8 h | Oral | 150, S | 60 | I.V. | 150, S | 10 |
| 346 | Etopl | I.V. | 75 S | 8 | Palbo | 12 h | Oral | 150, S | 60 | I.V. | 150, S | 20 |
| 347 | Etopl | I.V. | 75 S | 8 | Palbo | 24 h | Oral | 150, S | 60 | I.V. | 150, S | 10 |
| 348 | Etopl | I.P. | 75 S | 8 | G1T28 | 24 h | Oral | 100 | 60 | I.V. | 100, S | 10 |
| | Etopl | I.P. | 75 S | 8 | G1T28 | 24 h | Oral | 100 | 60 | I.V. | 100, S | 10 |
| 349 | Etopl | I.M. | 75 S | 8 | SHR-6390 | 0.5 h | Oral | 150, S | 30 | I.V. | 150, S | 10 |
| 350 | Etopl | I.M. | 75 S | 8 | SHR-6391 | 12 h | Oral | 150, S | 50 | I.V. | 150, S | 10 |
| 351 | Etopl | I.M. | 75 S | 8 | SHR-6392 | 24 h | Oral | 150, S | 50 | I.V. | 150, S | 10 |
| 352 | Etopl /Cis | I.P. | 50 (E), 5 (Cis), S | 7 | Ribo | 0.5 h | Oral | 125, S | 40 | I.V. | 125, S | 10 |
| | Etopl/Cis | I.P. | 50 (E), 5 (Cis), S | 7 | Ribo | 0.5 h | Oral | 125, S | 40 | I.V. | 125, S | 10 |
| 353 | Etopl /Cis | I.P. | 50 (E), 5 (Cis), S | 7 | Ribo | 4h | Oral | 125, S | 40 | I.V. | 125, S | 10 |
| 354 | Etopl /Cis | I.P. | 50 (E), 5 (Cis), S | 7 | Ribo | 8h | Oral | 125, S | 60 | I.V. | 125, S | 10 |
| 355 | Etopl /Cis | I.P. | 50 (E), 5 (Cis), S | 7 | Ribo | 12 h | Oral | 125, S | 60 | I.V. | 125, S | 20 |
| 356 | Etopl /Cis | I.P. | 50 (E), 5 (Cis), S | 7 | Ribo | 24 h | Oral | 125, S | 50 | I.V. | 125, S | 10 |
| 357 | Etopl /Cis | I.V. | 51 (E), 5 (Cis), S | 7 | Palbo | 4h | Oral | 125, S | 70 | I.V. | 125, S | 20 |
| | Etopl /Cis | I.V. | 52 (E), 5 (Cis), S | 7 | Palbo | 4h | Oral | 125, S | 70 | I.V. | 125, S | |
| 358 | Etopl /Cis | I.V. | 53 (E), 5 (Cis), S | 7 | Palbo | 24 h | Oral | 125, S | 40 | I.V. | 125, S | 10 |
| 359 | CBP | I.P. | 120, S | 10 | Ribo | 0.5 h | Oral | 200, S | 60 | I.V. | 200, S | 20 |
| 360 | CBP | I.P. | 120, S | 10 | Ribo | 4h | Oral | 200, S | 80 | I.V. | 200, S | 20 |
| 361 | CBP | I.P. | 120, S | 10 | Ribo | 8h | Oral | 200, S | 60 | I.V. | 200, S | 10 |
| 362 | CBP | I.P. | 120, S | 10 | Ribo | 12 h | Oral | 200, S | 60 | I.V. | 200, S | 10 |
| 363 | CBP | I.P. | 120, S | 10 | Ribo | 24 h | Oral | 200, S | 80 | I.V. | 200, S | 10 |
| 364 | CBP | I.M. | 150, S | 10 | Abema | 8h | Oral | 150, S | 50 | I.V. | 150, S | 20 |
| | CBP | I.M. | 150, S | 10 | Abema | 8h | Oral | 150, S | 50 | I.V. | 150, S | 20 |
| 365 | CBP | I.V. | 120, S | 10 | G1T28 | 24 h | Oral | 100, S | 30 | I.V. | 100, S | 10 |
| | CBP | I.V. | 120, S | 10 | G1T28 | 24 h | Oral | 100, S | 30 | I.V. | 100, S | 10 |
| 366 | CBP | Oral | 200, S | 10 | Palbo | 24 h | Oral | 150, S | 50 | I.V. | 150, S | 10 |
| | CBP | Oral | 200, S | 10 | Palbo | 24 h | Oral | 150, S | 50 | I.V. | 150, S | 10 |
| 367 | TP | I.P. | 10, S | 10 | G1T28 | 24 h | Oral | 100, S | 30 | I.V. | 200, S | 10 |
| | TP | I.P. | 10, S | 10 | G1T28 | 24 h | Oral | 100, S | 30 | I.V. | 200, S | 10 |
| 368 | TP | I.V. | 10, S | 10 | Dina | 0.5 h | Oral | 40, S | 20 | I.V. | 40, S | 10 |
| | TP | I.V. | 10, S | 10 | Dina | 0.5 h | Oral | 40, S | 20 | I.V. | 40, S | 10 |
| 369 | TP | I.M. | 10, S | 10 | KR-0601 | 12 h | Oral | 250, S | 20 | I.V. | 250 | 10 |
| | TP | I.M. | 10, S | 10 | KR-0602 | 12 h | Oral | 250, S | 20 | I.V. | S | 10 |
| 370 | TP | Oral | 20, S | 10 | Rivi | 24 h | Oral | 60, S | 30 | I.V. | 60 | 10 |
| | TP | Oral | 20, S | 10 | Rivi | 24 h | Oral | 60, S | 30 | I.V. | S | 10 |
| 371 | GEM | I.P. | 120, S | 8 | Abema | 0.5 h | Oral | 150, S | 30 | I.V. | 150, S | 10 |
| 372 | GEM | I.P. | 120, S | 8 | Abema | 4h | Oral | 150, S | 30 | I.V. | 150, S | 10 |
| 373 | GEM | I.P. | 120, S | 8 | Abema | 8h | Oral | 150, S | 50 | I.V. | 150, S | 20 |
| 374 | GEM | I.P. | 120, S | 8 | Abema | 12 h | Oral | 150, S | 50 | I.V. | 150, S | 20 |
| 375 | GEM | I.P. | 120, S | 8 | Abema | 24 h | Oral | 150, S | 40 | I.V. | 150, S | 10 |
| 376 | GEM | Oral | 300, S | 8 | Ribo | 24 h | Oral | 100, S | 60 | I.V. | 100, S | 20 |
| | GEM | Oral | 300, S | 8 | Ribo | 24 h | Oral | 100, S | 60 | I.V. | 100, S | 20 |
| 377 | GEM | I.M. | 120, S | 8 | G1T28 | 1 h | Oral | 50, S | 60 | I.V. | 50, S | 10 |
| | GEM | I.M. | 120, S | 8 | G1T28 | 1 h | Oral | 50, S | 60 | I.V. | 50, S | 10 |
| 378 | GEM | I.V. | 120, S | 8 | Palbo | 12 h | Oral | 200, S | 50 | I.V. | 200, S | 10 |
| | GEM | I.V. | 120, S | 8 | Palbo | 12 h | Oral | 200, S | 50 | I.V. | 200, S | 10 |
| 379 | GEM | I.V. | 120, S | 8 | Palbo | 12 h | Oral | 200, S | 60 | I.V. | 200, S | 20 |
| | GEM | I.V. | 120, S | 8 | Palbo | 12 h | Oral | 200, S | 60 | I.V. | 200, S | 20 |
| 380 | GEM /CBP | I.V. | 120 (G), 100 (C), S | 8 | G1T28 | 0.5 h | Oral | 100, S | 60 | I.V. | 100, S | 20 |
| | GEM /CBP | I.V. | 121 (G), 100 (C), S | 8 | G1T28 | 0.5 h | Oral | 100, S | 60 | I.V. | 100, S | 20 |
| 381 | GEM /CBP | I.V. | 122 (G), 100 (C), S | 8 | G1T28 | 24 h | Oral | 100, S | 40 | I.V. | 100, S | 10 |

| | | | | | | | | | | | | |
|---|---|---|---|---|---|---|---|---|---|---|---|---|
| Ex.*: Example No.; Chemo*: Chemotherapeutic agent; Admin.m.*: Administration mode; Dos*: Dosage (mg/kg); Ex. Cycle (d):Experimental cycle (days); CDK. i*; CDK inhibitor; Freq*: Admin.t.*: Administration time (before the chemotherapeutic agent); Dos. And Freq.*: Dosage (mg/kg) and Frequency; Rel.rem.oral(%)*: Relative remission rate in oral administration of CDK inhibitor (%); Rel.rem.I.V(%)*: Relative remission rate in intravenous injection of CDK inhibitor (%); S: Single; Etopl: Etoposide; Cis: Cisplatin; CBP: Carboplatin; TP: Topotecan; GEM: Gemcitabine; Palbo: Palbociclib; Ribo: Ribociclib; Abema: Abemaciclib; Dina: Dinaciclib; Rivi: Riviciclib; I.P.: Intraperitoneal injection; I.V: Intravenous injection; I.M.: Intramuscular injection; Oral: Oral adiminstration. | | | | | | | | | | | | |

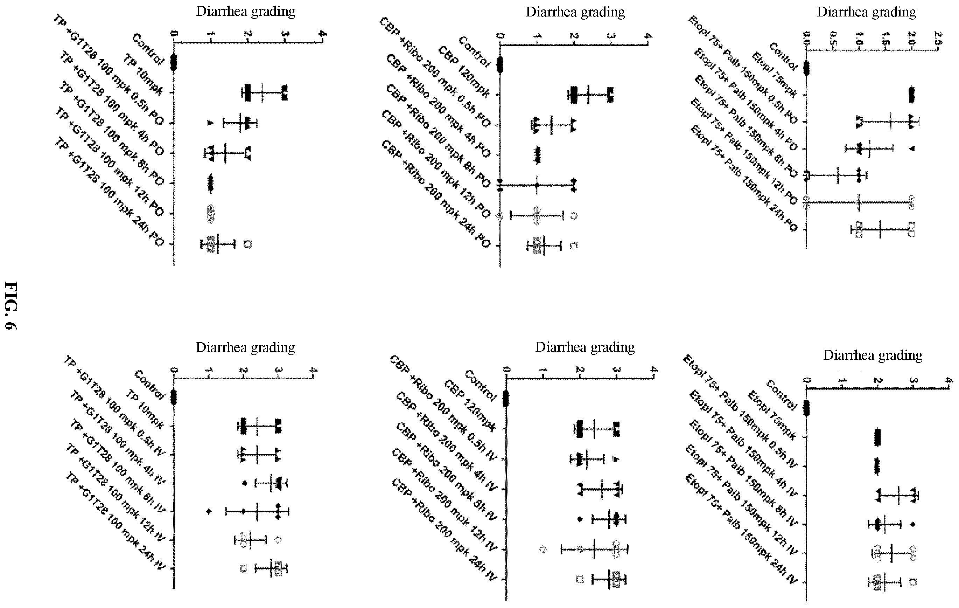

FIG. 6 shows the typical diarrhea grading results of the control group, the chemotherapeutic agent group, the oral administration group of the CDK inhibitor and the intravenous injection group of the CDK inhibitor in Table 6. It can be seen from Table 6 and FIG. 6 that, the oral administration and injection administration of palbociclib, abemaciclib, trilaciclib, ribociclib, SHR-6390, dinaciclib, KR-0601, riviciclib can both effectively relieve diarrhea caused by etoposide, etoposide in combination with cisplatin, carboplatin, gemcitabine, gemcitabine in combination with carboplatin, and topotecan, and oral administration has the most significant effect.

### Examples 382-405 Various administration modes of the CDK inhibitor can relieve diarrhea caused by a chemotherapeutic agent

Balb/c mice were fed for 1 week, and then divided into groups. The experiment was divided into a control group, a chemotherapeutic agent group, CDK inhibitor groups (including an oral administration group of a CDK inhibitor, an intraperitoneal injection group of a CDK inhibitor, an intramuscular injection group of a CDK inhibitor, a local administration group of a CDK inhibitor), each of which comprised 10 mice, to perform the administration experiment, for which the administration dosage, mode, time and frequency were shown in Table 7. The control group: being administered with the same solvent as that used in the CDK inhibitor group (the time and mode were shown in Table 7), and then being injected/intragastrically administered with the same solvent as that used in the chemotherapeutic agent group (the administration mode was shown in Table 7); the chemotherapeutic agent group: being administered with the same solvent as that used in the CDK inhibitor group (the time and mode were shown in Table 7), and then being injected/intragastrically administered with the chemotherapeutic agent (the type, administration mode and dosage were shown in Table 7); the oral administration group of the CDK inhibitor: being intragastrically administered with the CDK inhibitor (the time was shown in Table 7), and then being administered with the chemotherapeutic agent (the mode and dosage were shown in Table 7); the intraperitoneal injection group of the CDK inhibitor: being intraperitoneally injected with the CDK inhibitor (the time was shown in Table 7), and then being administered with the chemotherapeutic agent (the mode and dosage were shown in Table 7); the intramuscular injection group of the CDK inhibitor: being intramuscularly injected with the CDK inhibitor (the time was shown in Table 7), and then being administered with the chemotherapeutic agent (the mode and dosage were shown in Table 7); and the local administration group of the CDK inhibitor: the hair on the abdomen of mice was shaved (about 2 cm*2 cm), being applied with the CDK inhibitor (the time was shown in Table 7), and then being administered with the chemotherapeutic agent (the mode and dosage were shown in Table 7).

### Diarrhea observation

The scoring of diarrhea can refer to the method of Akinobu Kurita (Cancer Chemother Pharmacol 2000; 46:211-20.), Grade 0: normal feces; Grade 1: mild diarrhea, the stools is slightly wet and soft; Grade 2: moderate diarrhea, loose stools and mild perianal contamination; Grade 3: severe diarrhea, watery stools and accompanied by severe perianal staining (see FIG. 1). It is regarded as effective remission when the diarrhea grading of mice in the CDK inhibitor group is reduced compared with the average diarrhea grading of mice in the chemotherapeutic agent group.

The diarrhea grading of mice was observed and recorded every day. At the end of the experiment, the number of mice in the CDK inhibitor group whose diarrhea grading was lower than that of mice in the chemotherapeutic agent group was recorded. Table 7 lists various animal experiment combinations of chemotherapeutic agents and CDK inhibitors, as well as the corresponding experimental results. (Relative remission rate%= the number of mice in the CDK inhibitor group whose diarrhea has been effectively relieved/the total number of mice in the CDK inhibitor group * 100%. The establishment rate of diarrhea model in the chemotherapeutic agent group was 50%-70%, that is, about 5-7 of 10 mice developed diarrhea during the experiment, and there were cases of individual mice death or not developing diarrhea.)

**Table 7: Experimental Conditions and Results of Examples 382-405**

| Ex.* | Chem o.* | Admi n.m.* | Dos and Freq* | Ex. cy cle (s) * | CDK i* | Ad min . t.* | Adm in.m * | Dos and Freq* | Rel. rem. oral (%)* | Other admin. m.* | Dos and Freq* | Rel. rem. other (%)* |
|---|---|---|---|---|---|---|---|---|---|---|---|---|
| 382 | DTX | I.P. | 10, S | 8 | Palbo | 0.5 h | Oral | 150, S | 40 | I.P. | 150, S | 10 |
| 383 | DTX | I.P. | 10, S | 8 | Palbo | 4 h | Oral | 150, S | 40 | I.P. | 150, S | 10 |
| 384 | DTX | I.P. | 10, S | 8 | Palbo | 8 h | Oral | 150, S | 50 | I.P. | 150, S | 10 |
| 385 | DTX | I.P. | 10, S | 8 | Palbo | 12 h | Oral | 150, S | 70 | I.P. | 150, S | 20 |
| 386 | DTX | I.P. | 10, S | 8 | Palbo | 24 h | Oral | 150, S | 50 | I.P. | 150, S | 10 |
| 387 | DTX | I.V. | 10, S | 8 | SHR-6390 | 1 h | Oral | 150, S | 40 | I.M. | 150, S | 10 |
| | DTX | I.V. | 10, S | 8 | SHR-6390 | 1 h | Oral | 150, S | 40 | I.M. | 150, S | 10 |
| 388 | DTX | I.M. | 20, S | 8 | SHR-6390 | 0.5 h | Oral | 150, S | 40 | Local | 150, S | 10 |
| | DTX | I.M. | 20, S | 8 | SHR-6390 | 0.5 h | Oral | 150, S | 40 | Local | 150, S | 10 |
| 389 | CBP | I.P. | 120, S | 10 | Ribo | 0.5 h | Oral | 200, S | 30 | I.P. | 200, S | 10 |
| 390 | CBP | I.P. | 120, S | 10 | Ribo | 4 h | Oral | 200, S | 50 | I.P. | 200, S | 10 |
| 391 | CBP | I.P. | 120, S | 10 | Ribo | 8 h | Oral | 200, S | 50 | I.P. | 200, S | 10 |
| 392 | CBP | I.P. | 120, S | 10 | Ribo | 12 h | Oral | 200, S | 40 | I.P. | 200, S | 20 |
| 393 | CBP | I.P. | 120, S | 10 | Ribo | 24 h | Oral | 200, S | 40 | I.P. | 200, S | 10 |
| 394 | CBP | I.V. | 120, S | 10 | Abema | 8h | Oral | 150, S | 60 | I.M. | 150, S | 20 |
| | CBP | I.V. | 120, S | 10 | Abema | 8h | Oral | 150, S | 60 | I.M. | 150, S | 20 |
| 395 | CBP | Oral | 200, S | 10 | G1T28 | 24 h | Oral | 100, S | 60 | Local | 100, S | 20 |
| | CBP | Oral | 200, S | 10 | G1T28 | 24 h | Oral | 100, S | 60 | Local | 100, S | 20 |
| 396 | GEM | I.P. | 120, S | 8 | Abema | 0.5 h | Oral | 150, S | 40 | I.P. | 150, S | 10 |
| 397 | GEM | I.P. | 120, S | 8 | Abema | 4h | Oral | 150, S | 50 | I.P. | 150, S | 20 |
| 398 | GEM | I.P. | 120, S | 8 | Abema | 8h | Oral | 150, S | 60 | I.P. | 150, S | 30 |
| 399 | GEM | I.P. | 120, S | 8 | Abema | 12 h | Oral | 150, S | 60 | I.P. | 150, S | 30 |
| 400 | GEM | I.P. | 120, S | 8 | Abema | 24 h | Oral | 150, S | 40 | I.P. | 150, S | 10 |
| 401 | GEM | Oral | 300, S | 8 | Ribo | 24 h | Oral | 100, S | 50 | Local | 100, S | 20 |
| | GEM | Oral | 300, S | 8 | Ribo | 24 h | Oral | 100, S | 50 | Local | 100, S | 20 |
| 402 | GEM | I.M. | 120, S | 8 | G1T28 | 24 h | Oral | 50, S | 40 | I.M. | 50, S | 10 |
| | GEM | I.M. | 120, S | 8 | G1T28 | 24 h | Oral | 50, S | 40 | I.M. | 50, S | 10 |
| 403 | GEM | I.V. | 120, S | 8 | Palbo | 12 h | Oral | 200, S | 60 | I.V. | 200, S | 20 |
| | GEM | I.V. | 120, S | 8 | Palbo | 12 h | Oral | 200, S | 60 | I.V. | 200, S | 20 |
| 404 | GEM /CBP | I.P. | 120 (G), 100 (C), S | 8 | G1T28 | 12 h | Oral | 150, S | 60 | I.M. | 100, S | 20 |
| | GEM /CBP | I.P. | 120 (G), 100 (C), S | 8 | G1T28 | 12 h | Oral | 150, S | 60 | I.M. | 100, S | 20 |
| 405 | GEM /CBP | I.P. | 120 (G), 100 (C), S | 8 | G1T28 | 24 h | Oral | 150, S | 50 | I.M. | 100, S | 10 |

| | | | | | | | | | | | | |
|---|---|---|---|---|---|---|---|---|---|---|---|---|
| Ex.*: Example No.; Chemo*: Chemotherapeutic agent; Admin.m.*: Administration mode; Dos. And Freq*: Dosage (mg/kg) and Frequency; Ex. Cycle (d):Experimental cycle (days); CDK. i*; CDK inhibitor; Freq*: Admin.t.*: Administration time (before the chemotherapeutic agent); Rel.rem.oral(%)*: Relative remission rate in oral administration of CDK inhibitor (%); Other admin.m.: Other administration mode; Rel.rem.other(%)*: Relative remission rate of other administration mode (%); S: Single; ; DTX: Doceataxel; CBP: Carboplatin; GEM: Gemcitabine; Palbo: Palbociclib; Ribo: Ribociclib; Abema: Abemaciclib; I.P.: Intraperitoneal injection; I.V: Intravenous injection; I.M.: Intramuscular injection; Oral: Oral adiminstratio; Local: Local administration. | | | | | | | | | | | | |

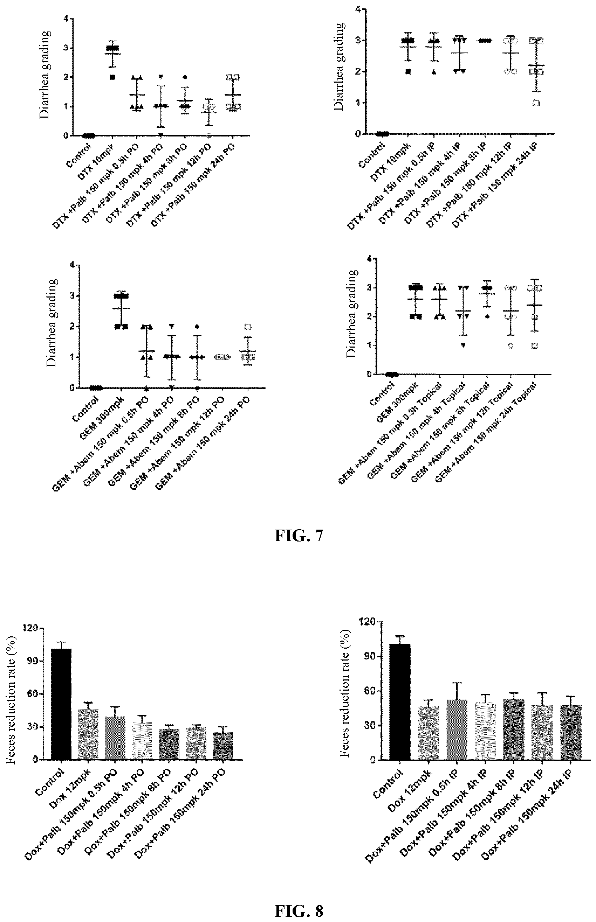

FIG. 7 shows the comparison of diarrhea grading results of the control group, the chemotherapeutic agent group, the oral administration group of the CDK inhibitor and other administration mode group of the CDK inhibitor in Table 7.

It can be seen from the results in Table 7 and FIG. 7 that, the oral administration, injection administration and local administration of palbociclib, abemaciclib, trilaciclib, ribociclib, SHR-6390 under certain conditions all can relieve diarrhea caused by docetaxel, carboplatin, gemcitabine, and the combination of gemcitabine and carboplatin, and the effect of oral administration is the most significant.

### Examples 406-443 Various administration modes of the CDK inhibitor can relieve constipation caused by a chemotherapeutic agent

Balb/c mice were fed for 1 week, and then divided into groups. The experiment was divided into a control group, a chemotherapeutic agent group, CDK inhibitor groups (including an oral administration group of a CDK inhibitor, an intravenous injection group of a CDK inhibitor, an intraperitoneal injection group of a CDK inhibitor, an intramuscular injection group of a CDK inhibitor, a local administration group of a CDK inhibitor), each of which comprised 10 mice, to perform the administration experiment, for which the administration dosage, mode, time and frequency were shown in Table 8. The control group: being administered with the same solvent as that used in the CDK inhibitor group (the time and mode were shown in Table 8), and then being injected/intragastrically administered with the same solvent as that used in the chemotherapeutic agent group (the administration mode was shown in Table 8); the chemotherapeutic agent group: being administered with the same solvent as that used in the CDK inhibitor group (the time and mode were shown in Table 8), and then being injected/intragastrically administered with the chemotherapeutic agent (the type, administration mode and dosage were shown in Table 8); the oral administration group of the CDK inhibitor: being intragastrically administered with the CDK inhibitor (the time was shown in Table 8), and then being administered with the chemotherapeutic agent (the mode and dosage were shown in Table 8); the intravenous injection group of the CDK inhibitor: being injected with the CDK inhibitor through tail vein (the time was shown in Table 8), and then being administered with the chemotherapeutic agent (the mode and dosage were shown in Table 8); the intraperitoneal injection group of the CDK inhibitor: being intraperitoneally injected with the CDK inhibitor (the time was shown in Table 8), and then being administered with the chemotherapeutic agent (the mode and dosage were shown in Table 8); the intramuscular injection group of the CDK inhibitor: being intramuscularly injected with the CDK inhibitor (the time was shown in Table 8), and then being administered with the chemotherapeutic agent (the mode and dosage were shown in Table 8); and the local administration group of the CDK inhibitor: the hair on the abdomen of mice was shaved (about 2 cm*2 cm), being applied with the CDK inhibitor (the time was shown in Table 8), and then being administered with the chemotherapeutic agent (the mode and dosage were shown in Table 8).

### Constipation observation

The feces of mice within 3 hours were collected, which were observed for shape and weighed with an electronic balance. The feces reduction rate was calculated with reference to the method of Ji Eun Kim (Lab Anim Res. 2016 Dec; 32(4): 231-240.). Feces reduction rate (%) = (the control group - the chemotherapeutic agent group or the CDK inhibitor group)/the control group*100%

It is regarded as effective remission when the feces reduction rate of mice in the CDK inhibitor group is reduced compared with that in the chemotherapeutic agent group.

The feces amount of mice within 3 hours was observed and recorded every day. At the end of the experiment, the number of mice in the CDK inhibitor group whose feces reduction rate was lower than that of mice in the chemotherapeutic agent group was recorded.

Table 8 lists various animal experiment combinations of chemotherapeutic agents and CDK inhibitors, as well as the corresponding experimental results. (Relative remission rate%= the number of mice in the CDK inhibitor group which have been effectively relieved/the total number of mice in the CDK inhibitor group * 100%. The establishment rate of constipation model in the chemotherapeutic agent group was 30%-60%, that is, about 3-6 of 10 mice showed reduced feces amount during the experiment, and there were cases of individual mice death or with no changes or an increasement in the feces amount.)

**Table 8: Experimental Conditions and Results of Examples 406-443**

| Ex.* | Chem o.* | Adm in.m. * | Dos and Freq* | Ex .cy cle (d) * | CDK i* | Ad min. t.* | Ad min. m.* | Dos and Freq* | Rel. rem. oral (%)* | Othe r admi n.m. * | Dos and Freq* | Rel. rem. other (%)* |
|---|---|---|---|---|---|---|---|---|---|---|---|---|
| 406 | Dox | I.P. | 12, S | 8 | Palbo | 0.5 h | Oral | 150, S | 20 | I.V. | 150, S | 10 |
| 407 | Dox | I.P. | 12, S | 8 | Palbo | 4 h | Oral | 150, S | 30 | I.V. | 150, S | 10 |
| 408 | Dox | I.P. | 12, S | 8 | Palbo | 8 h | Oral | 150, S | 30 | I.V. | 150, S | 10 |
| 409 | Dox | I.P. | 12, S | 8 | Palbo | 12 h | Oral | 150, S | 50 | I.V. | 150, S | 20 |
| 410 | Dox | I.P. | 12, S | 8 | Palbo | 24 h | Oral | 150, S | 40 | I.V. | 150, S | 20 |
| 411 | Dox | I.V. | 12, S | 8 | G1T28 | 24 h | Oral | 100, S | 30 | I.P. | 100, S | 10 |
| | Dox | I.V. | 12, S | 8 | G1T28 | 24 h | Oral | 100, S | 30 | I.P. | 100, S | 10 |
| 412 | Dox | I.M. | 12, S | 8 | SHR-6390 | 0.5 h | Oral | 100, S | 50 | Local | 100, S | 20 |
| 413 | Dox | I.M. | 12, S | 8 | SHR-6390 | 12 h | Oral | 100, S | 50 | Local | 100, S | 20 |
| 414 | Dox | I.M. | 12, S | 8 | SHR-6390 | 24 h | Oral | 100, S | 40 | Local | 100, S | 10 |
| 415 | Etopl | I.P. | 100, S | 8 | Palbo | 0.5 h | Oral | 150, S | 20 | I.V. | 150, S | 10 |
| 416 | Etopl | I.P. | 100, S | 8 | Palbo | 4 h | Oral | 150, S | 40 | I.V. | 150, S | 20 |
| 417 | Etopl | I.P. | 100, S | 8 | Palbo | 8 h | Oral | 150, S | 40 | I.V. | 150, S | 20 |
| 418 | Etopl | I.P. | 100, S | 8 | Palbo | 12 h | Oral | 150, S | 30 | I.V. | 150, S | 10 |
| 419 | Etopl | I.P. | 100, S | 8 | Palbo | 24 h | Oral | 150, S | 30 | I.V. | 150, S | 10 |
| 420 | Etopl | I.V. | 100, S | 8 | Doni | 1 h | Oral | 1, S | 20 | I.P. | 100, S | 10 |
| | Etopl | I.V. | 100, S | 8 | Doni | 1 h | Oral | 1, S | 20 | I.P. | 100, S | 10 |
| 421 | Etopl /Cis | I.P. | 50 (E), 5, S | 7 | G1T28 | 24 h | Oral | 100, S | 20 | I.V. | 100, S | 10 |
| | Etopl/ Cis | I.P. | 51 (E), 5, S | 7 | G1T28 | 24 h | Oral | 100, S | 20 | I.V. | 100, S | 10 |
| | Etopl /Cis | I.P. | 52 (E), 5, S | 7 | G1T28 | 24 h | Oral | 100, S | 20 | I.V. | 100, S | 10 |
| 422 | Etopl /Cis | I.V. | 50 and 5, S | 7 | Palbo | 0.5 h | Oral | 150, S | 40 | I.V. | 150, S | 10 |
| 423 | Etopl /Cis | I.V. | 51 and 5, S | 7 | Palbo | 24 h | Oral | 150, S | 60 | I.V. | 150, S | 20 |
| 424 | CBP | I.P. | 120, S | 10 | Ribo | 0.5 h | Oral | 200, S | 20 | I.P. | 200, S | 10 |
| 425 | CBP | I.P. | 120, S | 10 | Ribo | 4 h | Oral | 200, S | 30 | I.P. | 200, S | 10 |
| 426 | CBP | I.P. | 120, S | 10 | Ribo | 8 h | Oral | 200, S | 50 | I.P. | 200, S | 20 |
| 427 | CBP | I.P. | 120, S | 10 | Ribo | 12 h | Oral | 200, S | 50 | I.P. | 200, S | 20 |
| 428 | CBP | I.P. | 120, S | 10 | Ribo | 24 h | Oral | 200, S | 40 | I.P. | 200, S | 10 |
| 429 | CBP | I.V. | 120, S | 10 | Abema | 8 h | Oral | 150, S | 60 | I.M. | 150, S | 20 |
| | CBP | I.V. | 120, S | 10 | Abema | 8 h | Oral | 150, S | 60 | I.M. | 150, S | 20 |
| 430 | GEM | I.P. | 120, S | 8 | Abema | 0.5 h | Oral | 150, S | 40 | I.P. | 150, S | 10 |
| 431 | GEM | I.P. | 120, S | 8 | Abema | 4 h | Oral | 150, S | 40 | I.P. | 150, S | 10 |
| 432 | GEM | I.P. | 120, S | 8 | Abema | 8 h | Oral | 150, S | 40 | I.P. | 150, S | 10 |
| 433 | GEM | I.P. | 120, S | 8 | Abema | 12 h | Oral | 150, S | 60 | I.P. | 150, S | 20 |
| 434 | GEM | I.P. | 120, S | 8 | Abema | 24 h | Oral | 150, S | 60 | I.P. | 150, S | 20 |
| 435 | GEM | Oral | 300, S | 8 | Ribo | 24 h | Oral | 100, S | 50 | Local | 100, S | 20 |
| | GEM | Oral | 300, S | 8 | Ribo | 24 h | Oral | 100, S | 50 | Local | 100, S | 20 |
| 436 | GEM | I.M. | 120, S | 8 | G1T28 | 24 h | Oral | 100, S | 40 | I.M. | 100, S | 10 |
| | GEM | I.M. | 120, S | 8 | G1T28 | 24 h | Oral | 100, S | 40 | I.M. | 100, S | 10 |
| 437 | GEM | I.V. | 120, S | 8 | Palbo | 12 h | Oral | 200, S | 50 | I.V. | 200, S | 20 |
| | GEM | I.V. | 120, S | 8 | Palbo | 12 h | Oral | 200, S | 50 | I.V. | 200, S | 20 |
| 438 | GEM/ CBP | I.V. | 120 (G), 100(C) S | 8 | G1T28 | 12 h | Oral | 100, S | 50 | I.P. | 100, S | 20 |
| | GEM/ CBP | I.V. | 121 (G), 100(C) S | 8 | G1T28 | 12 h | Oral | 100, S | 50 | I.P. | 100, S | 20 |
| 439 | GEM/ CBP | I.V. | 122 (G), 100(C) S | 8 | G1T28 | 24 h | Oral | 100, S | 50 | I.P. | 100, S | 20 |
| 440 | GEM/ CBP | I.P. | 120 (G), 100(C) S | 8 | SHR-6390 | 1 h | Oral | 100, S | 40 | I.M. | 100, S | 10 |
| | GEM/ CBP | I.P. | 121 (G), 100(C) S | 8 | SHR-6390 | 1 h | Oral | 100, S | 40 | I.M. | 100, S | 10 |
| 441 | GEM/ CBP | I.P. | 122 (G), 100(C) S | 8 | SHR-6390 | 12 h | Oral | 100, S | 60 | I.M. | 100, S | 20 |
| 442 | GEM/ CBP | Oral | 300 (G), 200 (C), S | 8 | Seli | 4 h | Oral | 400, S | 20 | Local | 400, S | 10 |
| | GEM /CBP | Oral | 301 (G), 200 (C), S | 8 | Seli | 4 h | Oral | 400, S | 20 | Local | 400, S | 10 |
| 443 | GEM /CBP | Oral | 302 (G), 200 (C), S | 8 | Seli | 8 h | Oral | 400, S | 20 | Local | 400, S | 10 |

| | | | | | | | | | | | | |
|---|---|---|---|---|---|---|---|---|---|---|---|---|
| Ex.*: Example No.; Chemo*: Chemotherapeutic agent; Admin.m.*: Administration mode; Dos. And Freq*: Dosage (mg/kg) and Frequency; Ex. Cycle (d):Experimental cycle (days); CDK. i*; CDK inhibitor; Freq*: Admin.t.*: Administration time (before the chemotherapeutic agent); Rel.rem.oral(%)*: Relative remission rate in oral administration of CDK inhibitor (%); Other admin.m.: Other administration mode; Rel.rem.other(%)*: Relative remission rate of other administration mode (%); S: Single; Dox: Doxorubicin; Etolp: Etoposide; Cis: Cisplatin; CBP: Carboplatin; GEM: Gemcitabine; Palbo: Palbociclib; Doni: Doniciclib; Ribo: Ribociclib; Abema: Abemaciclib; Seli: Seliciclib; I.P.: Intraperitoneal injection; I.V: Intravenous injection; I.M.: Intramuscular injection; Oral: Oral adiminstratio; Local: Local administration. | | | | | | | | | | | | |

FIG. 8 shows the comparison of feces reduction rate results of the control group, the chemotherapeutic agent group, the oral administration group of the CDK inhibitor and other administration mode group of the CDK inhibitor in Table 8.

It can be seen from the results in Table 8 and FIG. 8 that, the oral administration, intraperitoneal injection, intramuscular injection, local administration of Palbociclib, Abemaciclib, Trilaciclib, Ribociclib, SHR-6390, Doniciclib, Seliciclib under certain conditions all can significantly improve constipation caused by doxorubicin, etoposide, the combination of etoposide and cisplatin, carboplatin, gemcitabine, and the combination of gemcitabine and carboplatin, and the relieving effect of oral administration is the most significant.

### Examples 444-469 Relieving effect of oral administration of a CDK inhibitor on diarrhea caused by the continuous administration of a chemotherapeutic agent

Balb/c mice were fed for 1 week, and then divided into groups. The experiment was divided into a control group, a chemotherapeutic agent group, and a CDK inhibitor group, each of which comprised 10 mice, to perform the administration experiment, for which the administration dosage, mode, time and frequency were shown in Table 9. The control group: being intragastrically administered with the same solvent as that used in the CDK inhibitor group (the time was shown in Table 9), and then being injected/intragastrically administered with the same solvent as that used in the chemotherapeutic agent group (the administration mode was shown in Table 9); the chemotherapeutic agent group: being intragastrically administered with the same solvent as that used in the CDK inhibitor group (the time was shown in Table 9), and then being injected/intragastrically administered with the chemotherapeutic agent (the type, administration mode, dosage and duration time were shown in Table 9); the CDK inhibitor group: being intragastrically administered with the CDK inhibitor (the time was shown in Table 9), and then being administered with the chemotherapeutic agent (the type, administration mode, dosage and duration time were shown in Table 9).

### Diarrhea observation

The scoring of diarrhea can refer to the method of Akinobu Kurita (Cancer Chemother Pharmacol 2000; 46:211-20.), Grade 0: normal feces; Grade 1: mild diarrhea, the stools is slightly wet and soft; Grade 2: moderate diarrhea, loose stools and mild perianal contamination; Grade 3: severe diarrhea, watery stools and accompanied by severe perianal staining (see FIG. 1). It is regarded as effective remission when the diarrhea grading of mice in the CDK inhibitor group is reduced compared with the average diarrhea grading of mice in the chemotherapeutic agent group.

The diarrhea grading of mice was observed and recorded every day. At the end of the experiment, the number of mice in the CDK inhibitor group whose diarrhea grading was lower than that of mice in the chemotherapeutic agent group was recorded. Table 9 lists various animal experiment combinations of chemotherapeutic agents and CDK inhibitors, as well as the corresponding experimental results. (Relative remission rate%= the number of mice in the CDK inhibitor group whose diarrhea has been effectively relieved/the total number of mice in the CDK inhibitor group * 100%. The establishment rate of diarrhea model in the chemotherapeutic agent group was 50%-80%, that is, about 5-8 of 10 mice developed diarrhea during the experiment, and there were cases of individual mice death or not developing diarrhea.)

**Table 9: Experimental Conditions and Results of Examples 444-469**

| Ex. * | Chemo. * | Admin .m.* | Dos.* | Freq. * | CDK i.* | Adm in.t. * | Admin .m.* | Dos. * | Freq. * | Ex. cycle (d) | Rel. rem (%) |
|---|---|---|---|---|---|---|---|---|---|---|---|
| 444 | 5-FU | I.P. | 70 | Daily, 3d | Palbo | 4 h | Oral | 150 | Daily, 3d | 10 | 70 |
| 445 | 5-FU | I.P. | 70 | Daily, 3d | Palbo | 6 h | Oral | 150 | Daily, 3d | 10 | 70 |
| 446 | 5-FU | I.P. | 70 | Daily, 3d | Palbo | 8 h | Oral | 150 | Daily, 3d | 10 | 70 |
| 447 | 5-FU | I.P. | 70 | Daily, 3d | Palbo | 12 h | Oral | 150 | Daily, 3d | 10 | 70 |
| 448 | 5-FU | I.P. | 70 | Daily, 5d | Palbo | 4 h | Oral | 150 | Daily, 5d | 9 | 60 |
| 449 | 5-FU | I.P. | 70 | Daily, 5d | Palbo | 6 h | Oral | 150 | Daily, 5d | 9 | 60 |
| 450 | 5-FU | I.P. | 70 | Daily, 5d | Palbo | 8 h | Oral | 150 | Daily, 5d | 9 | 60 |
| 451 | 5-FU | I.P. | 70 | Daily, 5d | Palbo | 12 h | Oral | 150 | Daily, 5d | 9 | 60 |
| 452 | 5-FU | I.P. | 70 | Daily, 5d | G1T28 | 2 h | Oral | 125 | Daily, 5d | 10 | 10 |
| 453 | 5-FU | I.P. | 70 | Daily, 7d | G1T28 | 4 h | Oral | 125 | Daily, 7d | 10 | 10 |
| 454 | 5-FU | I.P. | 70 | Daily, 7d | G1T28 | 6 h | Oral | 125 | Daily, 7d | 10 | 10 |
| 455 | 5-FU | I.P. | 70 | Daily, 7d | G1T28 | 8 h | Oral | 125 | Daily, 7d | 10 | 10 |
| 456 | 5-FU | I.V. | 70 | Daily, 3d | Ribo | 4 h | Oral | 200 | Daily, 3d | 8 | 80 |
| 457 | 5-FU | Oral | 50 | Daily, 14d | Abema | 8 h | Oral | 150 | Daily, 14d | 14 | 0 |
| 458 | CAP | Oral | 1000 | Daily, 5d | G1T28 | 2 h | Oral | 100 | Daily, 5d | 14 | 50 |
| 459 | CAP | Oral | 1000 | Daily, 5d | G1T28 | 4 h | Oral | 100 | Daily, 5d | 14 | 50 |
| 460 | CAP | Oral | 1000 | Daily, 5d | G1T28 | 6 h | Oral | 100 | Daily, 5d | 14 | 50 |
| 461 | CAP | Oral | 1000 | Daily, 5d | G1T28 | 8 h | Oral | 100 | Daily, 5d | 14 | 50 |
| 462 | CAP | Oral | 1000 | Daily, 7d | Palbo | 2 h | Oral | 150 | Daily, 7d | 14 | 0 |
| 463 | CAP | Oral | 1000 | Daily, 7d | Palbo | 4 h | Oral | 150 | Daily, 7d | 14 | 0 |
| 464 | CAP | Oral | 1000 | Daily, 7d | Palbo | 6 h | Oral | 150 | Daily, 7d | 14 | 0 |
| 465 | CAP | Oral | 1000 | Daily, 7d | Palbo | 8 h | Oral | 150 | Daily, 7d | 14 | 0 |
| 466 | CAP | Oral | 1000 | Daily, 14d | Palbo | 2 h | Oral | 150 | Daily, 14d | 14 | 10 |
| 467 | CAP | Oral | 1000 | Daily, 14d | Palbo | 4 h | Oral | 150 | Daily, 14d | 14 | 10 |
| 468 | CAP | Oral | 1000 | Daily, 14d | Palbo | 6 h | Oral | 150 | Daily, 14d | 14 | 10 |
| 469 | CAP | Oral | 1000 | Daily, 14d | Palbo | 8 h | Oral | 150 | Daily, 14d | 14 | 10 |

| | | | | | | | | | | | |
|---|---|---|---|---|---|---|---|---|---|---|---|
| Ex.*: Example No.; Chemo*: Chemotherapeutic agent; Admin.m.*: Administration mode; Dos*: Dosage (mg/kg); Freq*: Frequency; CDK. i*; CDK inhibitor; Admin.t.*: Administration time (before the chemotherapeutic agent); Ex. Cycle (d):Experimental cycle (days); Rel.rem.(%)*: Relative remission rate (%); S: Single; 5-FU: Fluorouracil; CAP: Capecitabine; Palbo: Palbociclib; Ribo: Ribociclib; Abema: Abemaciclib; I.P.: Intraperitoneal injection; I.V: Intravenous injection; Oral: Oral administration; Daily, 3d: Once a day, for 3 days. | | | | | | | | | | | |

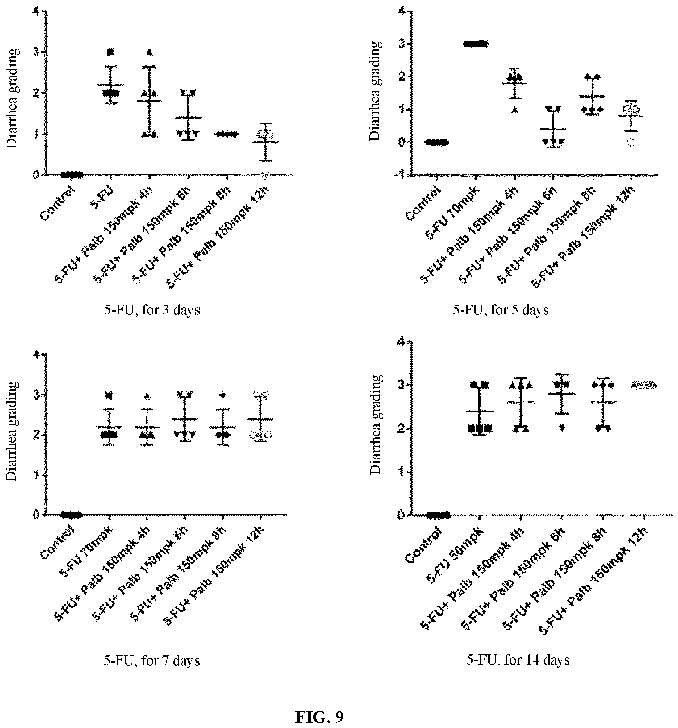

FIG. 9 shows the comparison on the partial results of diarrhea grading in the control group, the chemotherapeutic agent group, and the CDK inhibitor group in Table 9.

It can be seen from Table 9 and FIG. 9 that: Palbociclib, Abemaciclib, Trilaciclib, and Ribociclib all have significant relieving effects on diarrhea caused by the continuous administration of fluorouracil for less than 7 days, but their relieving effects on diarrhea caused by the continuous administration of fluorouracil and capecitabine for 7 days or above are not significant.

### Examples 470-481 Relieving effect of oral administration of a CDK inhibitor on constipation caused by the continuous administration of a chemotherapeutic agent

Balb/c mice were fed for 1 week, and then divided into groups. The experiment was divided into a control group, a chemotherapeutic agent group, and a CDK inhibitor group, each of which comprised 10 mice, to perform the administration experiment, for which the administration dosage, mode, time and frequency were shown in Table 10. The control group: being intragastrically administered with the same solvent as that used in the CDK inhibitor group (the time was shown in Table 10), and then being injected/intragastrically administered with the same solvent as that used in the chemotherapeutic agent group (the administration mode was shown in Table 10); the chemotherapeutic agent group: being intragastrically administered with the same solvent as that used in the CDK inhibitor group (the time was shown in Table 10), and then being injected/intragastrically administered with the chemotherapeutic agent (the type, administration mode, dosage and duration time were shown in Table 10); the CDK inhibitor group: being intragastrically administered with the CDK inhibitor (the time was shown in Table 10), and then being administered with the chemotherapeutic agent (the type, administration mode, dosage and duration time were shown in Table 10).

### Constipation observation

The feces of mice within 3 hours were collected, which were observed for shape and weighed with an electronic balance. The feces reduction rate was calculated with reference to the method of Ji Eun Kim (Lab Anim Res. 2016 Dec; 32(4): 231-240.). Feces reduction rate (%) = (the control group - the chemotherapeutic agent group or the CDK inhibitor group)/the control group*100%

It is regarded as effective remission when the feces reduction rate of mice in the CDK inhibitor group is reduced compared with that in the chemotherapeutic agent group.

The feces amount of mice within 3 hours was observed and recorded every day. At the end of the experiment, the number of mice in the CDK inhibitor group whose feces reduction rate was lower than that of mice in the chemotherapeutic agent group was recorded. Table 10 lists various animal experiment combinations of chemotherapeutic agents and CDK inhibitors, as well as the corresponding experimental results. (Relative remission rate%= the number of mice in the CDK inhibitor group which have been effectively relieved/the total number of mice in the CDK inhibitor group * 100%. The establishment rate of constipation model in the chemotherapeutic agent group was 30%-60%, that is, about 3-6 of 10 mice showed reduced feces amount during the experiment, and there were cases of individual mice death or with no changes or an increasement in the feces amount.)

**Table 10: Experimental Conditions and Results of Examples 470-481**

| Ex. * | Chemo. * | Adm in.m. * | Dos. * | Freq. * | CDK i.* | Ad min. t.* | Adm in.m. * | Dos. * | Freq. * | Ex. cycle (d) | Rel.rem (%) |
|---|---|---|---|---|---|---|---|---|---|---|---|
| 470 | 5-FU | I.P. | 100 | Daily, 3d | Palbo | 2 h | Oral | 150 | Daily, 3d | 10 | 40 |
| 471 | 5-FU | I.P. | 100 | Daily, 3d | Palbo | 4 h | Oral | 150 | Daily, 3d | 10 | 40 |
| 472 | 5-FU | I.P. | 100 | Daily, 3d | Palbo | 6 h | Oral | 150 | Daily, 3d | 10 | 40 |
| 473 | 5-FU | I.P. | 100 | Daily, 3d | Palbo | 8 h | Oral | 150 | Daily, 3d | 10 | 40 |
| 474 | 5-FU | I.P. | 100 | Daily, 5d | Palbo | 2 h | Oral | 125 | Daily, 5d | 9 | 50 |
| 475 | 5-FU | I.P. | 100 | Daily, 5d | Palbo | 4 h | Oral | 125 | Daily, 5d | 9 | 50 |
| 476 | 5-FU | I.P. | 100 | Daily, 5d | Palbo | 6 h | Oral | 125 | Daily, 5d | 9 | 50 |
| 477 | 5-FU | I.P. | 100 | Daily, 5d | Palbo | 8 h | Oral | 125 | Daily, 5d | 9 | 50 |
| 478 | 5-FU | I.P. | 100 | Daily, 7d | Palbo | 2 h | Oral | 125 | Daily, 7d | 10 | 10 |
| 479 | 5-FU | I.P. | 100 | Daily, 7d | Palbo | 4 h | Oral | 125 | Daily, 7d | 10 | 10 |
| 480 | 5-FU | I.P. | 100 | Daily, 7d | Palbo | 6 h | Oral | 125 | Daily, 7d | 10 | 10 |
| 481 | 5-FU | I.P. | 100 | Daily, 7d | Palbo | 8 h | Oral | 125 | Daily, 7d | 10 | 10 |

| | | | | | | | | | | | |
|---|---|---|---|---|---|---|---|---|---|---|---|
| Ex.*: Example No.; Chemo*: Chemotherapeutic agent; Admin.m.*: Administration mode; Dos*: Dosage (mg/kg); Freq*: Frequency; CDK. i*; CDK inhibitor; Admin.t.*: Administration time (before the chemotherapeutic agent); Ex. Cycle (d):Experimental cycle (days); Rel.rem.(%)*: Relative remission rate (%); S: Single; 5-FU: Fluorouracil; Palbo: Palbociclib; I.P.: Intraperitoneal injection; Oral: Oral administration; Daily, 3d: Once a day, for 3 days. | | | | | | | | | | | |

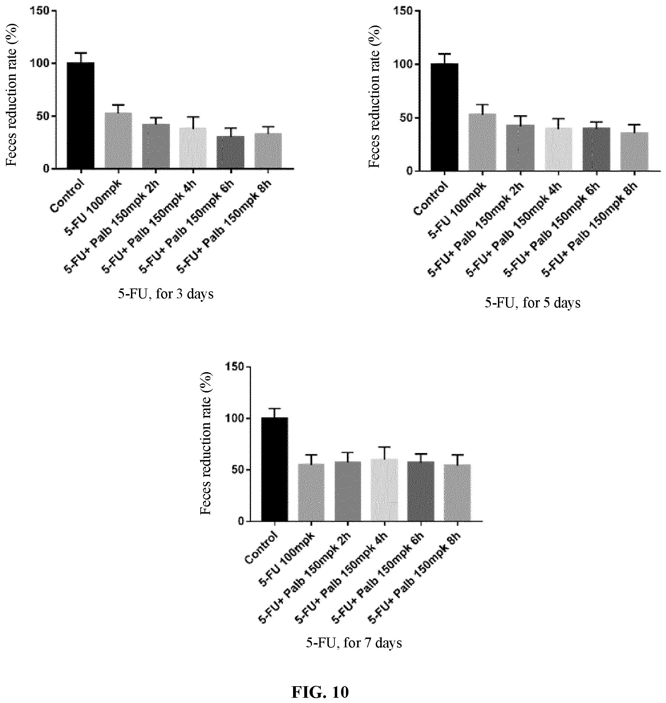

FIG. 10 shows the partial results of feces reduction rate in the control group, the chemotherapeutic agent group, and the CDK inhibitor group in Table 10.

It can be seen from Table 10 and FIG. 10 that: When being orally administered under certain conditions, Palbociclib has a significant relieving effect on constipation caused by the continuous administration of fluorouracil for less than 7 days, but its relieving effect on diarrhea caused by the continuous administration of fluorouracil for 7 days or above is not significant.

### Examples 482-485 Relieving effect of oral administration of a CDK inhibitor on diarrhea caused by other anti-tumor medicaments

Balb/c mice were fed for 1 week, and then divided into groups. The experiment was divided into a control group, an anti-tumor medicament group, and a CDK inhibitor group, each of which comprised 10 mice, to perform the administration experiment, for which the administration dosage, mode, time and frequency were shown in Table 11. The control group: being intragastrically administered with the same solvent as that used in the CDK inhibitor group (the time was shown in Table 11), and then being injected/intragastrically administered with the same solvent as that used in the anti-tumor medicament group (the administration mode was shown in Table 11); the anti-tumor medicament group: being intragastrically administered with the same solvent as that used in the CDK inhibitor group (the time was shown in Table 11), and then being injected/intragastrically administered with the anti-tumor medicament (the type, administration mode, dosage and duration time were shown in Table 11); the CDK inhibitor group: being intragastrically administered with the CDK inhibitor (the time was shown in Table 11), and then being administered with the anti-tumor medicament (the type, administration mode, dosage and duration time were shown in Table 11).

### Diarrhea observation

The scoring of diarrhea can refer to the method of Akinobu Kurita (Cancer Chemother Pharmacol 2000; 46:211-20.), Grade 0: normal feces; Grade 1: mild diarrhea, the stools is slightly wet and soft; Grade 2: moderate diarrhea, loose stools and mild perianal contamination; Grade 3: severe diarrhea, watery stools and accompanied by severe perianal staining (see FIG. 1). It is regarded as effective remission when the diarrhea grading of mice in the CDK inhibitor group is reduced compared with the average diarrhea grading of mice in the anti-tumor medicament group.

The diarrhea grading of mice was observed and recorded every day. At the end of the experiment, the number of mice in the CDK inhibitor group whose diarrhea grading was lower than that of mice in the anti-tumor medicament group was recorded. Table 11 lists various animal experiment combinations of anti-tumor medicaments and CDK inhibitors, as well as the corresponding experimental results. (Relative remission rate%= the number of mice in the CDK inhibitor group whose diarrhea has been effectively relieved/the total number of mice in the CDK inhibitor group * 100%. The establishment rate of diarrhea model in the chemotherapeutic agent group was 50%-80%, that is, about 5-8 of 10 mice developed diarrhea during the experiment, and there were cases of individual mice death or not developing diarrhea.)

**Table 11: Experimental Conditions and Results of Examples 482-485**

| Ex. * | Anti. medi. * | Target | Adm in.m * | Dos. * | Freq. * | CDK i. * | Ad min. t.* | Adm in.m. * | Dos. * | Freq. * | Ex. cycl e (d) | Rem. rate |
|---|---|---|---|---|---|---|---|---|---|---|---|---|
| 482 | Afat | EGFR | Oral | 25 | Daily, 14d | Palbo | 12 h | Oral | 150 | Daily, 14d | 14 | Ineff. |
| 483 | Idel | PI3K | Oral | 30 | Daily, 14d | Palbo | 12 h | Oral | 150 | Daily, 14d | 14 | Ineff. |
| 484 | Fiso | FGFR4 | Oral | 10 | Daily, 14d | Palbo | 12 h | Oral | 200 | Daily, 14d | 12 | Ineff. |
| 485 | Imat | TKI | Oral | 150 | Daily, 14d | Palbo | 12 h | Oral | 150 | Daily, 14d | 12 | Ineff. |

| | | | | | | | | | | | | |
|---|---|---|---|---|---|---|---|---|---|---|---|---|
| Ex.*: Example No.; Anti.medi: Anti-toumor medicament; Admin.m.*: Administration mode; Dos*: Dosage (mg/kg); Freq*: Frequency; CDK. i*; CDK inhibitor; Admin.t.*: Administration time (before the chemotherapeutic agent); Ex. Cycle (d):Experimental cycle (days); Rem.rate*: Remissions rate; Afat: Afatinib; Idel: Idelalisib; Fiso: Fisogatinib; Imat: Imattinib; Palbo: TKI: Tyrosine kinase inhibitor; Palbociclib; Oral: Oral administration; Daily, 14d: Once a day, for 14 days; Ineff: Ineffective. | | | | | | | | | | | | |

It can be seen from the experimental results in Table 11 that the CDK inhibitor has no relieving effect on diarrhea caused by the continuous administration of targeted anti-tumor medicaments.

### Examples 486-487 Relieving effect of oral administration of a CDK inhibitor on constipation caused by other anti-tumor medicaments

Balb/c mice were fed for 1 week, and then divided into groups. The experiment was divided into a control group, an anti-tumor medicament group, and a CDK inhibitor group, each of which comprised 10 mice, to perform the administration experiment, for which the administration dosage, mode, time and frequency were shown in Table 12. The control group: being intragastrically administered with the same solvent as that used in the CDK inhibitor group (the time was shown in Table 12), and then being injected/intragastrically administered with the same solvent as that used in the anti-tumor medicament group (the administration mode was shown in Table 12); the anti-tumor medicament group: being intragastrically administered with the same solvent as that used in the CDK inhibitor group (the time was shown in Table 12), and then being injected/intragastrically administered with the anti-tumor medicament (the type, administration mode, dosage and duration time were shown in Table 12); the CDK inhibitor group: being intragastrically administered with the CDK inhibitor (the time was shown in Table 12), and then being administered with the anti-tumor medicament (the type, administration mode, dosage and duration time were shown in Table 12).

### Constipation observation

The feces of mice within 3 hours were collected, which were observed for shape and weighed with an electronic balance. The feces reduction rate was calculated with reference to the method of Ji Eun Kim (Lab Anim Res. 2016 Dec; 32(4): 231-240.). Feces reduction rate (%) = (the control group - the anti-tumor medicament group or the CDK inhibitor group)/the control group*100%

It is regarded as effective remission when the feces reduction rate of mice in the CDK inhibitor group is reduced compared with that in the anti-tumor medicament group.

The feces amount of mice within 3 hours was observed and recorded every day. At the end of the experiment, the number of mice in the CDK inhibitor group whose feces reduction rate was lower than that of mice in the anti-tumor medicament group was recorded. Table 12 lists various animal experiment combinations of anti-tumor medicaments and CDK inhibitors, as well as the corresponding experimental results. (Relative remission rate%= the number of mice in the CDK inhibitor group which have been effectively relieved/the total number of mice in the CDK inhibitor group * 100%. The establishment rate of constipation model in the chemotherapeutic agent group was 30%-50%, that is, about 3-5 of 10 mice showed reduced feces amount during the experiment, and there were cases of individual mice death or with no changes or an increasement in the feces amount.)

**Table 12: Experimental Conditions and Results of Examples 486-487**

| Ex. * | Anti. medi. * | Target | Adm in.m * | Dos. * | Freq. * | CDK i.* | Ad min. t.* | Adm in.m. * | Dos. * | Freq. * | Ex. cycl e (d) | Rem. rate |
|---|---|---|---|---|---|---|---|---|---|---|---|---|
| 486 | Osim | EGFR | Oral | 80 | Daily, 14d | Palbo | 12 h | Oral | 150 | Daily, 14d | 14 | Ineff. |
| 487 | Imat | TKI | Oral | 150 | Daily, 14d | Palbo | 12 h | Oral | 150 | Daily, 14d | 12 | Ineff. |

| | | | | | | | | | | | | |
|---|---|---|---|---|---|---|---|---|---|---|---|---|
| Ex.*: Example No.; Anti.medi: Anti-toumor medicament; Admin.m.*: Administration mode; Dos*: Dosage (mg/kg); Freq*: Frequency; CDK. i*; CDK inhibitor; Admin.t.*: Administration time (before the chemotherapeutic agent); Ex. Cycle (d):Experimental cycle (days); Remrate*: Remissions rate; Osim: Osimertinib; Imat: Imattinib; Palbo: TKI: Tyrosine kinase inhibitor; Palbociclib; Oral: Oral administration; Daily, 14d: Once a day, for 14 days; Ineff. Ineffective. | | | | | | | | | | | | |

It can be seen from the experimental results in Table 12 that the CDK inhibitor has no relieving effect on constipation caused by the continuous administration of targeted anti-tumor medicaments.

The foregoing detailed description is provided by means of explanation and examples but is not intended to limit the scope of the attached claims. Various changes to the embodiments listed in the present application currently are apparent to those with ordinary skills in the art and are retained in the attached claims and their equivalent scope.

## Claims

1. A use of a cyclin-dependent kinase (CDK) inhibitor in preparing a medicament, said medicament is used for preventing, relieving and/or treating chemotherapy-associated gastrointestinal side effects in a subject.

2. The use of claim 1, wherein said CDK inhibitor comprises a reagent that reduces the expression of CDK, and/or a reagent that decreases the activity of CDK.

3. The use of any one of claims 1-2, wherein said CDK inhibitor acts directly on a CDK protein, a nucleic acid encoding a CDK protein, a cyclin and/or a nucleic acid encoding a cyclin.

4. The use of any one of claims 1-3, wherein said CDK inhibitor comprises CDK1 inhibitor, CDK2 inhibitor, CDK3 inhibitor, CDK4 inhibitor, CDK5 inhibitor, CDK6 inhibitor, CDK7 inhibitor, CDK8 inhibitor and/or CDK9 inhibitor.

5. The use of any one of claims 1-4, wherein said CDK inhibitor comprises CDK2 inhibitor.

6. The use of any one of claims 1-5, wherein said CDK inhibitor comprises CDK4 inhibitor.

7. The use of any one of claims 1-6, wherein said CDK inhibitor comprises CDK6 inhibitor.

8. The use of any one of claims 1-7, wherein said CDK inhibitor comprises CDK9 inhibitor.

9. The use of any one of claims 1-8, wherein said CDK inhibitor comprises CDK4/6 inhibitor.

10. The use of any one of claims 1-9, wherein said CDK inhibitor comprises CDK2/4/6 inhibitor.

11. The use of any one of claims 1-10, wherein said CDK inhibitor comprises CDK4/6/9 inhibitor.

12. The use of any one of claims 1-11, wherein said CDK inhibitor comprises a CDK inhibitor for local intestinal exposure.

13. The use of any one of claims 1-12, wherein said CDK inhibitor comprises a small molecular compound, a protein and/or a nucleic acid molecule.

14. The use of claim 13, wherein said small molecular CDK inhibitor comprises a small molecular CDK inhibitor that binds reversibly to CDK, a small molecular CDK inhibitor that binds irreversibly to CDK and/or a small molecular CDK inhibitor that binds specifically to mutant CDK.

15. The use of any one of claims 13-14, wherein said small molecular CDK inhibitor has a molecular weight that is less than or equal to 2000 Daltons, less than or equal to 1500 Daltons, less than or equal to 1200 Daltons, less than or equal to 1000 Daltons, less than or equal to 900 Daltons, less than or equal to 800 Daltons, less than or equal to 700 Daltons, less than or equal to 600 Daltons, less than or equal to 500 Daltons, less than or equal to 400 Daltons, less than or equal to 300 Daltons, less than or equal to 200 Daltons and/or less than or equal to 100 Daltons.

16. The use of any one of claims 1-15, wherein said CDK inhibitor comprises one or more compounds selected from the following group: Trilaciclib, Palbociclib, Ribociclib, Abemaciclib, FLX-925, SHR-6390, BPI-1178, BPI-16350, FCN 437, G2T28, XZP-3287, BEBT-209, TY-302, TQB-3616, HS-10342, PF-06842874, CS-3002, MM-D37K, zotiraciclib, XZP-3287, Rigosertib, KRX-0601, Riviciclib, roniciclib, Milciclib, Seliciclib, Roscovitine, Indisulam, Alvocidib, NUV-422, BEY-1107, GLR-2007, FN-1501, BCD-115, TP-1287, BAY-1251152, Atuveciclib, SEL-120, HX-301, Voruciclib, Fadraciclib, AGM-130, PHA-793887, PHA-690509, Dinaciclib, RO4584820, R547, AT-7519, RGB-286638, ZK-304709, IIIM-290, PF-07104091 and G1T38.

17. The use of any one of claims 1-16, wherein said chemotherapy comprises administration of a chemotherapeutic agent.

18. The use of claim 17, wherein said chemotherapeutic agent is a cytotoxic agent.

19. The use of any one of claims 17-18, wherein said chemotherapeutic agent is selected from one or more of the following group: a DNA synthesis inhibitor, an RNA synthesis inhibitor, a protein synthesis inhibitor, a cell division inhibitor, a DNA base analogue, a topoisomerase inhibitor and/or a telomerase synthesis inhibitor.

20. The use of any one of claims 17-19, wherein said chemotherapeutic agent is selected from fluorouracil, oxaliplatin, topotecan, irinotecan, tetrahydrofolic acid, docetaxel, gemcitabine, carboplatin, cisplatin, etoposide, methotrexate, doxorubicin, cytarabine, vinorelbine and capecitabine, and a combination thereof.

21. The use of any one of claims 17-20, wherein said chemotherapeutic agent is administered continuously and/or noncontinuously.

22. The use of any one of claims 17-21, wherein said chemotherapeutic agent does not comprise chemotherapeutic agents of which the continuous administration time is 7 days or above.

23. The use of any one of claims 1-22, wherein said chemotherapy is employed in combination with one or more other therapies.

24. The use of any one of claims 1-23, wherein said chemotherapy-associated gastrointestinal side effects comprise gastrointestinal side effects caused by said chemotherapy.

25. The use of any one of claims 1-24, wherein said chemotherapy-associated gastrointestinal side effects comprise gastrointestinal adverse events that occur or deteriorate after the administration of said chemotherapeutic agent.

26. The use of any one of claims 17-25, wherein said gastrointestinal adverse events occur or deteriorate about 1 hr, about 2 hrs, about 3 hrs, about 4 hrs, about 5 hrs, about 6 hrs, about 7 hrs, about 8 hrs, about 9 hrs, about 10 hrs, about 11 hrs, about 12 hrs, about 1 day, about 2 days, about 4 days, about 7 days, about 2 weeks, about 3 weeks, about 1 month, about 2 months or longer after the administration of said chemotherapeutic agent, in the absence of prevention or treatment.

27. The use of any one of claims 1-26, wherein said gastrointestinal side effects comprise gastric mucosal injury diseases and/or intestinal mucosal injury diseases.

28. The use of any one of claims 1-27, wherein said gastrointestinal side effects comprise diarrhea, abdominal pain, nausea, vomiting, mucositis, loss of appetite, gastric ulcer, gastritis, constipation, enteritis, intestinal perforation, intestinal bleeding, ulcer and/or intestinal necrosis.

29. The use of any one of claims 1-28, wherein said gastrointestinal side effects comprise diarrhea and/or constipation.

30. The use of any one of claims 1-29, wherein the severity of said gastrointestinal side effects is Grade 1 or above, Grade 2 or above, Grade 3 or above, Grade 4 or above, or Grade 5, as evaluated in accordance with NCI-CTCAE.

31. The use of any one of claims 1-30, wherein said subject comprises a cancer patient.

32. The use of any one of claims 1-31, wherein said medicament does not substantially affect the therapeutic effect of said chemotherapy.

33. The use of any one of claims 1-32, wherein said medicament is administered about 0.5 hrs, about 1 hr, about 2 hrs, about 3 hrs, about 4 hrs, about 5 hrs, about 6 hrs, about 7 hrs, about 8 hrs, about 9 hrs, about 10 hrs, about 11 hrs, about 12 hrs, about 13 hrs, about 14 hrs, about 15 hrs, about 16 hrs, about 17 hrs, about 18 hrs, about 19 hrs, about 20 hrs or longer before the administration of said chemotherapy.

34. The use of any one of claims 1-33, wherein said medicament is administered about 0.5-12 hrs before the administration of said chemotherapy.

35. The use of any one of claims 1-34, wherein said medicament is prepared for oral administration, intravenous injection, subcutaneous injection, intraperitoneal injection and/or intramuscular injection.

36. The use of any one of claims 1-35, wherein said medicament is prepared for oral administration.

37. The use of any one of claims 1-36, wherein said medicament is prepared as tablets and/or capsules.

38. The use of any one of claims 1-37, wherein said medicament further comprises one or more other active ingredients.

39. The use of any one of claims 1-38, said CDK inhibitor is capable of improving or reducing said gastrointestinal side effects independently.

40. A method of preventing, relieving and/or treating chemotherapy-associated gastrointestinal side effects in a subject, comprising administering to the subject in need thereof said CDK inhibitor as claimed in the use of any one of claims 1-39.

41. The CDK inhibitor as claimed in the use of any one of claims 1-39, which is used for preventing, relieving and/or treating chemotherapy-associated gastrointestinal side effects in a subj ect.

42. A pharmaceutical combination, comprising said CDK inhibitor and said chemotherapeutic agent as claimed in the use of any one of claims 1-39.

43. A kit, comprising said CDK inhibitor and said chemotherapeutic agent as claimed in the use of any one of claims 1-39.
